# EUROPEAN PATENT SPECIFICATION

(11) **EP 2 486 404 B1**
(45) Date of publication and mention of the grant of the patent: **15.08.2018**
(21) Application number: 10765551.6
(22) Date of filing: 06.10.2010
(51) Int. Cl.: G01N 33/50

(54) **METABOLIC BIOMARKERS OF DRUG-INDUCED CARDIOTOXICITY**
STOFFWECHSEL-BIOMARKER FÜR ARZNEIMITTELINDUZIERTER KARDIOTOXIZITÄT
BIOMARQUEURS MÉTABOLIQUES DE CARDIOTOXICITÉ INDUITE PAR LES MÉDICAMENTS

(30) Priority: 06.10.2009 US 249150 P
(43) Date of publication of application: 15.08.2012
(73) Proprietor: Wisconsin Alumni Research Foundation, Madison, WI 53707 (US); Stemina Biomarker Discovery, Inc., Madison, WI 53719 (US)
(72) Inventor: CEZAR, Gabriela, Middleton, WI 53562 (US); SMITH, Alan, Madison, WI 53715 (US)
(74) Representative: J A Kemp
(86) International application number: PCT/US2010/051654
(87) International publication number: WO 2011/044253

(56) References cited:
- WO-A2-2007/103374
- WO-A2-2008/097491
- LEWIS GREGORY D ET AL: "Metabolite profiling of blood from individuals undergoing planned myocardial infarction reveals early markers of myocardial injury", JOURNAL OF CLINICAL INVESTIGATION, vol. 118, no. 10, October 2008 (2008-10), pages 3503-3512, XP002613889, ISSN: 0021-9738
- BRINDLE J T ET AL: "Rapid and noninvasive diagnosis of the presence and severity of coronary heart disease using 1H-NMR-based metabonomics", NATURE MEDICINE, NATURE PUBLISHING GROUP, NEW YORK, NY, US, vol. 8, no. 12, 1 January 2002 (2002-01-01), pages 1439-1444, XP002975960, ISSN: 1078-8956, DOI: 10.1038/NM802
- ANDREADOU IOANNA ET AL: "Metabonomic identification of novel biomarkers in doxorubicin cardiotoxicity and protective effect of the natural antioxidant oleuropein.", NMR IN BIOMEDICINE JUL 2009 LNKD- PUBMED:19308947, vol. 22, no. 6, July 2009 (2009-07), pages 585-592, XP002613890, ISSN: 1099-1492
- Anonymous: "CardioPlate 96-well assay-ready plates enable predictive toxicology and high throughput screening assays.", California Stem Cell: News and Events , 17 September 2009 (2009-09-17), XP002613891, Retrieved from the Internet: URL:http://www.californiastemcell.com/cgi- bin/pressrel?090917 [retrieved on 2010-12-13] -& Smith et al: "Predictive Metabolism Based Model of Cardiomyopathy using human embryonic stem cell derived cardiac precursors", Stemina Biomarker Discovery: Publications , 17 September 2009 (2009-09-17), XP002613892, Retrieved from the Internet: URL:http://www.stemina.com/web/CardioTOX_A SMS2010v3_final.pdf [retrieved on 2010-12-13]
- CEZAR GABRIELA G ET AL: "Identification of small molecules from human embryonic stem cells using metabolomics.", STEM CELLS AND DEVELOPMENT DEC 2007 LNKD- PUBMED:18042039, vol. 16, no. 6, December 2007 (2007-12), pages 869-882, XP002613893, ISSN: 1547-3287
- SCHNACKENBERG ET AL: "Metabolomic biomarkers: their role in the critical path", DRUG DISCOVERY TODAY: TECHNOLOGIES, ELSEVIER, vol. 4, no. 1, 3 December 2007 (2007-12-03), pages 13-16, XP022382635, ISSN: 1740-6749, DOI: 10.1016/J.DDTEC.2007.10.012
- SCHNACKENBERG LAURA K ET AL: "The role of metabolic biomarkers in drug toxicity studies", TOXICOLOGY MECHANISMS AND METHODS, TAYLOR & FRANCIS INC., PHILADELPHIA, PA, US, vol. 18, no. 4, 1 January 2008 (2008-01-01), pages 301-311, XP009142350, ISSN: 1537-6516, DOI: 10.1080/15376510701623193
- BISTOLA V ET AL: "Long-term primary cultures of human adult atrial cardiac myocytes: Cell viability, structural properties and BNP secretion in vitro", INTERNATIONAL JOURNAL OF CARDIOLOGY, ELSEVIER SCIENCE PUBLISHERS, AMSTERDAM, NL, vol. 131, no. 1, 17 December 2008 (2008-12-17), pages 113-122, XP025690912, ISSN: 0167-5273, DOI: 10.1016/J.IJCARD.2007.10.058 [retrieved on 2008-02-05]
- Silas G. Villas-Bôas ET AL: "Mass spectrometry in metabolome analysis", MASS SPECTROMETRY REVIEWS., vol. 24, no. 5, 1 January 2005 (2005-01-01), pages 613-646, XP055243707, US ISSN: 0277-7037, DOI: 10.1002/mas.20032
- SMITH A M ET AL: "Predictive Metabolism Based Model of Cardiomyopathy Using Human Embryonic Stem Cell Derived Cardiac Precursors", STEMINA BIOMARKER DISCOVERY: PUBLICATIONS, , 17 September 2009 (2009-09-17), 17 August 2017 (2017-08-17), page 1, XP002613892, Retrieved from the Internet: URL:http://www.stemina.com/web/CardioTOX_A SMS2010v3_final.pdf [retrieved on 2010-12-13]
- Alan Smith: "ASMS 2010 Salt Lake City", Predictive Metabolism based model of cardiomyopathy using human embryonic stem cell derived cardiac precursors, 1 January 2010 (2010-01-01), XP055400152,

## Description

### Field of the Invention

This invention relates to methods and biomarkers for identifying cardiotoxic effects of pharmaceuticals, biologics, and other chemical compounds and environmental agents. The invention specifically relates to methods for identifying low molecular weight metabolites secreted by cardiomyocytes in response to *in vitro* exposure to cardiotoxic compounds. Metabolomic methods are provided for identifying candidate biomarkers predictive of cardiotoxicity by measuring low molecular weight metabolites produced and secreted by cardiomyocytes contacted with a chemical compound, pharmaceutical, biologic or environmental agent. Predictive biomarkers for cardiotoxic effects are also identified.

### Background of the Invention

Cardiotoxicity has become one of the leading causes of pharmaceutical lead compound attrition and subsequent withdrawal of FDA-approved drugs from the market. The development of screening methods that provide specificity and accuracy for predicting cardiotoxicity are needed to better enable safe drug development and to help reduce soaring financial losses associated with preclinical drug failure.

Currently, cardiotoxicity can only be inferred, predominantly by measuring *in vitro* alterations to the action potential duration (APD) in cardiomyocytes using patch-clamp procedures. Despite the invaluable knowledge generated by electrophysiology assays, patch clamp procedures are extremely time consuming and low throughput. Briefly, the APD response to pharmaceutical compounds is measured a single cell at a time, and even so-called "high throughput" systems, such as PatchExpress®, only permit recordings of dozens of cells per assay. Most importantly, however, the mechanism of pharmacological cardiotoxicity is not uniform across drugs; thus electrophysiology recordings are limited in their ability to predict the cardiotoxicity of multiple compounds. While certain compounds exert their toxicity primarily by interfering with proper function of cardiac ion channels (which translate into changes to the APD and thus can be detected using conventional assays), others are known disruptors of cardiomyocyte metabolism that are not currently assayed. The primary toxicity of chemotherapies and kinase inhibitors used for cancer therapy, for example, results in significant changes to metabolic indicators in cardiomyocytes. Independent of the mechanism, cardiotoxicity would ultimately produce changes to the comprehensive collection of low molecular weight molecules from cardiomyocytes.

Dysregulation of metabolite synthesis, processing and abundance has been associated with cardiotoxicity. Chemotherapeutic and anti-tumor regimens are accompanied by marked changes to mitochondrial function, including interference with oxidative phosphorylation and inhibition of ATP synthesis, myofibrillar structure, and other aspects of energy metabolism. (Takemura & Fugiwara, 2007, Progress in Cardiovascular Diseases 49(5): 330-352). Other metabolic processes that have been implicated in the cardiotoxicity of cancer drugs include lipid peroxidation, oxidation of proteins and DNA, and depletion of glutathione and pyridine nucleotide reducing equivalents. Cardiotoxic side-effects are not limited to pharmaceutical compounds, as cardiotoxicity has been observed with monoclonal antibody therapies and biologics. Therapeutic antibodies such as HER2/ERBB2 monoclonal antibodies and trastuzumab in association with paclitaxel treatment regimen have been shown to have a synergistic negative impact on adult cardiomyocytes. (Pentassuglia et al., 2007, Experimental Cell Research, 313: 1588-1601). Detrimental effects of biologics on cardiac safety are prevalent independent of combined therapies: for example, eleven percent of patients on trastuzumab develop cardiac toxicity (Guarneri et al., 2006, Journal of Clinical Oncology, 24: 4107-4115).

Ioanna et al., 2009, NMR in Biomedicine, 22(6): 585-592 refers to metabonomic identification of novel biomarkers in doxorubicin cardiotoxicity and protective effect of the natural antioxidant oleuropein. A press release from California Stem Cell: News and Events, published on 17 September 2009, refers to CardioPlate 96-well assay-ready plates that enable predictive toxicology and high throughput screening assays.

There remains a need in this art for *in vitro* methods for reliably determining cardiotoxicity of pharmaceuticals, biologics, and other chemical compounds and environmental agents.

### Summary of the Invention

The present invention provides a method of identifying cellular metabolites differentially produced in human cardiomyocyte cells in the presence or absence of a test compound, the method comprising the steps of:
a) contacting cardiomyocyte cells in vitro with a test compound;
b) separating a plurality of cellular metabolites of from about 10 to about 1500 Daltons that are secreted from said cardiomyocyte cells using mass spectrometry; and
c) identifying one or a plurality of cellular metabolites of from about 10 to about 1500 Daltons that are differentially secreted from cardiomyocytes contacted with the test compound compared to cardiomyocytes not contacted with the test compound;
wherein said one or a plurality of cellular metabolites comprise one or a plurality of cellular metabolites set forth in Tables 2A-2D.

The present invention also provides a method for identifying cellular metabolites differentially produced by human cardiomyocyte cells in the presence or absence of a plurality of cardiotoxic test compounds, the method comprising the steps of:
a) separately contacting each of a plurality of experimental sets of cardiomyocyte cells in vitro with a different cardiotoxic test compound;
b) separating a plurality of cellular metabolites of from about 10 to about 1500 Daltons that are secreted from each experimental set of cells using mass spectrometry;
c) identifying one or a plurality of cellular metabolites of from about 10 to about 1500 Daltons that are differentially secreted from cardiomyocytes contacted with each of the cardiotoxic test compounds compared to cardiomyocytes not contacted with the cardiotoxic test compound; and
d) identifying one or a plurality of cellular metabolites differentially produced by substantially all of said experimental sets of cardiomyocyte cells exposed to said test compounds;
wherein said one or a plurality of cellular metabolites comprise one or a plurality of cellular metabolites set forth in Tables 2A-2D.

The present invention further provides a method of assessing cardiotoxicity of a test compound comprising the steps of:
a) contacting human cardiomyocyte cells in vitro with the test compound;
b) separating a plurality of cellular metabolites of from about 10 to about 1500 Daltons that are secreted from said cardiomyocyte cells using mass spectometry; and
c) identifying the test compound as a cardiotoxic compound if at least one or a plurality of cellular metabolites of from about 10 to about 1500 Daltons that are differentially secreted from cardiomyocytes contacted with the test compound comprise a metabolic profile of cardiotoxicity;
wherein said one or a plurality of cellular metabolites comprise one or a plurality of cellular metabolites set forth in Tables 2A-2D.

Described herein are reagents and methods for identifying a plurality of low molecular weight molecules, preferably secreted by cardiomyocytes or hESC-derived or human iPS-derived cardiac-specific cells, in response to pharmaceuticals, biologics, and other chemical compounds or environmental agents. In addition, the disclosure provides reagents and methods for identifying particular metabolites produced by cardiomyocytes in response to a pharmaceutical, biologic, other chemical compound or environmental agent, as well as pluralities of cellular metabolites produced by cardiomyocytes in response to a pharmaceutical, biologic, other chemical compound or environmental agent, thereby also providing metabolic profiles of specific metabolites produced, *for example,* as the result of cardiotoxicity and that are secreted in response to exposure to particular pharmaceuticals, biologics, and other chemical compounds and environmental agents. The present disclosure thus provides reagents and methods for predicting cardiotoxic effects of pharmaceuticals, biologics, and other chemical compounds and environmental agents using profiles of low molecular weight metabolites identified via metabolomic analysis of human cardiomyocytes contacted with such agents *in vitro.*

Low molecular weight metabolites can be sensitively detected in even low quantities by methods and technologies known in the art, including most particularly variations of liquid chromatography high resolution mass spectrometry (LC-MS) and/or electrospray ionization time of flight mass spectrometry (ESI-TOF). As disclosed herein the sensitivity of applying such methods to detecting metabolites produced by cardiomyocytes in response to pharmaceuticals, biologics, and other chemical compounds and environmental agents provides improved outcomes for detecting cardiotoxicity compared with less robust methods known in the art. Advantages of the inventive methods disclosed herein include that they provide direct products of the cardiotoxic response - metabolites produced by cardiomyocytes in response to insults from pharmaceuticals, environmental agents, chemical compounds and/or biologic therapies. The invention disclosed herein also advantageously provides metabolite profiles produced by contacting cardiomyocytes *in vitro* with specific pharmaceuticals, biologics, and other chemical compounds and environmental agents. These profiles are comprised of non-limiting collections of candidate biomarkers, providing a biochemical metabolic signature indicative of cardiotoxicity.

In particular aspects, the disclosure provides reagents and methods for *in vitro* screening using cardiomyocytes to detect metabolites associated with cardiotoxicity of specific pharmaceuticals, biologics, and other chemical compounds and environmental agents. The patterns and collections of metabolite biomarkers establish that such cardiomyocytes have a characteristic metabolic response to cardiotoxicity produced by contact with specific pharmaceuticals, biologics, and other chemical compounds and environmental agents.

Practice of the provided methods illustrates the cardiomyocyte metabolome includes potential human biomarkers for disease and cardiotoxic response. These biomarkers are identified by contacting cardiomyocytes with specific pharmaceuticals, environmental agents, chemical compounds and biologic therapies. The results set forth herein demonstrate that exposure of cardiomyocytes to known cardiotoxic drugs induced significant changes in different metabolic pathways, consistent with known activity as cardiotoxins, and further providing an exemplar for the practice of the inventive methods with uncharacterized pharmaceuticals, biologics, and other chemical compounds and environmental agents to determine the extent of any cardiotoxicity exhibited by these compounds.

Specific embodiments of this invention will become evident from the following more detailed description of certain preferred embodiments and the claims.

### Brief Description of the Drawings

These and other objects and features of this invention will be better understood from the following detailed description taken in conjunction with the drawings wherein:

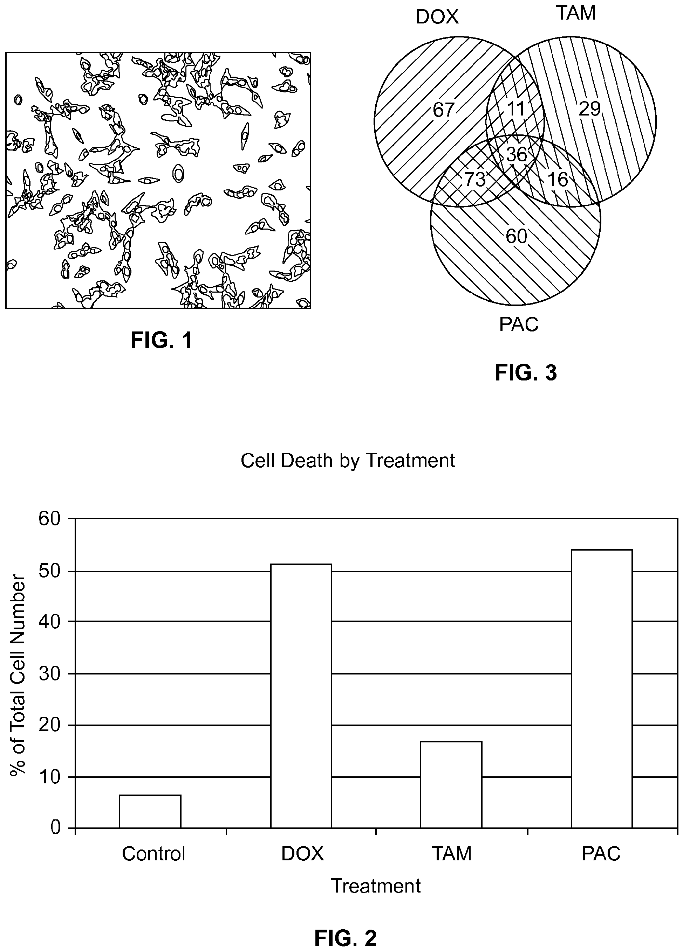

Figure 1 is a photograph of cardiac cells subjected to immunohistochemical (IHC) treatment for cardiac alpha actin. The IHC staining of alpha actin confirmed the cardiac origin of cells exposed to doxorubicin, paclitaxel and tamoxifen. Cardiac cells were subjected to drug treatment for the identification of predictive metabolic biomarkers of cardiotoxicity.
Figure 2 is a graph of percentages of cell death of human cardiomyocytes in response to exposure to anti-tumor drugs as measured by Trypan Blue dye inclusion.
Figure 3 is a Venn diagram of statistically significant mass features, representing different metabolites, in human cardiomyocytes treated with doxorubicin (DOX), paclitaxel (PAC), and tamoxifen (TAM) at 0.05 False Discovery Rates (FDR). Seventy-three features were common to strong cardiotoxicants DOX and PAC.

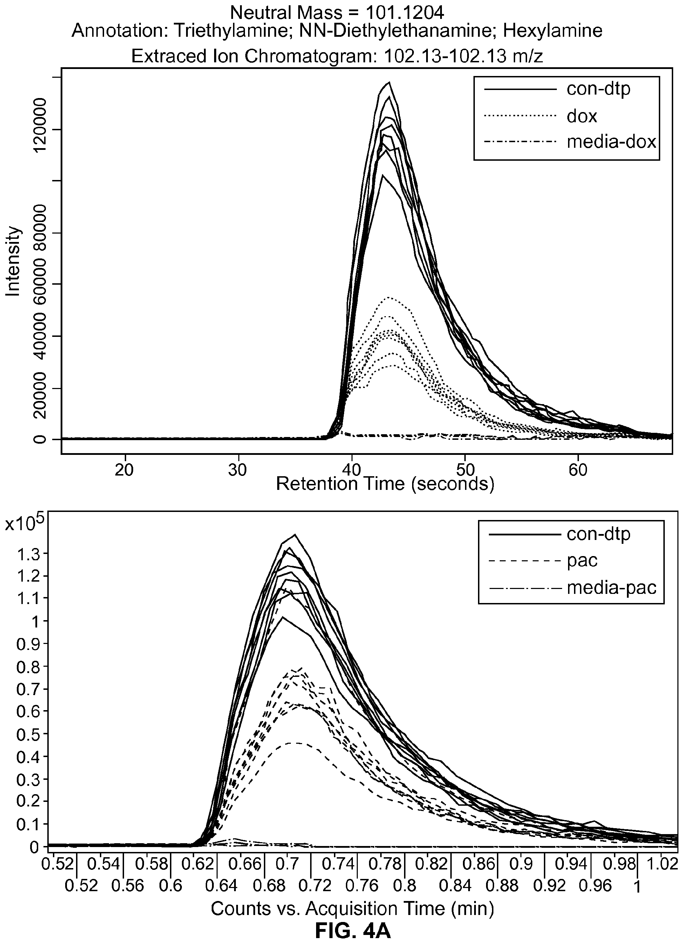

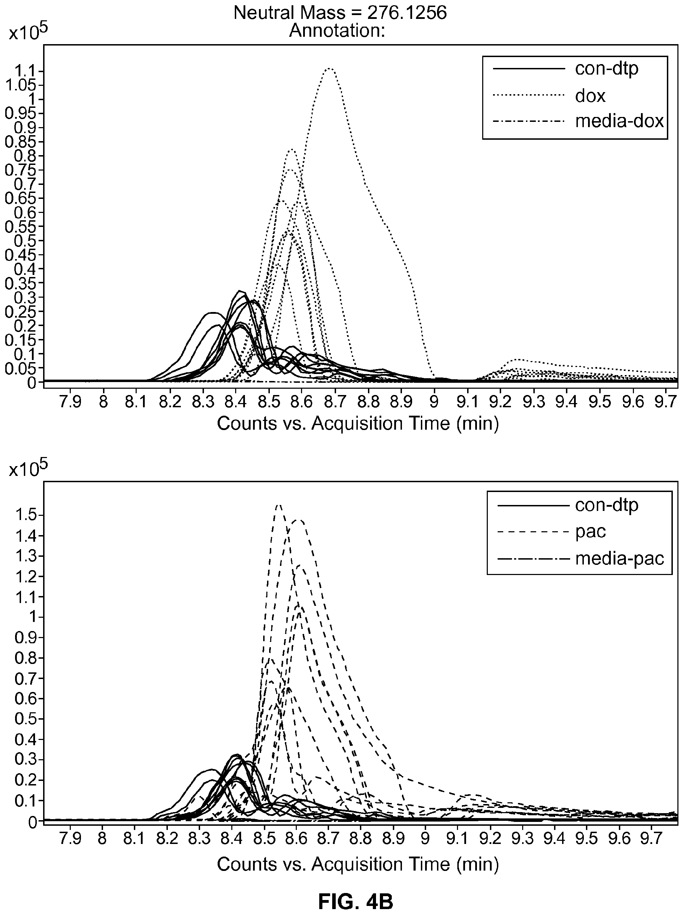

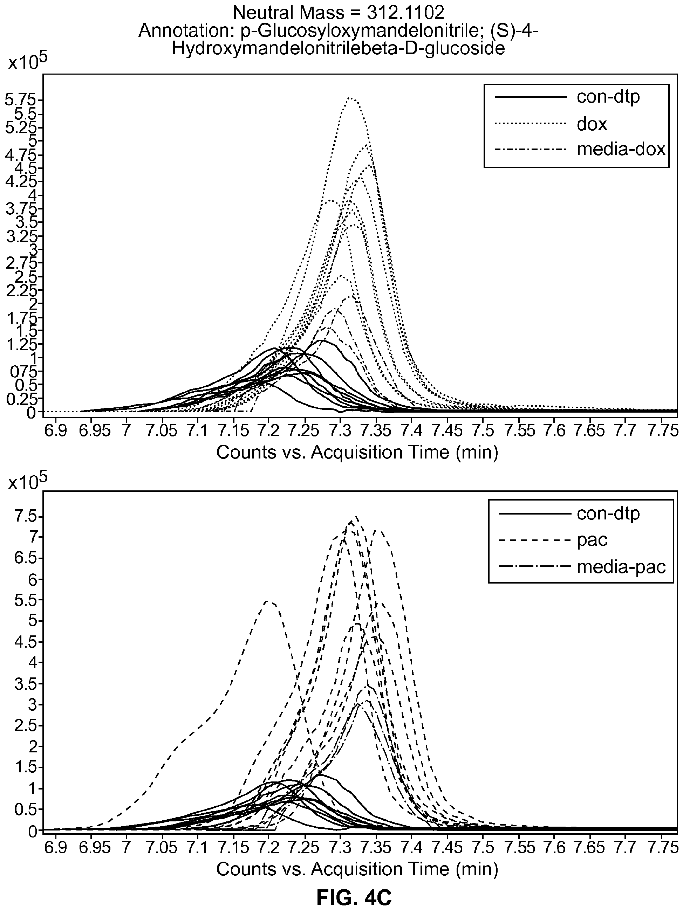

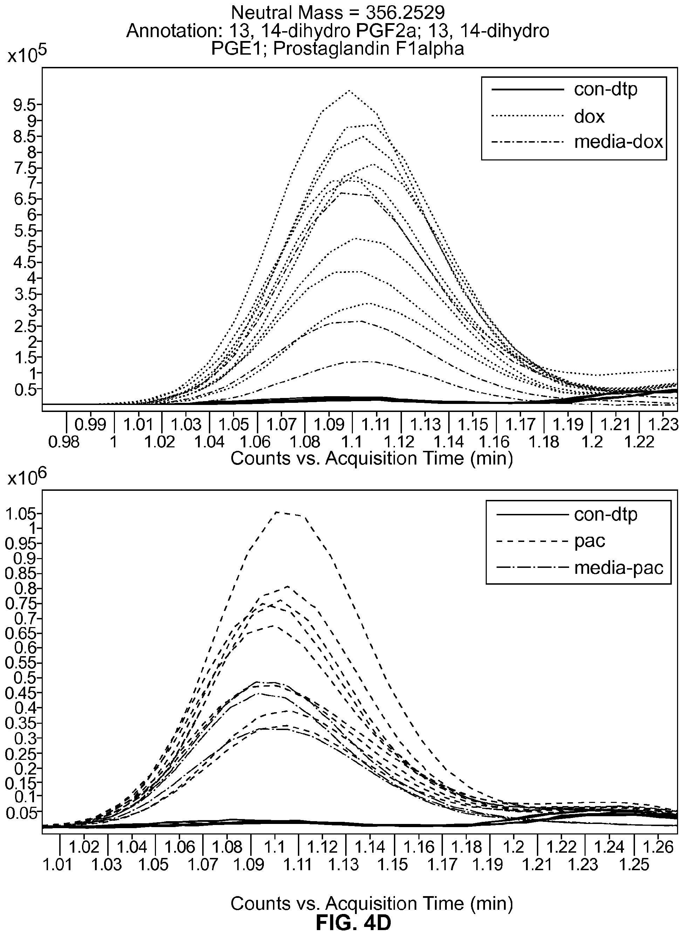

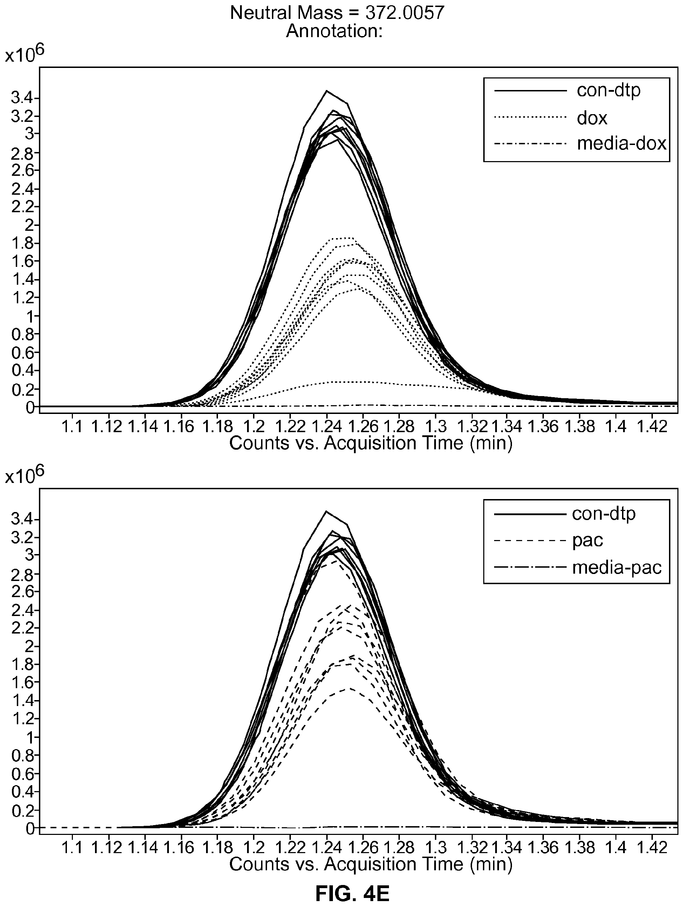

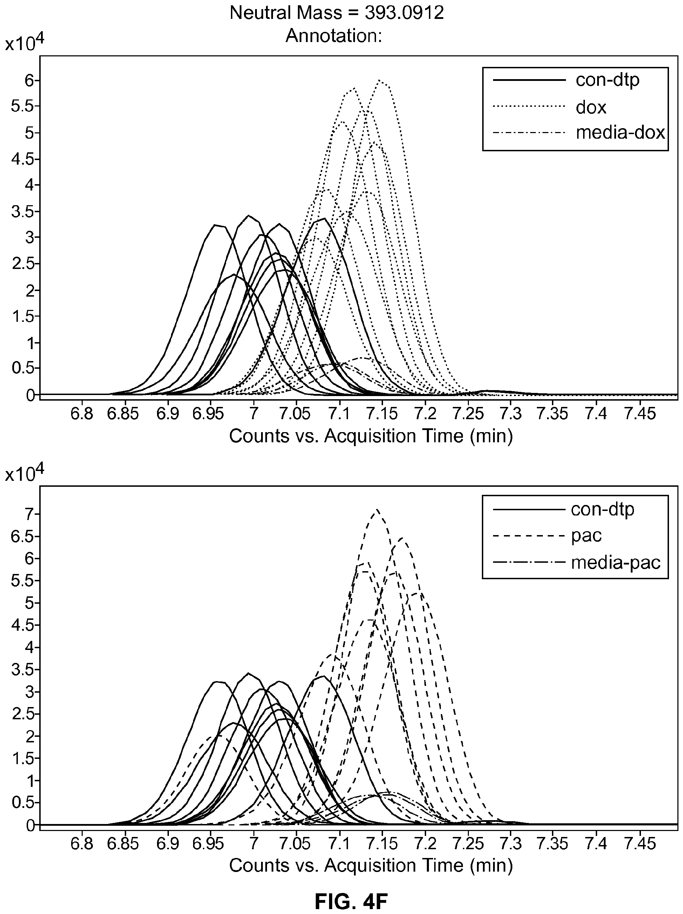

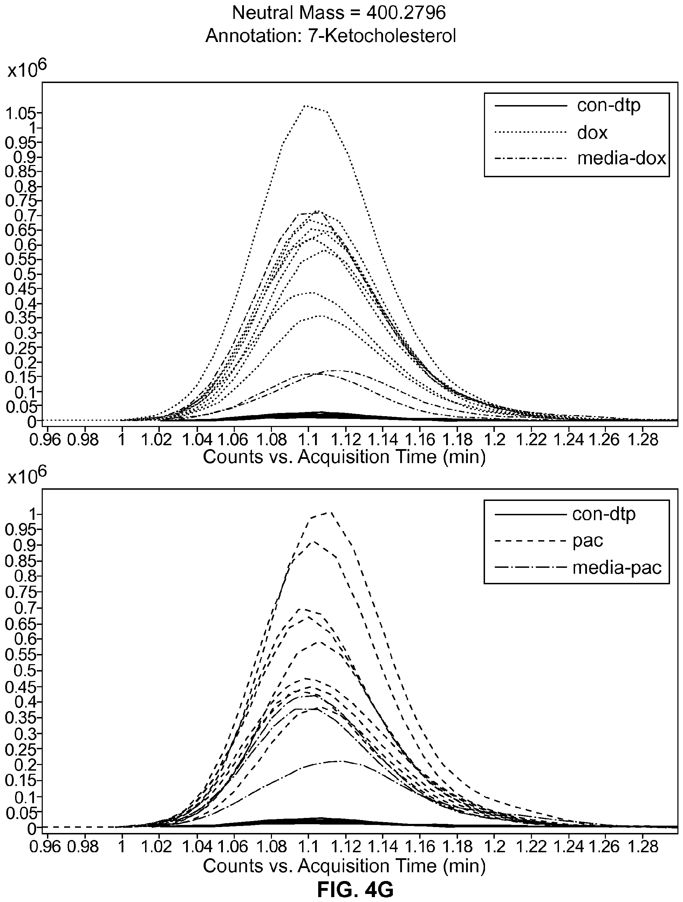

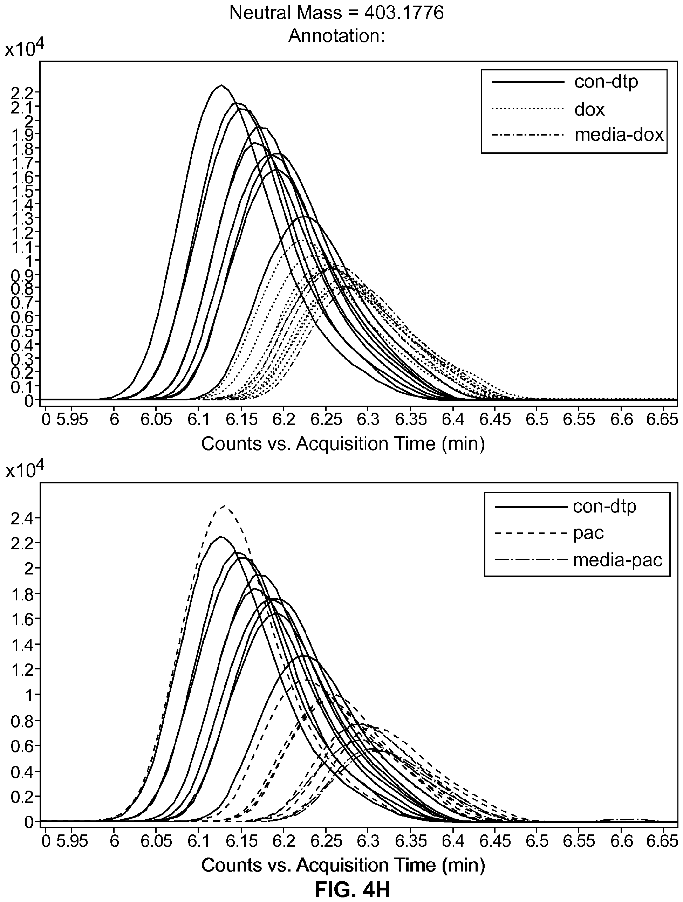

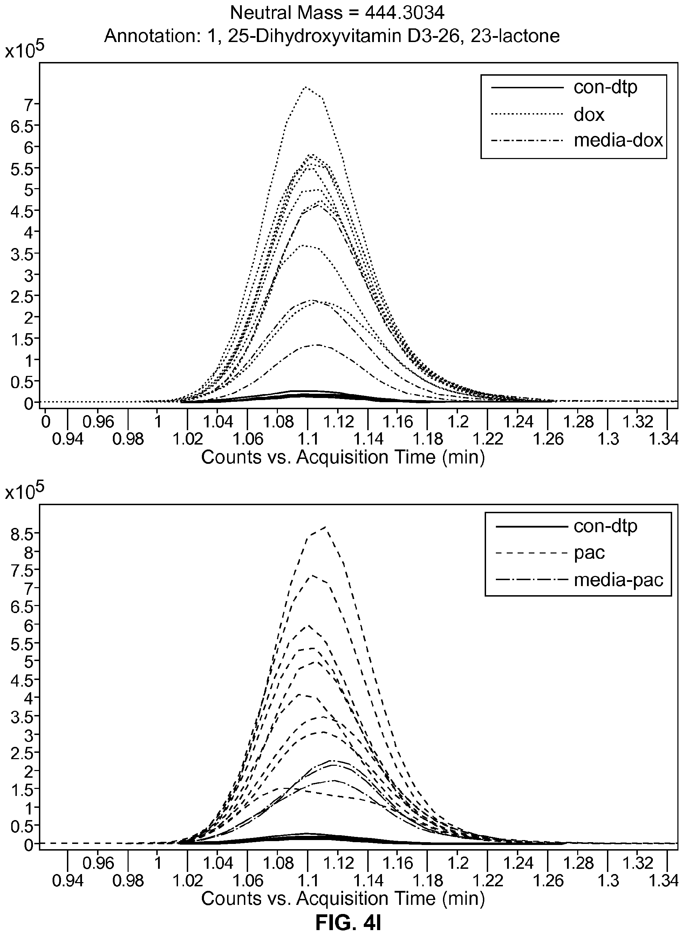

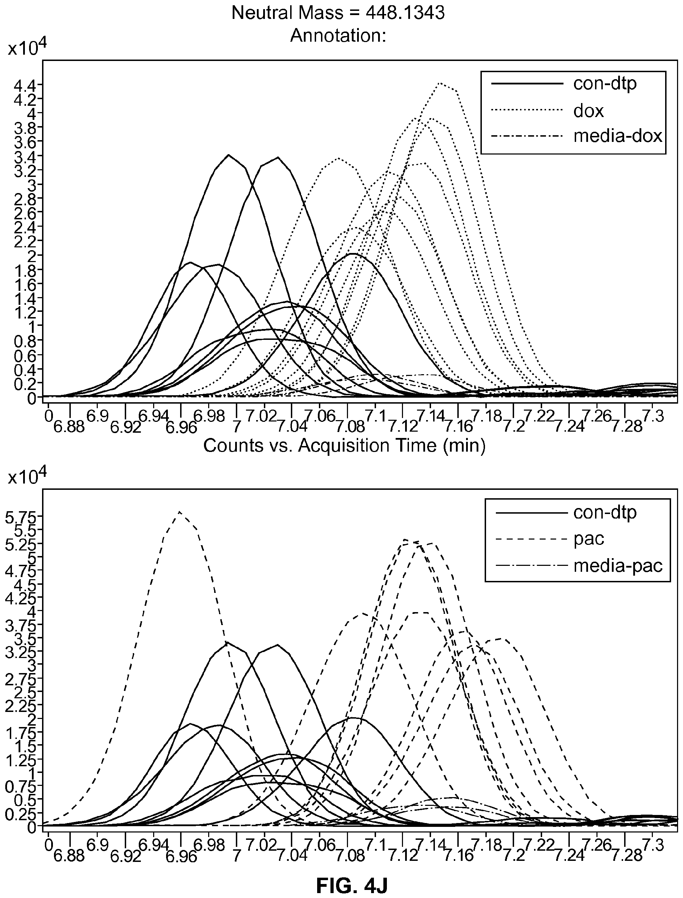

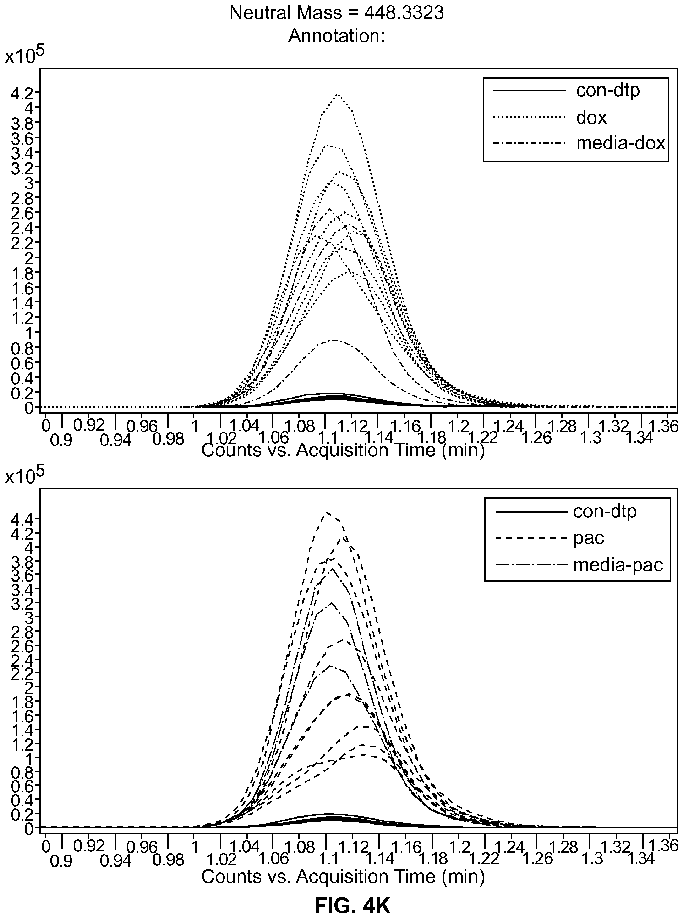

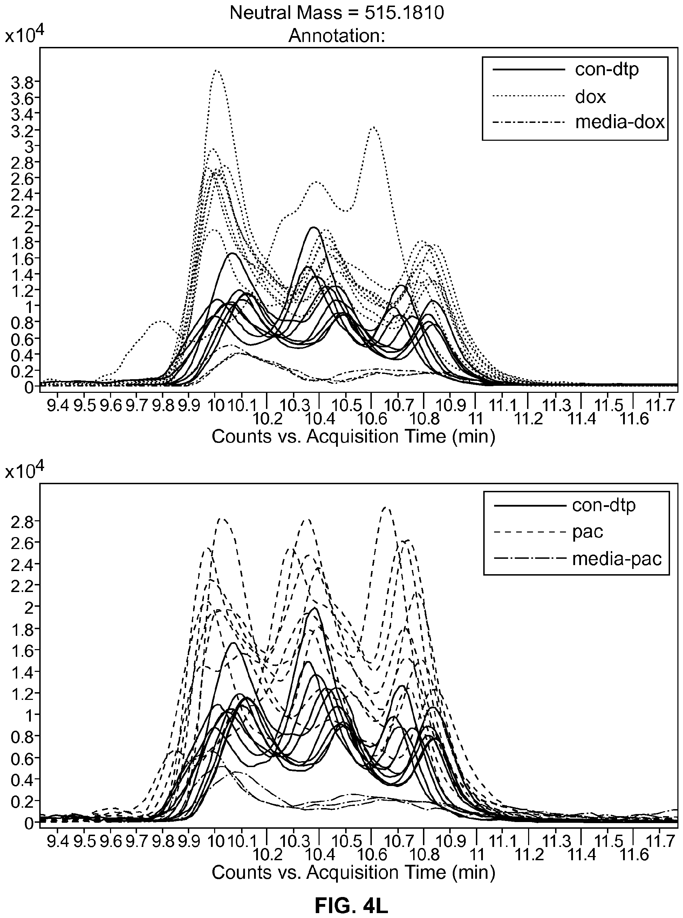

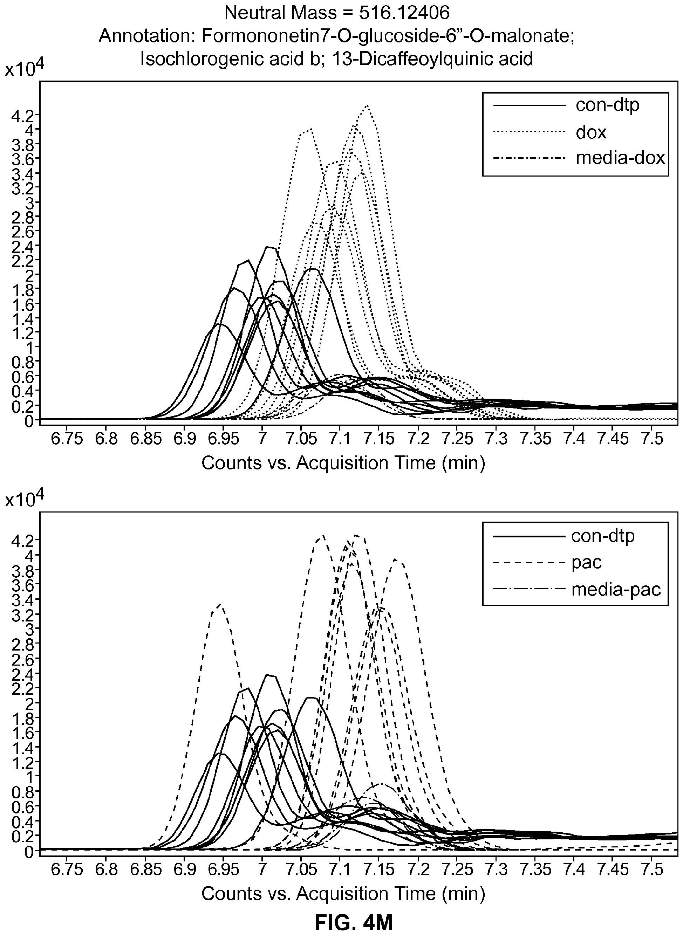

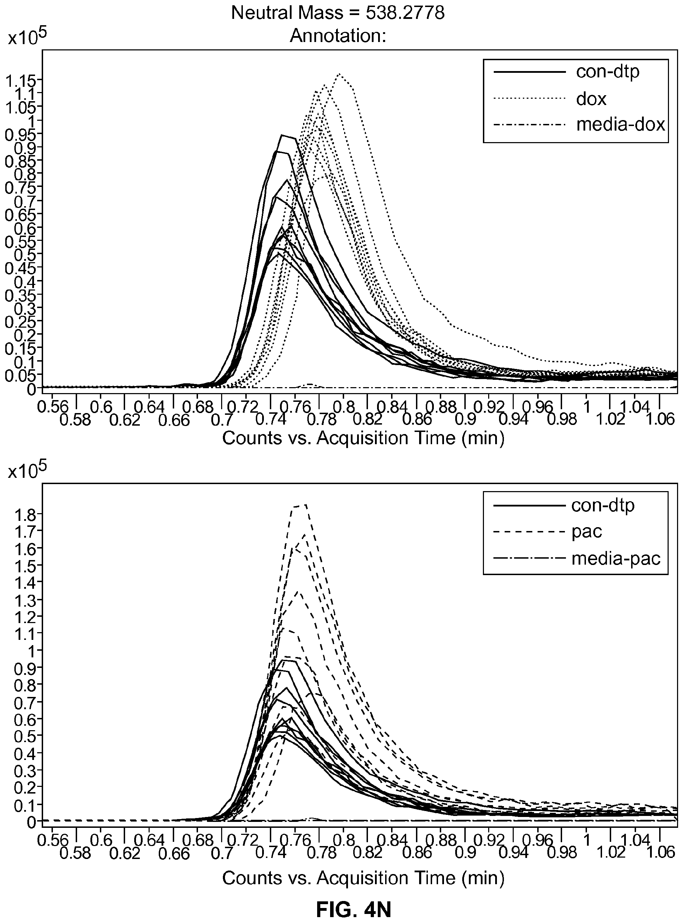

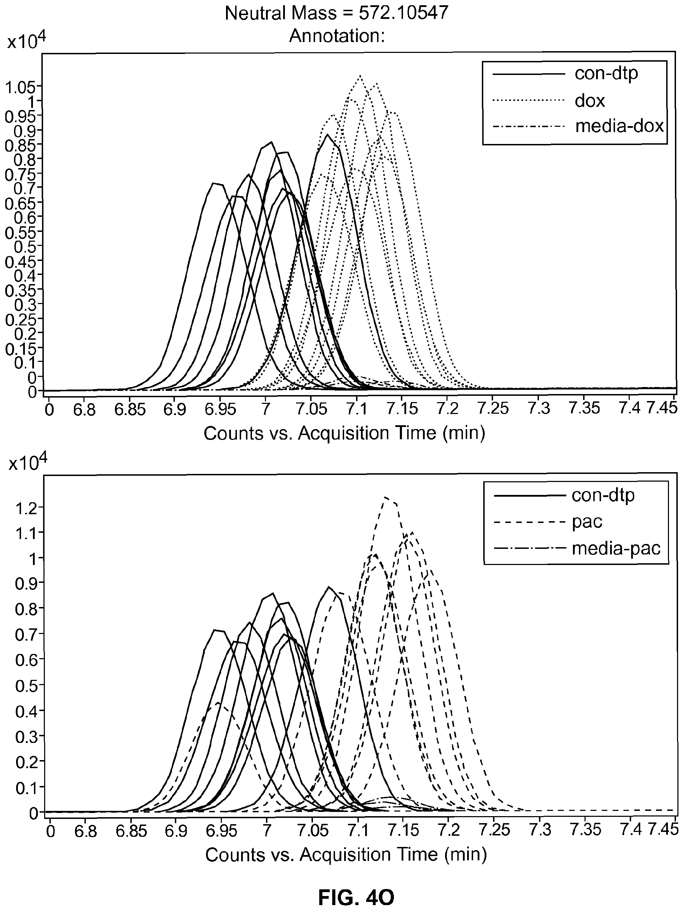

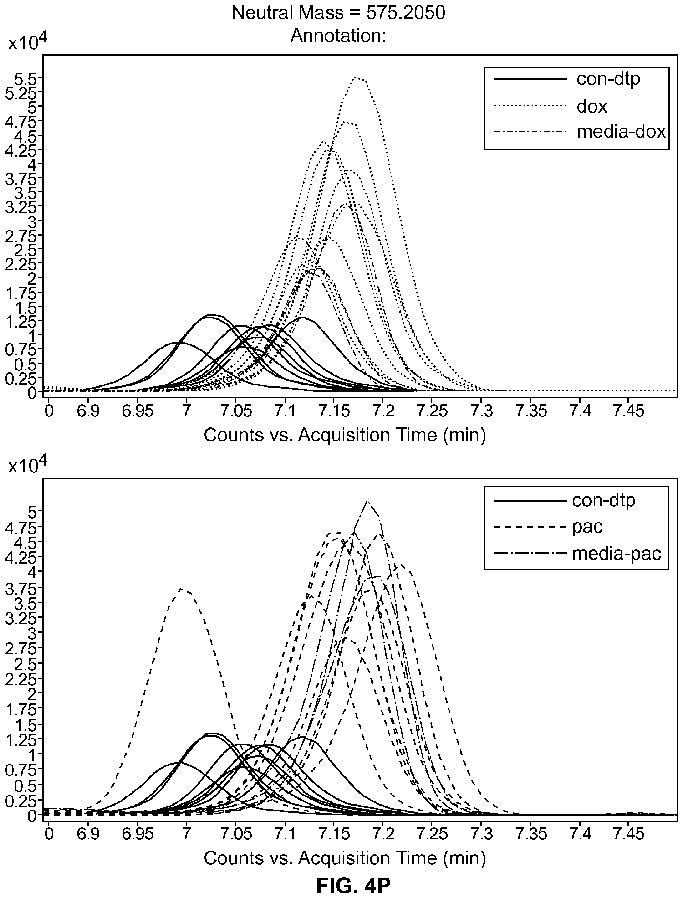

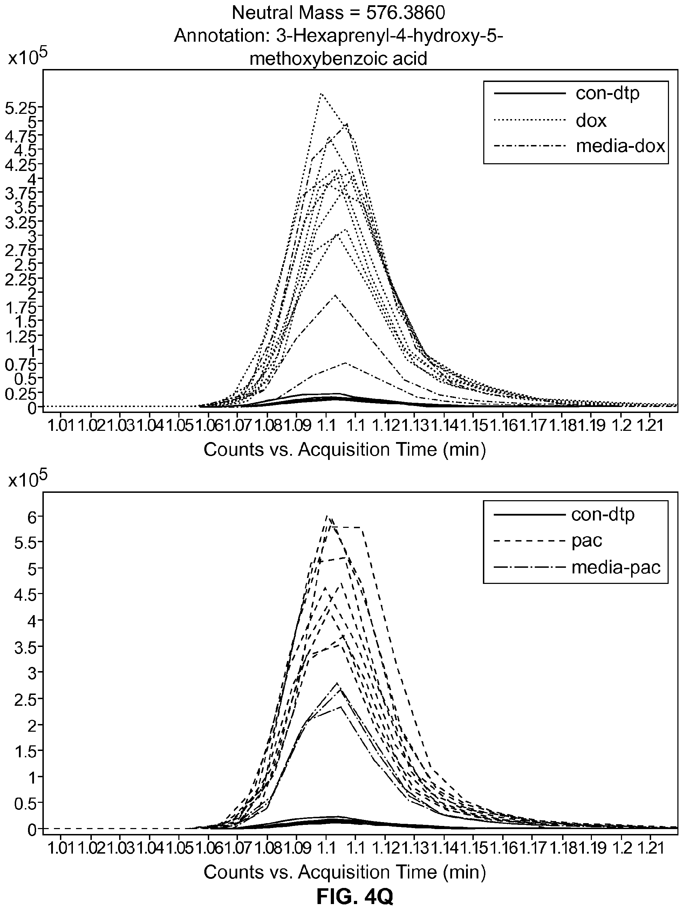

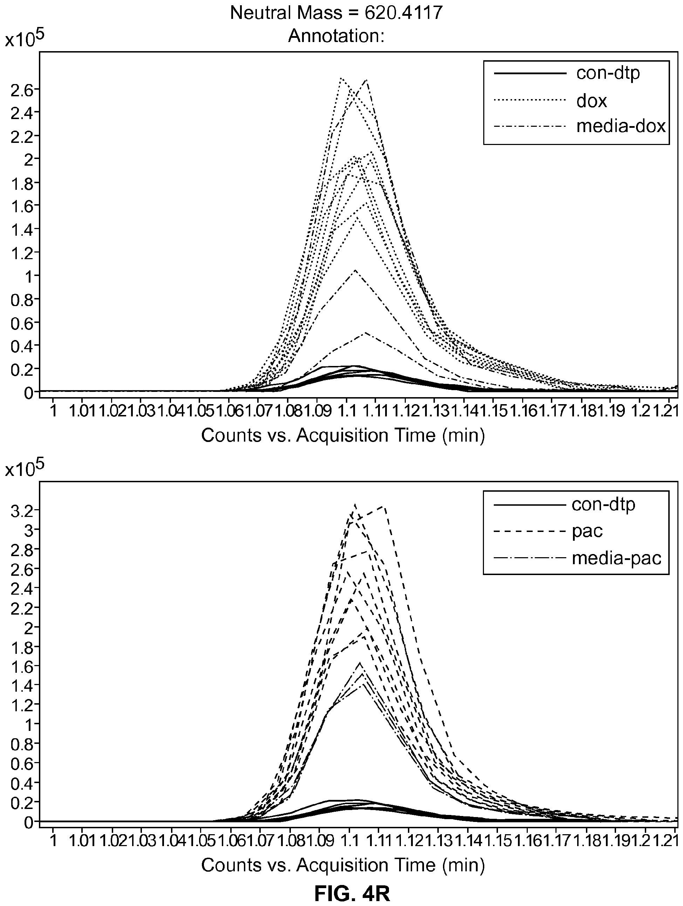

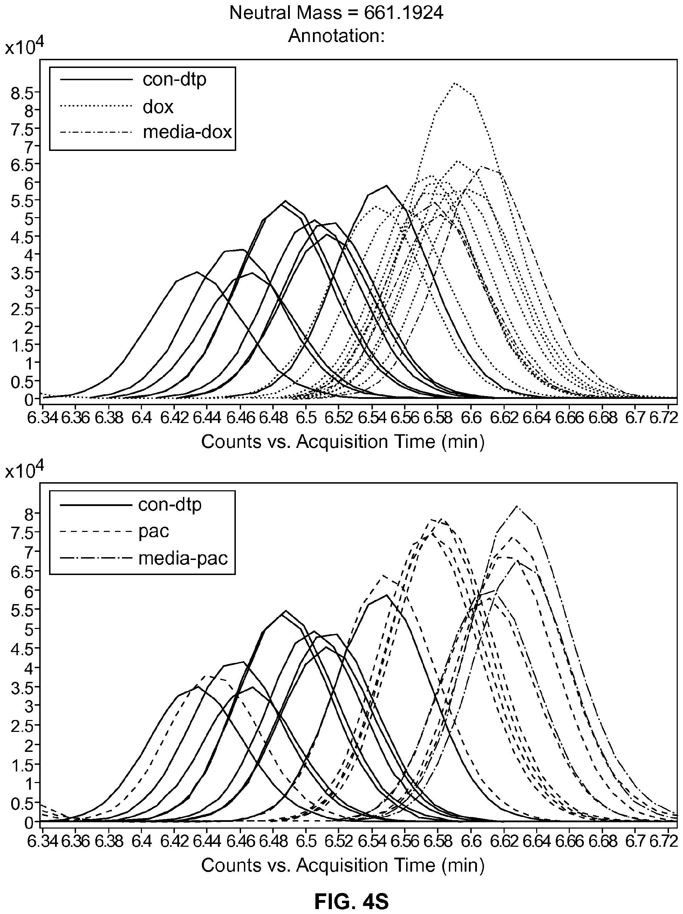

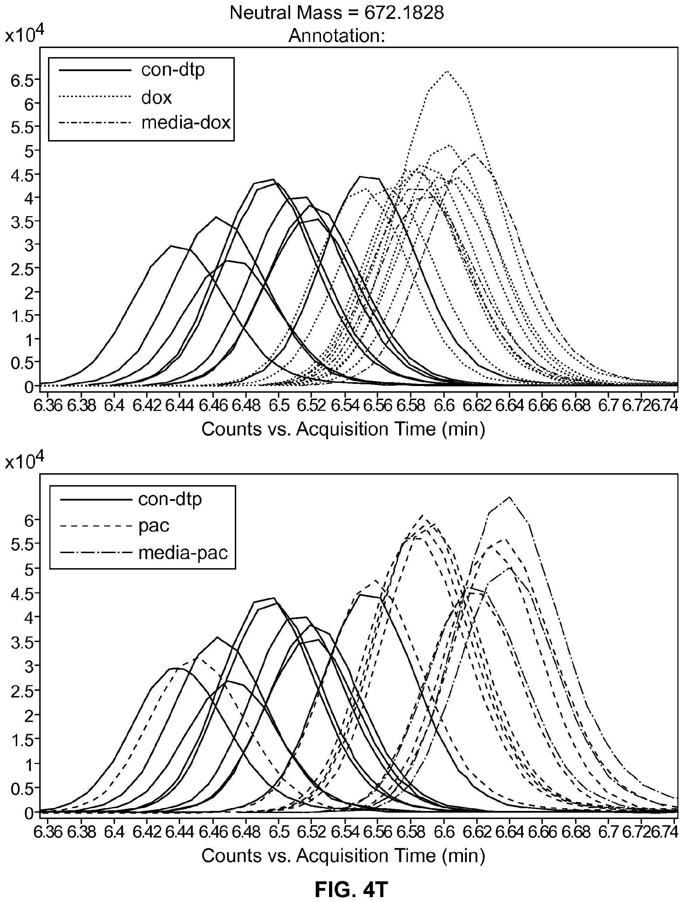

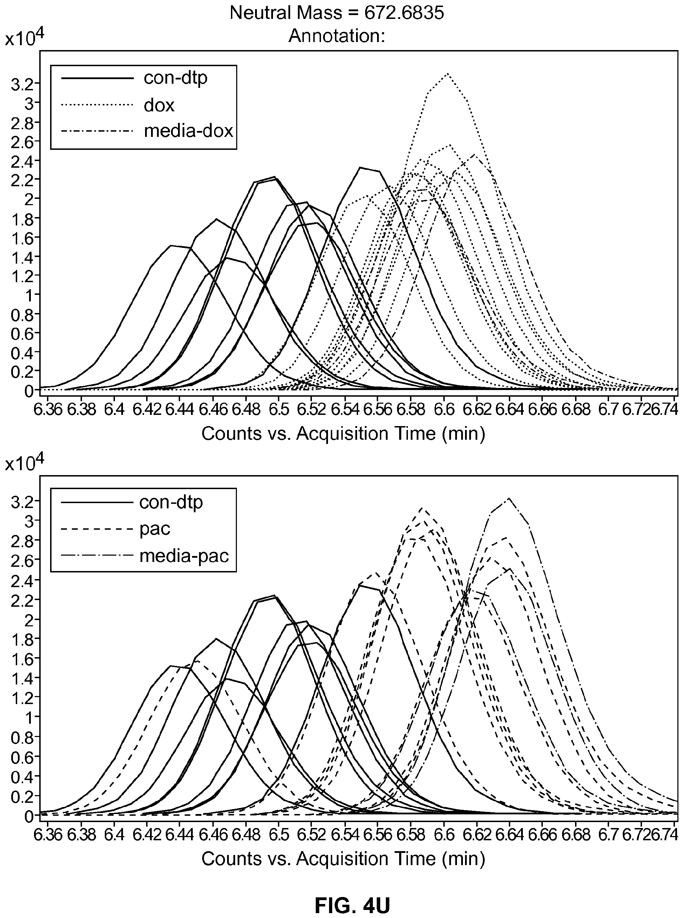

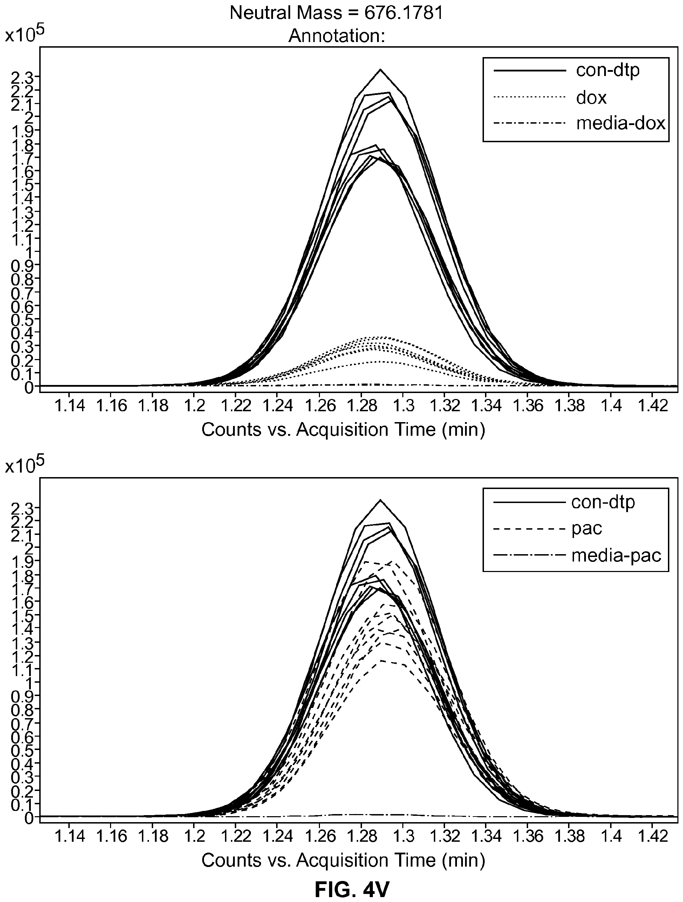

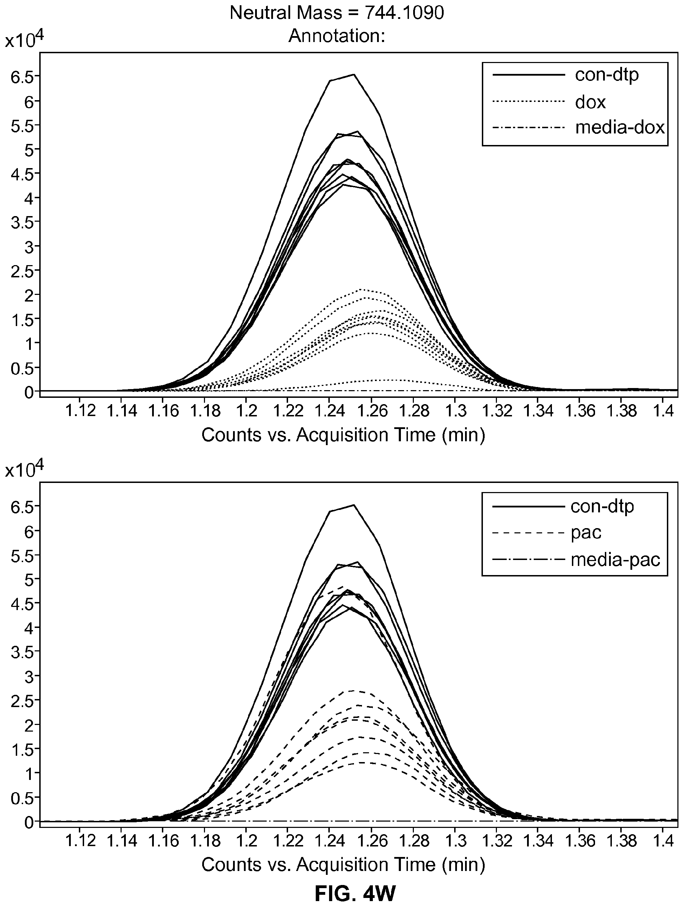

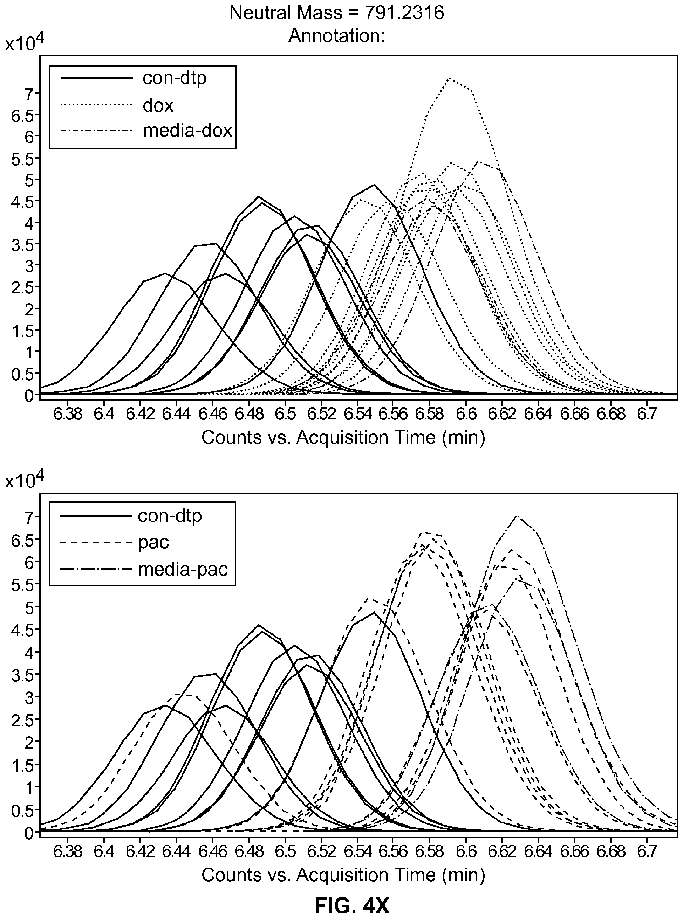

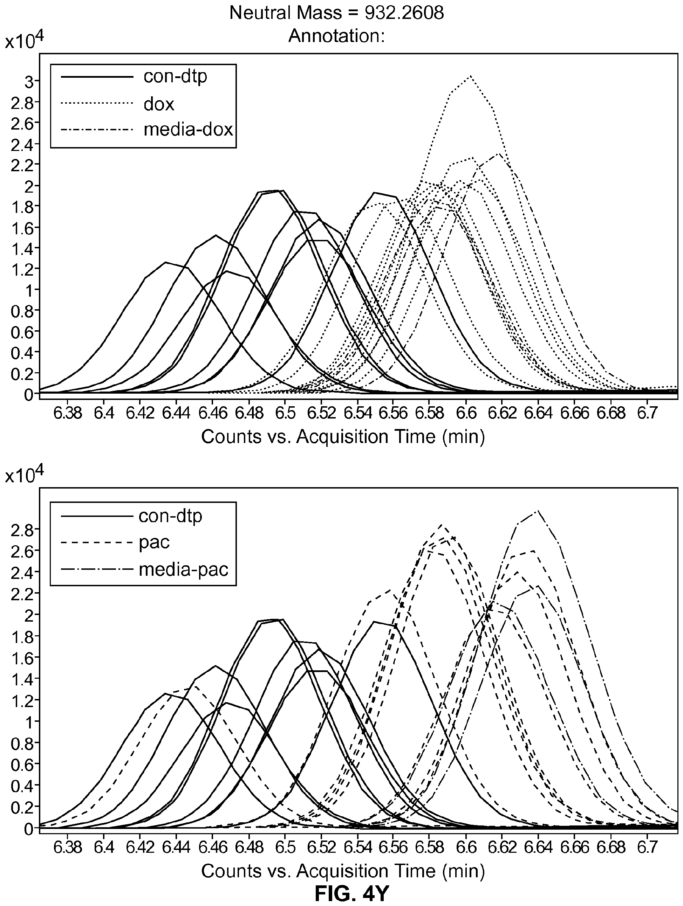

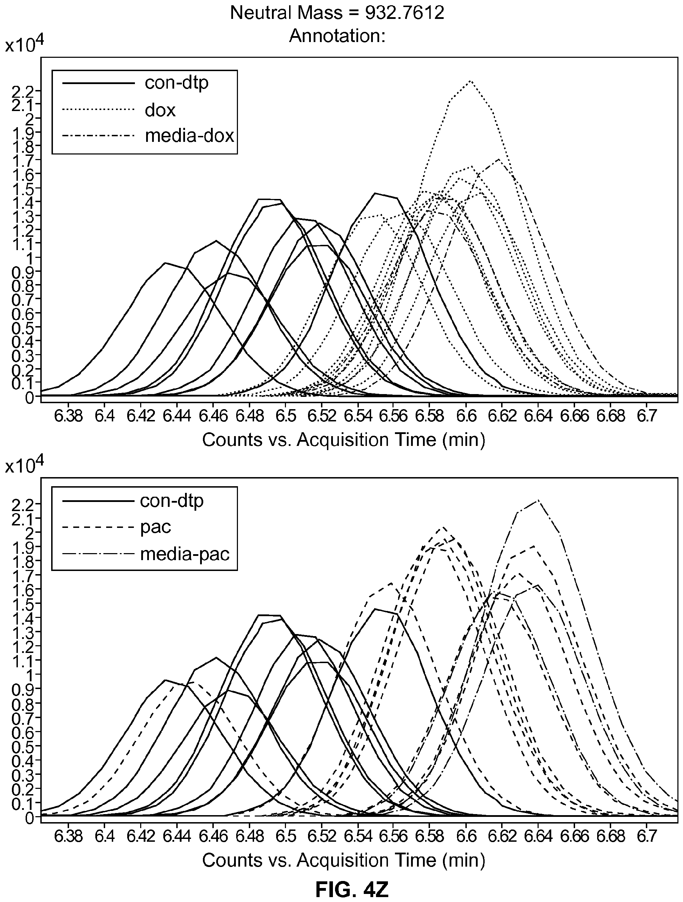

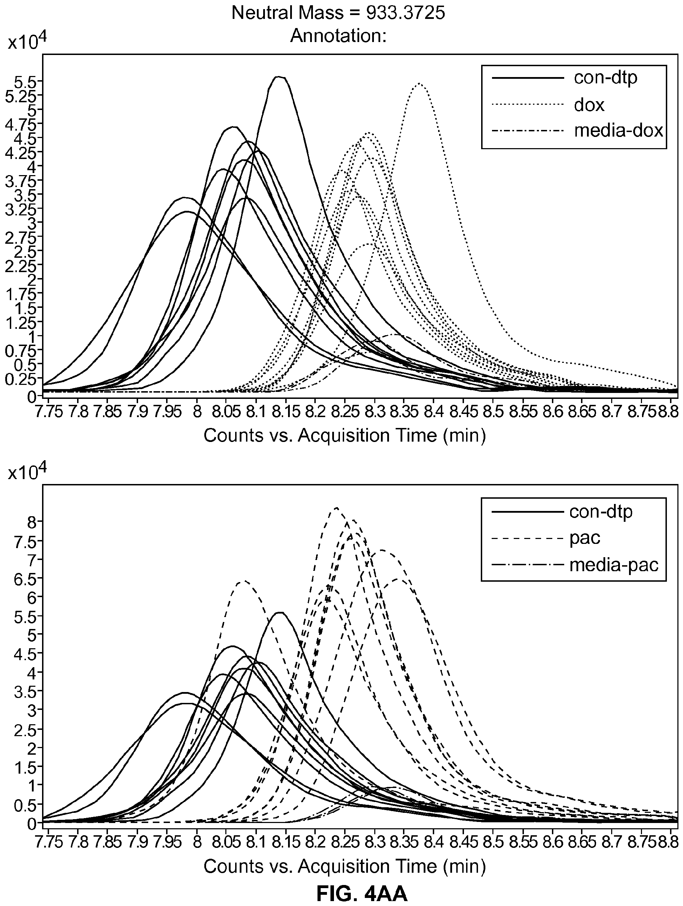

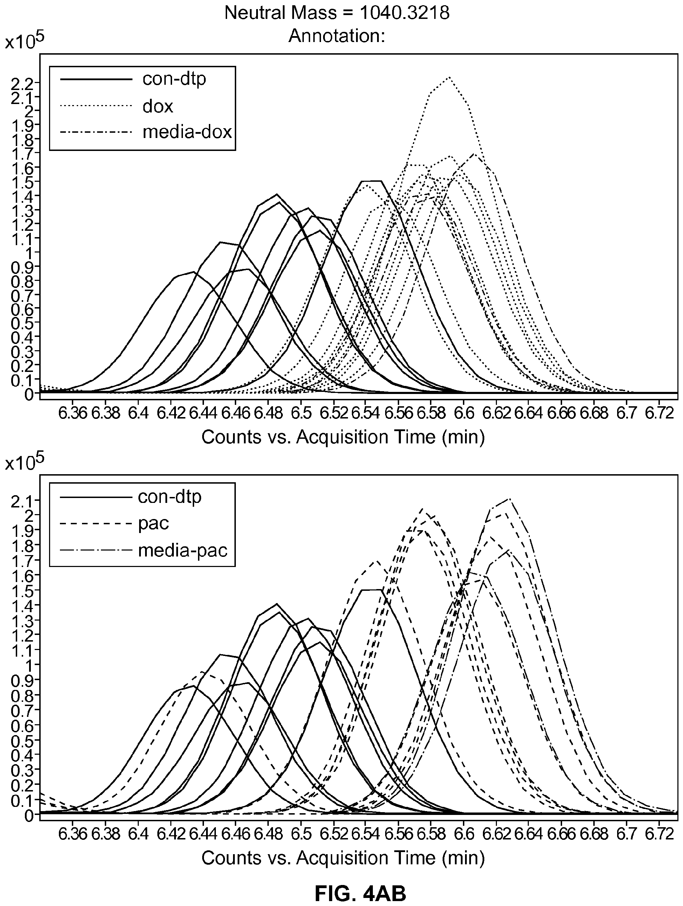

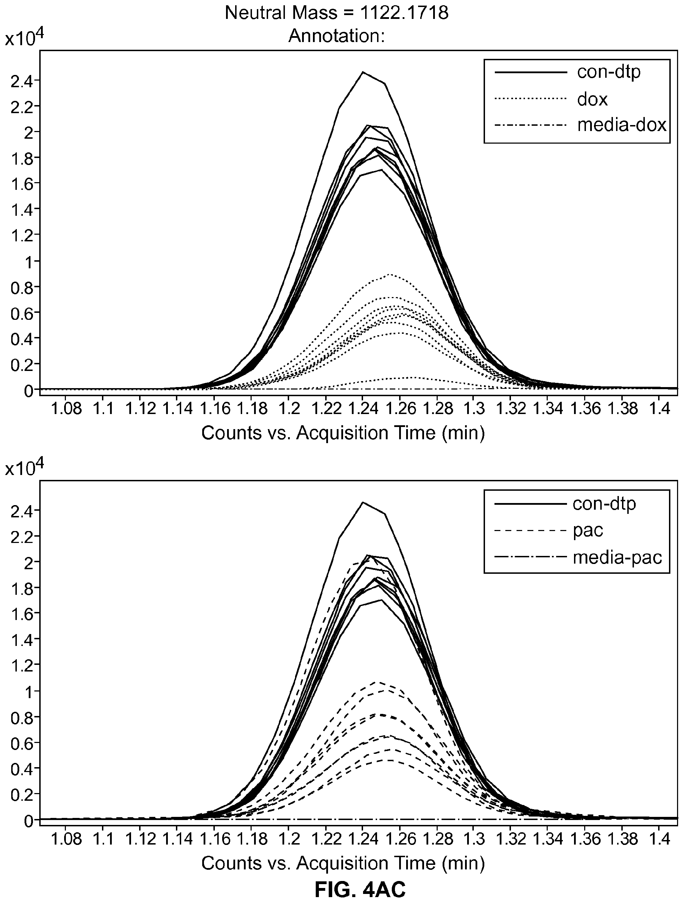

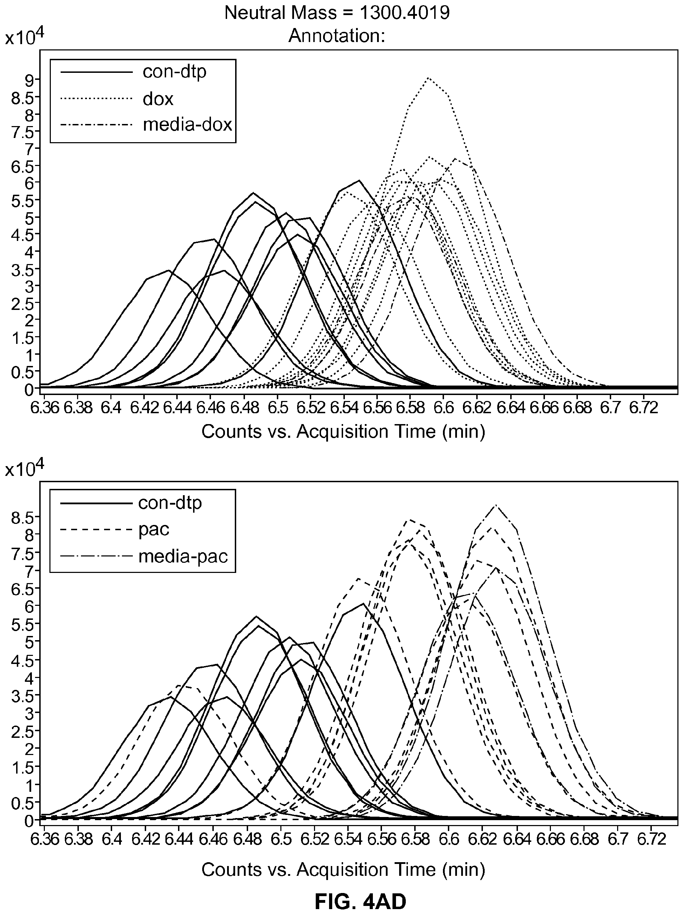

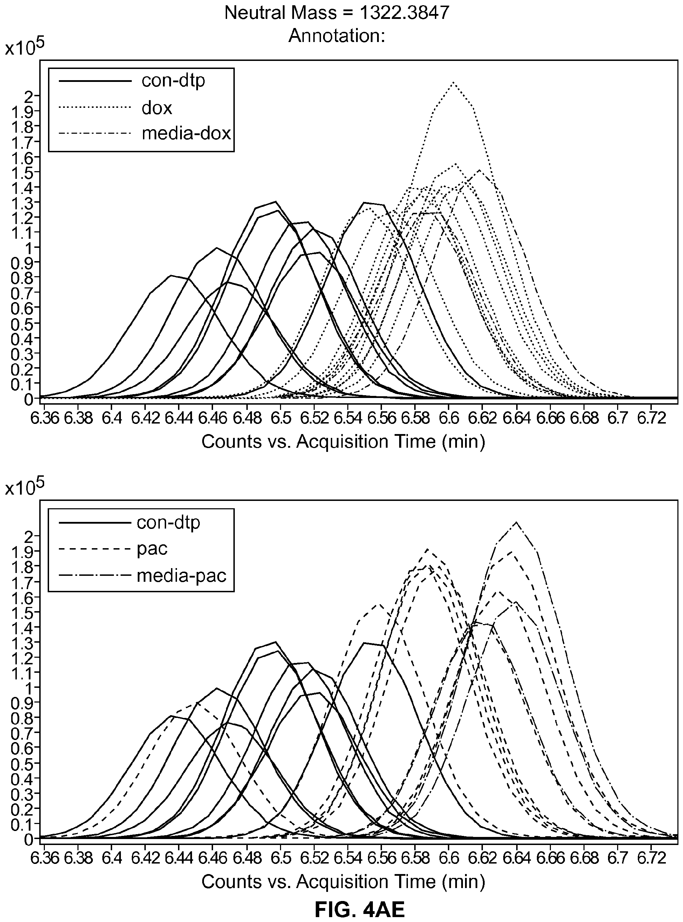

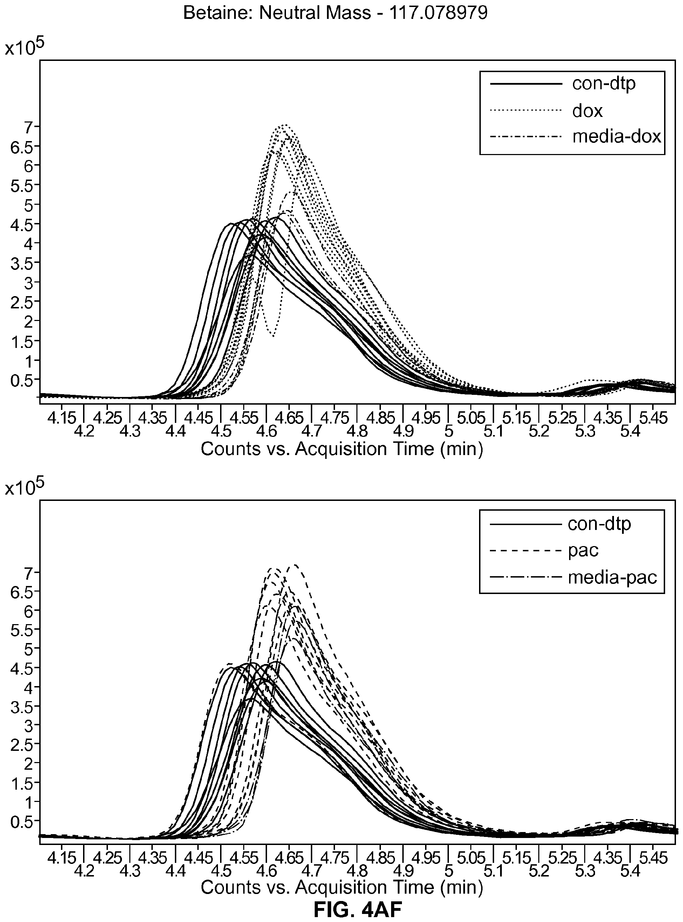

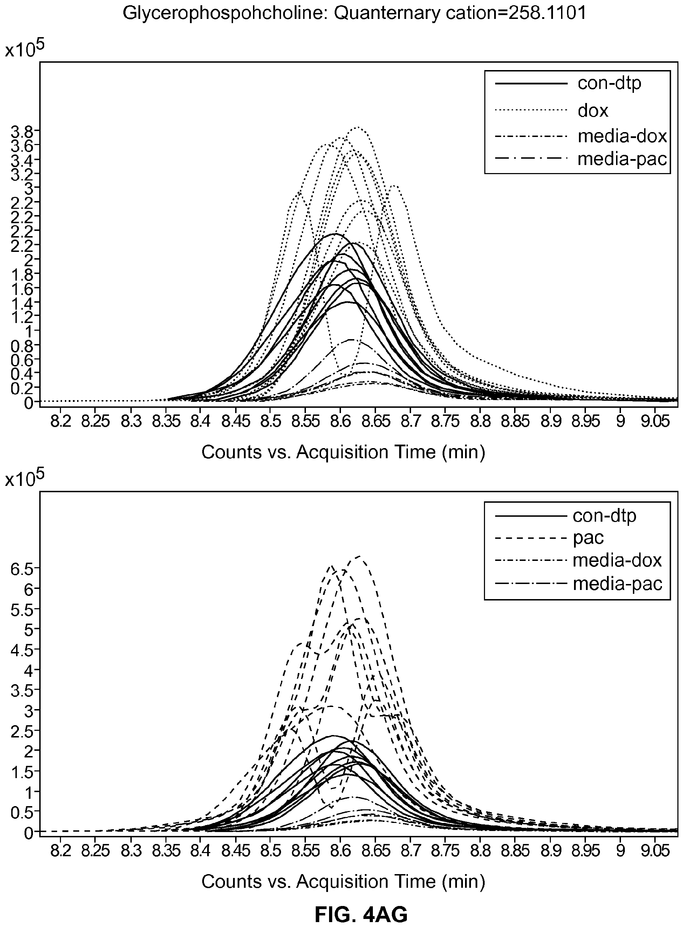

Figures 4A through 4AG are ion extracted chromatograms (EICs) from statistically significant mass features (i.e., candidate metabolite biomarkers of cardiotoxicity) detected in human cardiomyocytes treated with 26 µM doxorubicin (dotted lines) in comparison to untreated controls (solid black lines) and doxorubicin media (refer to legend in graph for line designations) and cardiomyocytes treated with 15 µM of paclitaxel (dashed lines) in comparison to untreated controls (solid black lines) and media (refer to legend). Doxorubicin (DOX), paclitaxel (PAC), and tamoxifen (TAM).

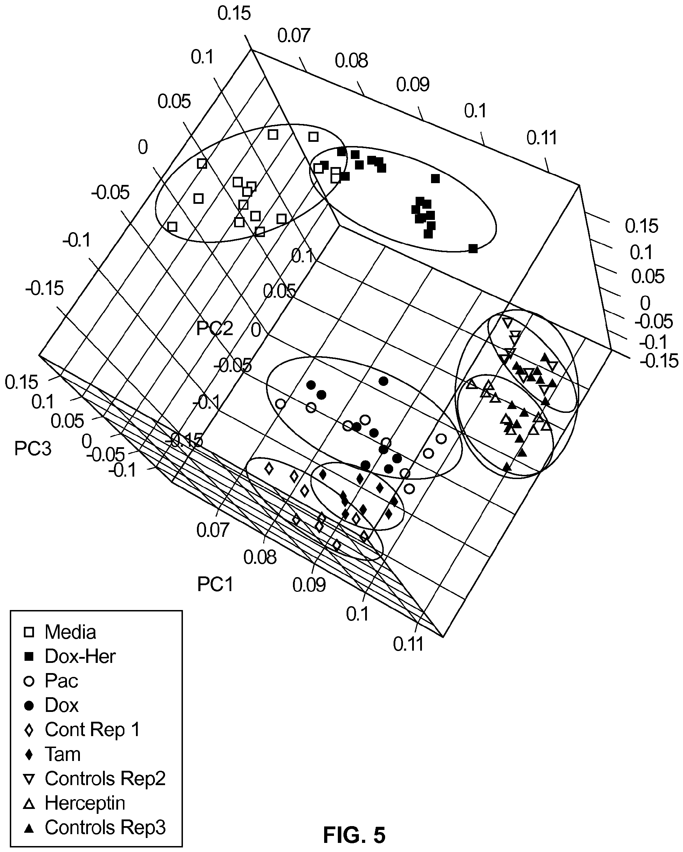

Figure 5 is a depiction of hierarchical clustering of the metabolomic features following various experimental treatments. The NIPALS Principal Cluster Analysis (PCA) illustrates strong cardiotoxicants (DOX, PAC) exhibiting similar trends (clustering) in comparison to weak cardiotoxicants (TAM). Doxorubicin (DOX), paclitaxel (PAC), tamoxifen (TAM), and Herceptin (HER).

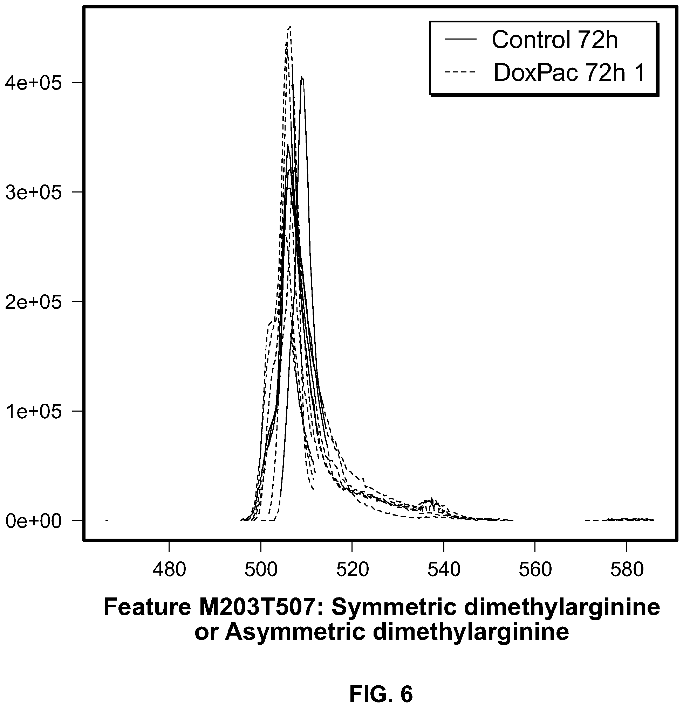

Figure 6 is an ion extracted chromatogram of statistically significant mass feature M203T507 in the cell culture media of cardiac precursor cells treated with doxorubicin (26uM) for 24 hours and then paclitaxel (15 uM) for 48 hours. The EIC demonstrates a statistically significant decrease in the accumulation of Symmetric dimethylarginine or Asymmetric dimethylarginine in treated cardiac precursors. Y-axis is intensity and X-axis is time in seconds.

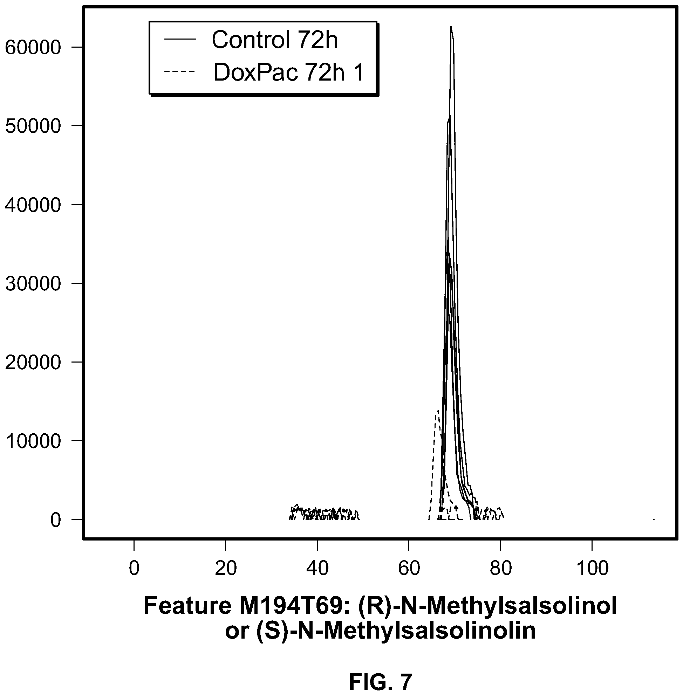

Figure 7 is an ion extracted chromatogram of mass feature M194T69 in the cell culture media of cardiac precursor cells treated with doxorubicin (26uM) for 24 hours and then paclitaxel (15 uM) for 48 hours. The EIC demonstrates a lack of (R)-N-Methylsalsolinol or (S)-N-Methylsalsolinolin the cell culture media of treated cardiac precursors. Y-axis is intensity and X-axis is time in seconds.

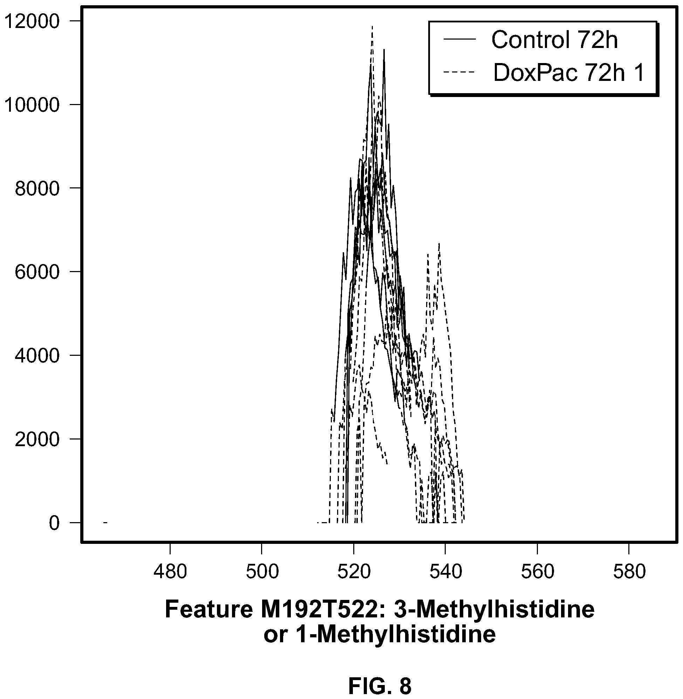

Figure 8 is an extracted ion chromatogram of statistically significant mass feature M192T522 in the cell culture media of cardiac precursor cells treated with doxorubicin (26uM) for 24 hours and then paclitaxel (15 uM) for 48 hours. The EIC demonstrates a statistically significant decrease in the accumulation of 3-Methylhistidine or 1-Methylhistidine in the cell culture media of treated cardiac precursors. Y-axis is intensity and X-axis is time in seconds.

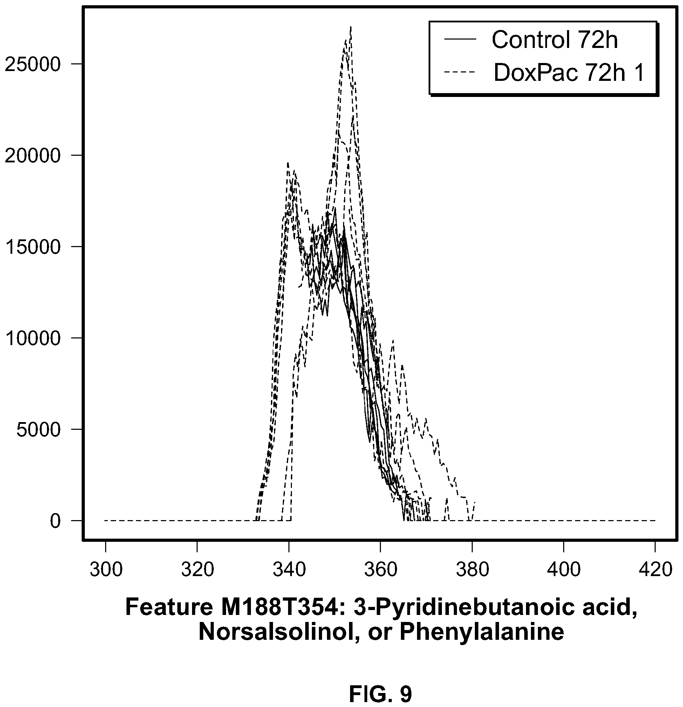

Figure 9 is an ion extracted chromatogram of statistically significant mass feature M188T354 in the cell culture media of cardiac precursor cells treated with doxorubicin (26uM) for 24 hours and then paclitaxel (15 uM) for 48 hours. The EIC demonstrates a statistically significant increase in the accumulation of 3-Pyridinebutanoic acid, Norsalsolinol, or Phenylalanine in the cell culture media of treated cardiac precursors. Y-axis is intensity and X-axis is time in seconds.

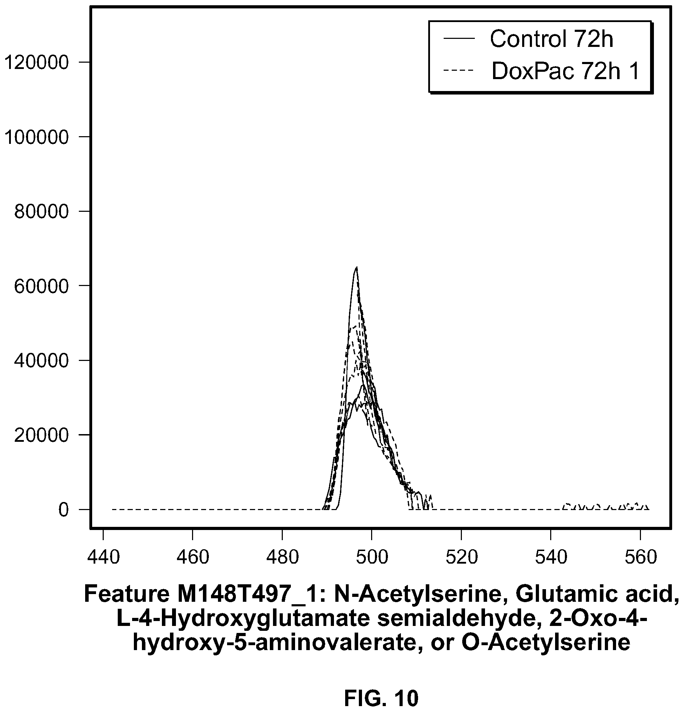

Figure 10 is an ion extracted chromatogram of statistically significant mass feature M148T497_1 in the cell culture media of cardiac precursor cells treated with doxorubicin (26uM) for 24 hours and then paclitaxel (15 uM) for 48 hours. The EIC demonstrates a statistically significant increase in the accumulation of N-Acetylserine, Glutamic acid, L-4-Hydroxyglutamate semialdehyde, 2-Oxo-4-hydroxy-5-aminovalerate, or O-Acetylserine in the cell culture media of treated cardiac precursors. Y-axis is intensity and X-axis is time in seconds.

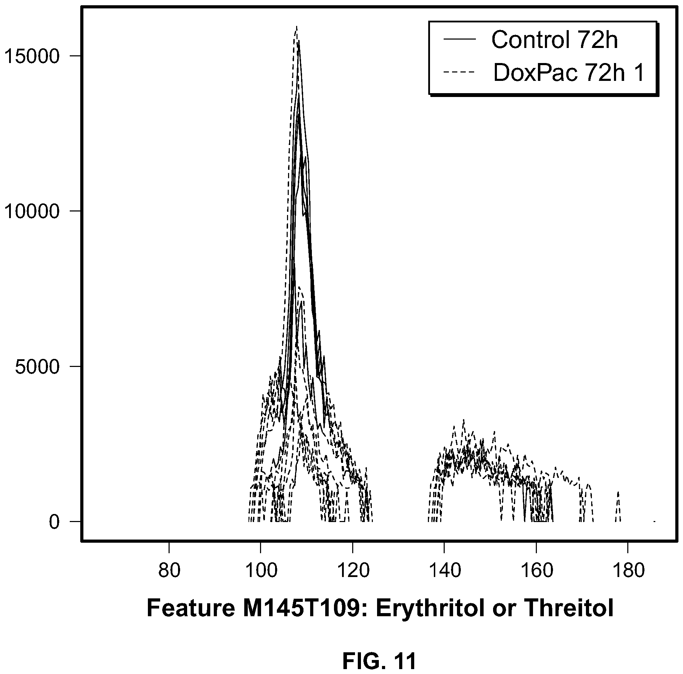

Figure 11 is an extracted ion chromatogram of statistically significant mass feature M145T109 in the cell culture media of cardiac precursor cells treated with doxorubicin (26uM) for 24 hours and then paclitaxel (15 uM) for 48 hours. The EIC demonstrates a statistically significant decrease in the accumulation of Erythritol or Threitol in the cell culture media of treated cardiac precursors. Y-axis is intensity and X-axis is time in seconds.

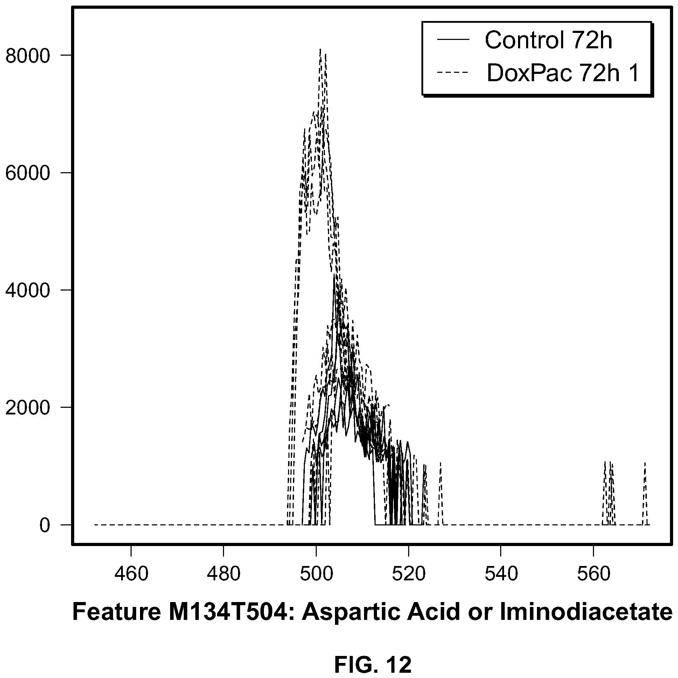

Figure 12 is an extracted ion chromatogram of statistically significant mass feature M134T504 in the cell culture media of cardiac precursor cells treated with doxorubicin (26uM) for 24 hours and then paclitaxel (15 uM) for 48 hours. The EIC demonstrates a statistically significant decrease in the accumulation of Aspartic Acid or Iminodiacetate in the cell culture media of treated cardiac precursors. Y-axis is intensity and X-axis is time in seconds.

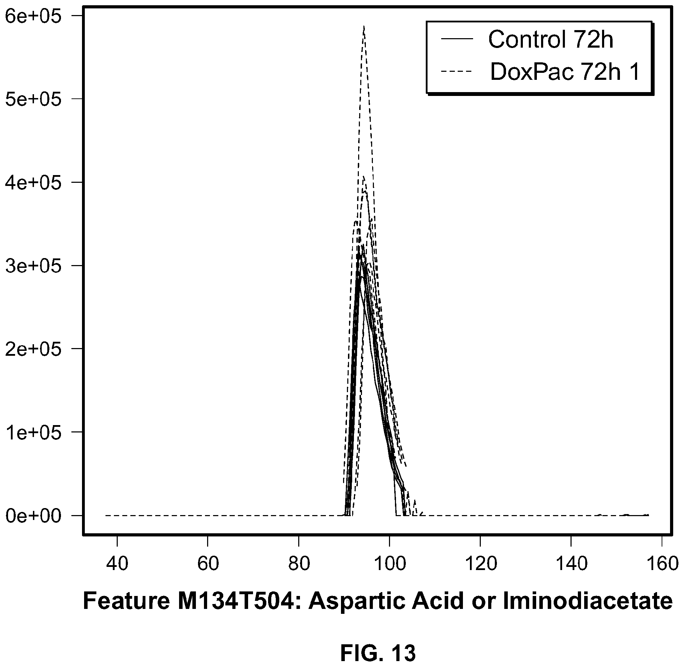

Figure 13 is an extracted ion chromatogram of statistically significant mass feature M134T504 in the cell culture media of cardiac precursor cells treated with doxorubicin (26uM) for 24 hours and then paclitaxel (15 uM) for 48 hours. The EIC demonstrates a statistically significant decrease in the accumulation of Aspartic Acid or Iminodiacetate in the cell culture media of treated cardiac precursors. Y-axis is intensity and X-axis is time in seconds.

### Detailed Description of Preferred Embodiments

This invention is more particularly described below and the Examples set forth herein are intended as illustrative only, since numerous modifications and variations therein will be apparent to those skilled in the art.

As used in the description herein and throughout the claims that follow, the meaning of "a", "an", and "the" includes plural reference unless the context clearly dictates otherwise. The terms used in the specification generally have their ordinary meanings in the art, within the context of the invention, and in the specific context where each term is used. Some terms have been more specifically defined below to provide additional guidance to the practioner regarding the description of the invention. In particular, the term "cell" as used herein can be singular or plural, but in a preferred embodiment is plural.

In one aspect, the disclosure includes reagents and methods for determining the cellular and/or biochemical effects of exposure to cardiotoxic compounds. The term "cellular metabolite" or the plural form, "cellular metabolites," as used herein refers to a low molecular weight molecule secreted by a cell. In general the size of the metabolites is in the range of about 55 Daltons to about 1500 Daltons. A cellular metabolite may include but is not limited to the following types of low molecular weight molecules: acids, bases, lipids, sugars, glycosides, amines, organic acids, lipids, amino acids, oximes, esters, dipeptides, tripeptides, fatty acids, cholesterols, oxysterols, glycerols, steroids, and/or hormones. In an alternative aspect, the cellular metabolite is secreted from cardiomyocytes, human embryonic stem cell (hESC)-derived cardiomyocytes or human induced pluripotent stem cell (iPS)-derived cardiomyocytes. In a preferred embodiment, the cellular metabolites include but are not limited to the following low molecular weight molecules: Triethylamine; NN-Diethylamine; Hexylamine; p-Glucosyloxymandelonitrile; (s)-4-Hydroxymandelonitrilebeta-D-glucoside; 13, 14-dihydro PGE1 (Prostaglandin E1); 7-Ketocholesterol; 1,25-Dihydroxyvitamin D3-26,23-lactone; Formononetin 7-O-glucoside-6"-O-malonate; Isochlorogenic acid b; 13-Dicaffeoylquinic acid; 3-Hexaprenyl-4-hydroxy-5-methoxybenzoic acid; 2-Phenylglycine; (E)-4-Hydroxyphenylacetaldehyde-oxime; (Z)-4-Hydroxyphenylacetaldehyde-oxime; Betaine; 2-Ethylhexyl-4-hydroxybenzoate; Glycerophosphocholine; N-Acetylgalactosamine; CGP52608; Biotin; DL-Homocystine; Ethenodeoxyadenosine; Queuine; N-Acetylaspartylglutamic acid; Tetrahydrocortisone; Cyclic Phosphatidic acid; 2-Methoxyestrone3-glucuronide; Diacylglycerol; Quercetin3-(2G-xylosylrutinoside); Niacinamide; Aspartic Acid; Iminodiacetate; Erythritol; D-Threitol; N-Acetylserine; L-Glutamic acid; L-4-Hydroxyglutamate semialdehyde; 2-Oxo-4-hydroxy-5-aminovalerate; O-Acetylserine; DL-Glutamate; DL-Glutaminic acid; 2-Aminoglutaric acid; Glutamate; D-Glutamic acid; 3-Pyridinebutanoic acid; Norsalsolinol; D-Phenylalanine; D-alpha-Amino-beta-phenylpropionic acid; L-Phenylalanine; 3-Methylhistidine; 1-Methylhistidine; (R)-N-Methylsalsolinol; (S)-N-Methylsalsolinol; Symmetric dimethylarginine; or Asymmetric dimethylarginine.

The phrase "identifying one or a plurality of cellular metabolites ... differentially produced" as used herein includes but is not limited to comparisons of cells exposed to a test compound to untreated (*i.e.,* control) cells. Detection or measurement of variations in low molecular weight molecule populations secreted by a cell, between experimental and control cells are included in this definition. As used herein, the terms "secrete," "secreting," and "secretion" are intended to encompass any cellular process by which a cellular metabolite produced by a cell is translocated outside the cell. Metabolites or small molecules, particularly those species secreted, excreted or consumed by the cells, or those metabolites that are fluxed through the cells, that participate in functional mechanisms of cellular response to pathological or chemical insult. Metabolites may also be produced as a result of apoptosis or necrosis.

In a preferred aspect, alterations in cells or cell activity are measured by determining a profile of changes in low molecular weight molecules in treated versus untreated cells. Also included are comparisons between cells treated with different amounts, types or concentrations, durations or intensities of cardiotoxic or potential cardiotoxic compounds.

Alterations in cellular metabolites such as sugars, organic acids, amino acids, fatty acids, and low molecular weight compounds are measured and used to assess the effects of specific pharmaceuticals, environmental agents, chemical compounds and biologic therapies on biochemical pathways in cardiomyocytes. The screened low molecular weight compounds (*i.e.,* metabolites) are secreted in response to a variety of biological activities, including, but not limited to inflammation, anti-inflammation, vasodilation, neuroprotection, fatty acid metabolism, collagen matrix degradation, oxidative stress, antioxidant activity, DNA replication and cell cycle control, methylation, biosynthesis of nucleotides, carbohydrates, amino acids and lipids, among others. Secreted low molecular weight molecules are precursors, intermediates and/or end products of *in vivo* biochemical reactions. Alterations in specific subsets of molecules correspond to a particular biochemical pathway and thus reveal the biochemical effects of cardiotoxicity.

The term "cardiomyocyte" or "cardiomyocyte cell(s)" as described herein refers to primary cardiomyocytes, cardiomyocyte precursor cells, clonal cardiomyocytes derived from adult human heart, immortalized cardiomyocytes, human embryonic stem cell (hESC)-derived cardiomyocytes, human induced pluripotent stem cell (iPS)-derived cardiomyocytes, or any cell displaying cardiomyocyte-specific markers such that a pathologist, scientist, or laboratory technician would recognize the cell to be cardiomyocyte-specific or cardiomyocyte derived.

The term "cardiotoxic" as described herein refers to a substance or treatment, particularly pharmaceuticals, biologics, and other chemical compounds and environmental agents, that induce cardiomyopathy, heart disease, and/or abnormal heart pathology and physiology. Examples of cardiotoxicities encompassed by the definition of the term as used herein include heart abnormalities that would be recognized by a physician, cardiologist, or medical researcher, which could be attributed to or a potential result of a drug-treatment regimen.

In a preferred embodiment the term "compound" or "test compound" includes but is not limited to pharmaceuticals, environmental agents, chemical compounds and biologic therapies, including antibody-based treatments, vaccines, or recombinant proteins and enzymes. In a particularly preferred embodiment, cardiotoxic compounds include tamoxifen, doxorubicin, and paclitaxel. In a further embodiment, potentially cardiotoxic compounds are screened for metabolite similarities to already known cardiotoxic compounds.

The term "cardiomyopathy" refers to heart disease, including but not limited to inflammation of the heart muscle and reduction of heart function. Cardiomyopathy can be classified as primary or secondary and may further include dilated, hypertrophic and restrictive cardiomyopathies. The heart cavity can be enlarged and stretched (*e.g*., cardiac dilation), and may not pump normally. Abnormal heart rhythms called arrhythmias and disturbances in the heart's electrical conduction also can occur. In this condition, the muscle mass of the left ventricle enlarges or "hypertrophies."

Mass spectrometry-based platforms have been proposed as a means to select peptides and proteins, but not small-molecule metabolites, as candidate biomarkers of cardiotoxicity. For example, brain natriuretic peptide (BNP) and N-terminal proBNP (NTproBNP) are clinical biomarkers of heart failure. BNP hormone and the inactive NTproBNP are predominantly secreted in the ventricles of the heart in response to pressure overload and, consequently, are being investigated as markers of drug-induced cardiac hypertrophy in rat. (*See* Berna et al., 2008, Anal Chem 80: 561-566). In addition, myosin light chain 1 (Myl3), a 23-kDa isoform of one of the subunits of myosin and troponin have been proposed as biomarkers of cardiac necrosis to predict drug-induced cardiotoxicity (*See* Adamcova et al., 2005, Expert Opinion on Drug Safety 4(3): 457-472). Such peptides and proteins have been recognized in the art as products of the degenerative changes in heart muscle associated with cardiomyopathies.

Certain of the compounds used herein to demonstrate the usefulness of a metabolomics approach for identifying candidate biomarkers for cardiotoxicity in cardiomyocytes are known cardiotoxic compounds. These compounds are thus illustrative of the reagents and methods for detecting metabolomic markers for cardiotoxicity, and include doxorubicin, paclitaxel and tamoxifen. The assessment of low molecular weight molecule metabolic products secreted by cardiomyocytes in response to exposure to multiple drug-treatment regimens thus provides novel profiles of candidate biomarkers of cardiotoxicity that can be rationalized with these clinical indicia.

The term "control cell(s)" as used herein refers in general to non-cardiac derived cell types. In a preferred aspect, control cells include human fibroblasts.

The term "control cardiomyocytes" as used herein refers to cardiomyocyte or cardiomyocyte-derived cells that are exposed to control conditions.

The term "control sets" as used herein refers to the exposure of a particular cell type to a condition that one of skill in the art would recognize as a control treatment. In a preferred aspect this includes but is not limited to the following experimental conditions: the exposure of cardiac cells to non-toxic compounds, or the exposure of non-cardiac cells to cardiotoxic compounds. Conversly, as used herein, an "experimental set" includes cardiac-specific cells exposed to a compound of interest (e.g., test compound), such as specific pharmaceuticals, biologics, and other chemical compounds and environmental agents.

The term "subtracting" as used herein refers to the identification of common cellular metabolites secreted by experimental cells and control cells followed by the selective removal of those metabolites in common from a metabolic signature or biomarker profile of specific cardiotoxic response.

When identifying low molecular weight metabolites that are secreted by cardiomyocytes, a skilled technician or scientist would understand that such metabolites can be measured, for example, those metabolites secreted and/or released into cellular supernatant and/or present in cellular extracts, as well as a variety of other methods available for the assessment of secreted molecules. Identified metabolites may also be waste products excreted by cells.

The phrase "exposure to test compound" may refer to cell samples exposed to an individual compound separately or a plurality of compounds sequentially and/or collectively. In one embodiment, cells are exposed to an individual test compound. In a further embodiment, cells are exposed to multiple compounds. In an alternative embodiment, cells are not exposed to any compound (*i.e.,* control). Cells may be cultured in the presence or absence of test compounds.

The phrase "selecting those with commonality" as used herein refers to secreted metabolites produced in commonality across more than one set of cells. Thus, for example, the metabolites in various cell sets are identified, compared, and those in common may be further selected for commonality.

The term "physical separation method" as used herein refers to any method known to those with skill in the art sufficient to produce a profile of changes and differences in low molecular weight molecules produced by cells exposed to pharmaceuticals, environmental agents, chemical compounds and biologic therapies according to the methods of this invention. In certain aspects, physical separation methods permit detection of low molecular weight molecules including but not limited to acids, bases, lipids, sugars, glycosides, amines, organic acids, lipids, amino acids, oximes, esters, dipeptides, tripeptides, fatty acids, cholesterols, oxysterols, glycerols, steroids, and/or hormones. In particular aspects, this analysis is performed by liquid chromatography high resolution mass spectrometry (LC-MS) and/or liquid chromatography/electrospray ionization time of flight mass spectrometry (LC-ESI-TOF-MS), however it will be understood that low molecular weight compounds as set forth herein can be detected using alternative spectrometry methods or other methods known in the art. For example, nuclear magnetic resonance (NMR) is another method that can identify low molecular weight compounds. Similar analyses have been applied to other biological systems in the art (Want et al., 2005, Chem Bio Chem 6:1941-51), providing biomarkers of disease or toxic responses that can be detected in biological fluids (Sabatine et al., 2005, Circulation 112: 3868-875). It is understood that different instruments may detect different low molecular weight compounds. Thus, for example, the profile developed by LC-MS and/or LC-ESI-TOF-MS may be the same as or different than the profile developed by NMR.

A "biological sample" includes but is not limited to cells cultured *in vitro,* a patient sample, or biopsied cells dispersed and cultured *in vitro.* A "patient" may be a human or animal. A "patient sample" includes but is not limited to blood, plasma, serum, lymph, urine, cerebrospinal fluid, saliva or any other biofluid or waste.

The term "biomarker" as used herein refers, *inter alia* to low molecular weight compounds as set forth herein that exhibit significant alterations between experimental cell sets and control cell sets, particularly with regard to exposure to cardiotoxic compounds. The present invention employs mass spectrometry. In certain embodiments, biomarkers are identified as set forth above, by methods including, for example, LC-MS and/or LC-ESI-TOF-MS. In certain embodiments, the following low molecular weight molecules are employed herein, taken alone or in any informative combination, as biomarkers of cardiotoxicity: Triethylamine; NN-Diethylamine; Hexylamine; p-Glucosyloxymandelonitrile; (s)-4-Hydroxymandelonitrilebeta-D-glucoside; 13, 14-dihydro PGE1 (Prostaglandin E1); 7-Ketocholesterol; 1,25-Dihydroxyvitamin D3-26,23-lactone; Formononetin 7-O-glucoside-6"7-O-malonate; Isochlorogenic acid b; 13-Dicaffeoylquinic acid; 3-Hexaprenyl-4-hydroxy-5-methoxybenzoic acid; 2-Phenylglycine; (E)-4-Hydroxyphenylacetaldehyde-oxime; (Z)-4-Hydroxyphenylacetaldehyde-oxime; Betaine; 2-Ethylhexyl-4-hydroxybenzoate; Glycerophosphocholine; N-Acetylgalactosamine; CGP52608; Biotin; DL-Homocystine; Ethenodeoxyadenosine; Queuine; N-Acetylaspartylglutamic acid; Tetrahydrocortisone; Cyclic Phosphatidic acid; 2-Methoxyestrone3-glucuronide; Diacylglycerol; Quercetin3-(2G-xylosylrutinoside); Niacinamide; Aspartic Acid; Iminodiacetate; Erythritol; D-Threitol; N-Acetylserine; L-Glutamic acid; L-4-Hydroxyglutamate semialdehyde; 2-Oxo-4-hydroxy-5-aminovalerate; O-Acetylserine; DL-Glutamate; DL-Glutaminic acid; 2-Aminoglutaric acid; Glutamate; D-Glutamic acid; 3-Pyridinebutanoic acid; Norsalsolinol; D-Phenylalanine; D-alpha-Amino-beta-phenylpropionic acid; L-Phenylalanine; 3-Methylhistidine; 1-Methylhistidine; (R)-N-Methylsalsolinol; (S)-N-Methylsalsolinol; Symmetric dimethylarginine; or Asymmetric dimethylarginine. In a preferred embodiment, the low molecular weight molecules described herein in Tables 2A-2D taken alone or in any informative combination, are reliable biomarkers of cardiotoxicity. Many of the identified low molecular weight molecules are identified by unique mass feature size or neutral mass, however some molecules are further identified by compound name.

The terms "metabolic signature" and "metabolic profile" as used herein refer to one or a plurality of metabolites identified by the inventive methods. Metabolic signatures and profiles can provide a molecular "fingerprint" of the effects of cardiotoxicity and identify low molecular weight compounds significantly altered following exposure to pharmaceuticals, environmental agents, chemical compounds and biologic therapies that are cardiotoxic. In certain aspects, metabolic signatures or metabolic profiles can be used to predict cardiotoxicity of a compound. In an alternative aspect, a metabolic signature or profile may diagnose cardiotoxic effects from drug treatment regimens, pharmaceuticals, environmental agents, chemical compounds or biologic therapies.

In certain aspects, cardiotoxicity of a test compound can be identified by cardiomyocyte secretion of a single known cardiotoxic biomarker. As an example, a single marker may include Betaine or Glycerophosphocholine. This may include metabolite(s) secreted in response to exposure to a single established cardiotoxic compound (e.g. doxorubicin). In other aspects, cardiotoxicity is affirmed by detection of a metabolic signature (i.e., one or a plurality of low molecular weight metabolites) commonly produced by cardiomyocytes in response to two or more known cardiotoxic compounds (*e.g*., doxorubicin and paclitaxel, or doxorubicin, paclitaxel, and tamoxifen). In further aspects, metabolic signatures of cardiotoxicity comprising one or a plurality of cellular metabolites provided in Tables 2A-2D, or described in the chromatograms of Figures 4A-AG and Figures 6-13 are provided.

Data for statistical analysis were extracted from chromatograms using the Agilent Mass Hunter software (Product No. G3297AA, Agilent Technologies, Inc., Santa Clara, CA); it will be understood that alternative statistical analysis methods can be used. Masses were binned together if they were within 10 ppm and eluted within a 2 minutes retention time window. A binned mass was considered to be the same molecule across different LC-ESI-TOF-MS analyses (referred to herein as an "exact mass," which will be understood to be ± 10ppm). Binning of the data is required for statistical analysis and comparison of masses across the entire experiment. If multiple peaks with the same mass at the same retention time within a single sample were detected by Mass Hunter, they were averaged to assist data analysis. Masses lacking a natural isotopic distribution or with an absolute height of less than 1000 were removed from the data prior to analysis. It would be understood that the results from this assay provide relative values that are assessed according to annotated values within 10 ppm to provide an identity for the molecular weight detected. Thus, a mass shift within 10 ppm is considered consistent with determining the identity of a specific cellular metabolite previously annotated due to differences in ionization source and instrumentation, e.g. between different experiments or using different instruments.

As used herein, a mass was considered to be the same across LC/ESI-TOF-MS runs using a simple algorithm that first sorts the data by mass and retention time. After sorting, a compound was considered unique if it had an ordered retention time difference of less than or equal to 0.1 minutes and a mass difference less than or equal the weighted formula: consecutive masses did not differ by 10 ppm if under 175 Da, by 7 ppm over the range 175 to 300 Da, and by 5 ppm when greater 300 Da. If a series of measurements fit this definition it was considered to be from the same compound. If either the mass or the retention time varied by more than the limits listed above it was considered to be a different compound and given a new unique designation.

The data from the most reproducible mass features was log base 2 transformed and median centered prior to statistical analysis. Statistical analysis was performed using the open source statistical programming and analysis software R. Statistical significance of individual mass features were performed under the null hypothesis that no difference in abundance exists between control and drug treatment using a permutation based test statistic or a Welch T-test. To test the null hypothesis a one way permutation based t-test assuming a normal approximation of the conditional distribution was used and implement using the Conditional Inference Procedures in a Permutation Test Framework (Coin) library, a contributed package of programming code. Statistics tests were performed without replacement of missing values decrease the degrees of freedom rather than imputing missing values. This oneway test method is ideally suited for analysis of complex data sets where one may not be able to assume that every feature tested will have a normal distribution (Hothorn et al., 2006, Amer. Statistician, 60:257-263). False discovery rates (FDR) were controlled using the Q value estimator (Storey et al., 2003, Proc Natl Acad Sci., 100:9440-5) and implemented using the qvalue library in R (Dabney *et al.,* 2003, qvalue: Q-value estimation for false discovery rate control. R package version 1.10.0., www.CRAN.R-project.org).

In certain aspects, a cardiotoxic biomarker may reference one or a collection of cellular metabolites produced by cardiomyocytes following exposure to known cardiotoxins. A cardiotoxic metabolic signature can comprise about 1, or about 6, or about 10, or about 20, or about 30 differentially secreted low molecular weight molecules, and while the cardiotoxic signature as disclosed herein comprises from about 1 to about 30 metabolites and includes the low molecular weight molecules set forth in Table 2A-2D herein, said cardiotoxic signature generally comprises a sufficient number of metabolites to independently identify an experimental test compound as being cardiotoxic. It will be understood by those with skill in the art that the differential fold change in metabolite secretion between untreated and treated cells can vary for each metabolite.

### Examples

The Examples which follow are illustrative of specific embodiments of the invention, and various uses thereof. They set forth for explanatory purposes only, and are not to be taken as limiting the invention.

### Example 1

### Verification of Cardiac-Specific Cells and Measurement of Cardiac Cell Death After Exposure to Cardiotoxic Agents

Human cardiomyocytes, clonal cardiomyocytes derived from adult human heart (Celprogen 36044-15at, San Pedro, CA) or cardiac precursor cells, were treated with varying doses of pharmacological compounds known to have cardiotoxic effects. Cardiomyocytes were treated with doxorubicin and paclitaxel, which are strong toxicants, as well as tamoxifen, a weak toxicant, for 24 or 48 hours. Some combinatorial treatments regimens appeared to exhibit synergistic cardiotoxic effects (*e.g.,* for doxorubicin and trastuzumab combined therapies, *see* Pentassuglia et al., 2007, Experimental Cell Research 313: 1588-1601; for paclitaxel and doxorubicin combined therapies, *see* Robert, 2007, Cardiovasc Toxicol 7: 135-139)).

The cardiac origin of these cells was confirmed by immunohistochemistry using antibodies against cardiac alpha-actin protein (Figure 1). The percentage of cell death, inherently and after drug treatment, was calculated by Trypan Blue staining (Figure 2). Cell death was significantly higher in human cardiomyocytes treated with doxorubicin or paclitaxel (50 - 55%) in comparison to tamoxifen (18%) and untreated controls (7%).

### Example 2

### Identification of Metabolites Produced by Cardiomyocytes Exposed to Cardiotoxic Pharmacologics

In order to identify low molecular weight metabolites secreted by cardiomyocytes or cardiac precursors following exposure to cardiotoxic compounds, cells as described above in Example 1 were treated with doxorubicin, paclitaxel, and tamoxifen, for 24 or 48 hours.

The extracellular media from treated and untreated cells was processed as described in Cezar *et al.,* (2007, *Stem Cells Development* 16: 869-882), for extraction of low molecular weight molecules (<3kD) for metabolomics analysis. Extracellular low molecular weight molecule preparations were separated by liquid chromatography followed by electrospray ionization time of flight (LC-ESI-TOF-MS) mass spectrometry for ionization and detection of the full spectra of low molecular weight molecules present in each sample. More specifically, the samples were separated using the ESI_Luna_HILIC_95t06OACN_16min method (HILIC chromatography). Statistical differences were inferred by subsequent bioinformatics and *in silico* mapping of deisotoped ESI-TOF-MS mass features as described below (also provided in Cezar *et al.* (2007, *id*.)).

Briefly, ionization (100m/z-1500 m/z) was acquired on an Agilent 6520 Accurate-Mass Q-TOF in extended dynamic range and positive mode. Mass features were generated using two independent methods. First MassHunter Qualitative Analysis was used to generate mass features using the Molecular Feature Extraction algorithm (MFE). Features generated by MFE were binned in R and analyzed for differential accumulation in response to the drug treatments. The Agilent data files were also converted to mzData file format using Agilent's MassHunter Qualitative Analysis Workstation. The mzData files were analyzed in R using the software library XCMS to find mass feature bins differentially present in the presence of drug. MHD files created by MFE were converted to text files using MassHunterMFE version 44. The MHD text files were loaded into R and meta data corresponding to the file name, cell line (Celprogen Cardiomyocytes or solvent), plate (0, 1, 2 or 3), well (solvent, A, B, or C), experiment replication, cells (supernatant, uncultured media or solvent), cell culture passage number, drug treatment (15uM tamoxifen, 15µM paclitaxel, control, 26µM doxorubicin), feature retention time group, retention time, feature neutral mass, mass feature mass standard deviation, abundance, saturation, height, number of ions in feature, min charge, max charge, charge number, width, and group feature count were added to each file.

In order to identify metabolites secreted by cardiomyocytes in response to cytotoxic drug treatment, metabolomic analysis was performed on cardiomyocytes from similar cell passages. Statistically-significant features that were common between the cytotoxic drug treatments were identified. Mass features that were present in at least 25% of LC-MS samples of control and drug treated cardiomyocytes were selected. The statistical significance of individual mass features was determined under the null hypothesis that no difference in abundance existed between control and drug treatment using a permutation-based test statistic like Students *t*-test. A one-way test assuming a normal approximation of the conditional distribution (*see* Horthon et al., 2006a, The American Statistician 60(3): 257-263) was used to test the null hypothesis and was implemented using the Conditional Inference Procedures in a Permutation Test Framework (Coin) library in R (*see* Horthon et al., 2006b, Conditional Inference Procedures in a Permutation Test Framework, R package version 0.4-5, CRAN.R-project.org). Statistics tests were performed on log base two transformed, median normalized abundance values without replacement of missing values reducing the degrees of freedom when a missing value was present. False discovery rates (FDR) were controlled using the Q value estimator (Storey et al., 2003, Proc. Natl. Acad. Sci. USA 100: 9440-45) with a lambda of 0 and implemented using the q value library in R (Dabney et al., 2003, qvalue: Q-value estimation for false discovery rate control. R package versions 1.10; CRAN.R-project.org; R Development Core Team. R: A Language and Environment for Statistical Computing. Vienna, Austria: R Foundation for Statistical Computing: 2007. ISBN 3-900051-07-0; www.R-project.org). After performing statistics, a universe of statistically-significant mass features was created from the comparisons of control to each drug treatment based on FDR-adjusted *p* values. Boolean logic was utilized to find the statistically significant features in common between the different drug treatments. An intersection of mass features that exhibited statistically significant differences in the drugs affecting cell viability (DOX, PAC), but that were not statistically significant in (TAM) was selected. This intersection represented common mass features that were associated with cardiotoxicity because they exhibited a statistically-significant change in cytotoxic treatments, but no statistically-significant change in non-cytotoxic treatments.

A mass was considered to be the same across LC/ESI-MS runs using a simple algorithm that sorts the data by mass and retention time as performed by the software and methods described above. The criteria used for treated-cells were based on a sliding mass scale to compensate for detector efficiency. Because of flow rate, a mass was considered equivalent if it was within (0.00001 x mass) when under 175 Da, (0.000007 x mass) when 176 Da-300Da, and (0.000005 x mass) when over 300 Da with a retention time difference of 1.5 min. If a series of measurements fit this definition, it was considered to be from the same compound within each experiment. If either the mass retention time varied by more than the limits listed above, the compound was considered to be a different one and given a different bin description. Specifically, 774,645 features were identified by the MassHunter software with an average of 6455 and a median of 5869 features per LC/MS run. The mass features were then sorted by mass and retention time groupings and feature ID bins were created for each set of mass and retention groupings that did not differ.

The neutral exact mass and/or empirical chemical formula of each compound, detected by LC-ESI-TOF-MS, was queried in public searchable databases, METLIN (metlin.crips.edu), The Human Metabolome Database (hmda.ca), Kyoto Encyclopedia of Genes and Genomes (genome.jp/keg), and the Biological Magnetic Resonance Bank (bmrb.wisc.edu/metabolomics) for candidate identities. LC-MS-measured mass signals matched small molecules present in the databases if their exact masses were within 10 parts per million (0.00001 x mass). Exact mass measurements and chemical formulae are generally nonambiguous for small molecules up to a certain size. Analytical-grade chemical standards were purchased from Sigma for comparative LC-MS. Aliquots of conditioned medium used in experiments were spiked with 1 mM chemical standards followed by standard LC-ESI-TOF-MS, as described above. The neutral exact masses and retention times for standard compounds in spiked conditioned medium were used to re-extract peaks in experimental samples using Analyst software (Agilent).

The doses for each compound were based upon published standards, equivalent to therapeutic circulating levels whenever possible (see Table 1). Trypan Blue exclusion/cell death assays using the aforementioned concentrations have shown that these doses corroborate published findings, whereby doxorubicin and paclitaxel induced significantly higher cell death as described in Example 1. (Figure 2).

**Table 1: Dosages of Cardiotoxic Pharmacologics**

| **Compound** | **Dose** | **Exposure** | **Therapeutic levels** |
|---|---|---|---|
| | 15mg/kg | | *Maximum cumulative dose 550mg/m² (Takemura and Fugiwara, 2007, Progress in Cardiovascular Diseases 49(5): 330-352; Kang et al., 2000, The Journal of Biological Chemistry 275(41): 31682-31688; Rahman et al., 2007, International Journal of Nanomedicine 2(4): 567-83). |
| Doxorubicin | 26µM (Han et al., 2008 The Journal of Pharmacology and Experimental Therapeutics 326(1): 127-134). | 24 hours | |
| | 15µM | 48 hours | |
| Paclitaxel | (Alloatti et al., 1998, The Journal of Pharmacology and Experimental Therapeutics 284(2): 561-567; Spencer and Faulds, 1994, Drugs 48(5): 794-847). | | |
| | 15µM | 24 hours | |
| Tamoxifen | (Daosukho *et al.,* | | |
| | 2007, Free Radical Biology & Medicine 42: 1818-1825). | | |

Metabolite trends observed in initial studies are shown in Figure 5, wherein strong cardiotoxic compounds exhibit similar mass features (low molecular weight molecules) and thus cluster together upon unsupervised multivariate analysis (NIPALS Principal Cluster Analysis).

Identified features are provided in Table 2A-2D. Specifically, Table 2A provides identified mass features with commonality between paclitaxel and doxorubicin treatments. Table 2B provides identified mass features with commonality between paclitaxel, doxorubicin, and tamoxifen treatments. Table 2D provides identified mass features secreted from cardiac precursor cells treated with doxorubicin and then paclitaxel.

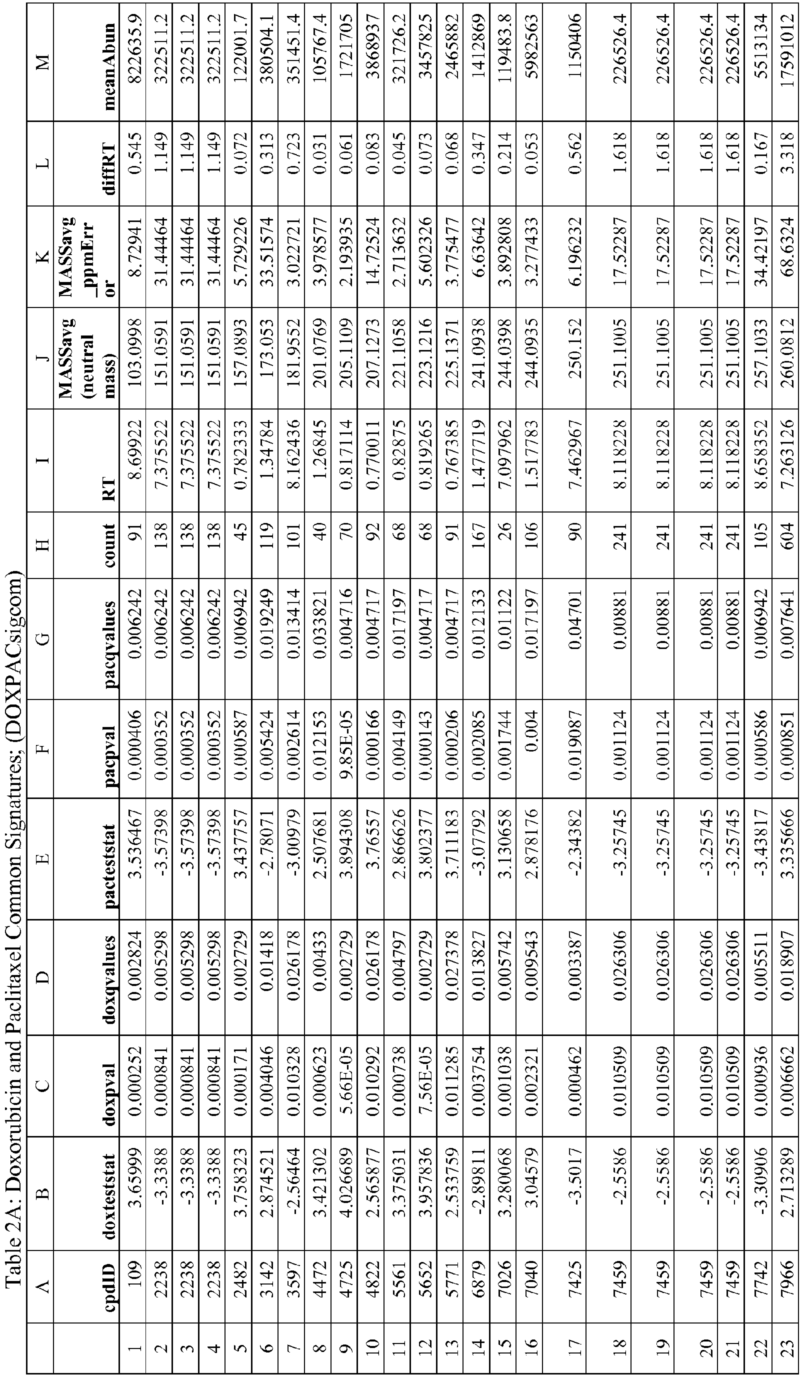

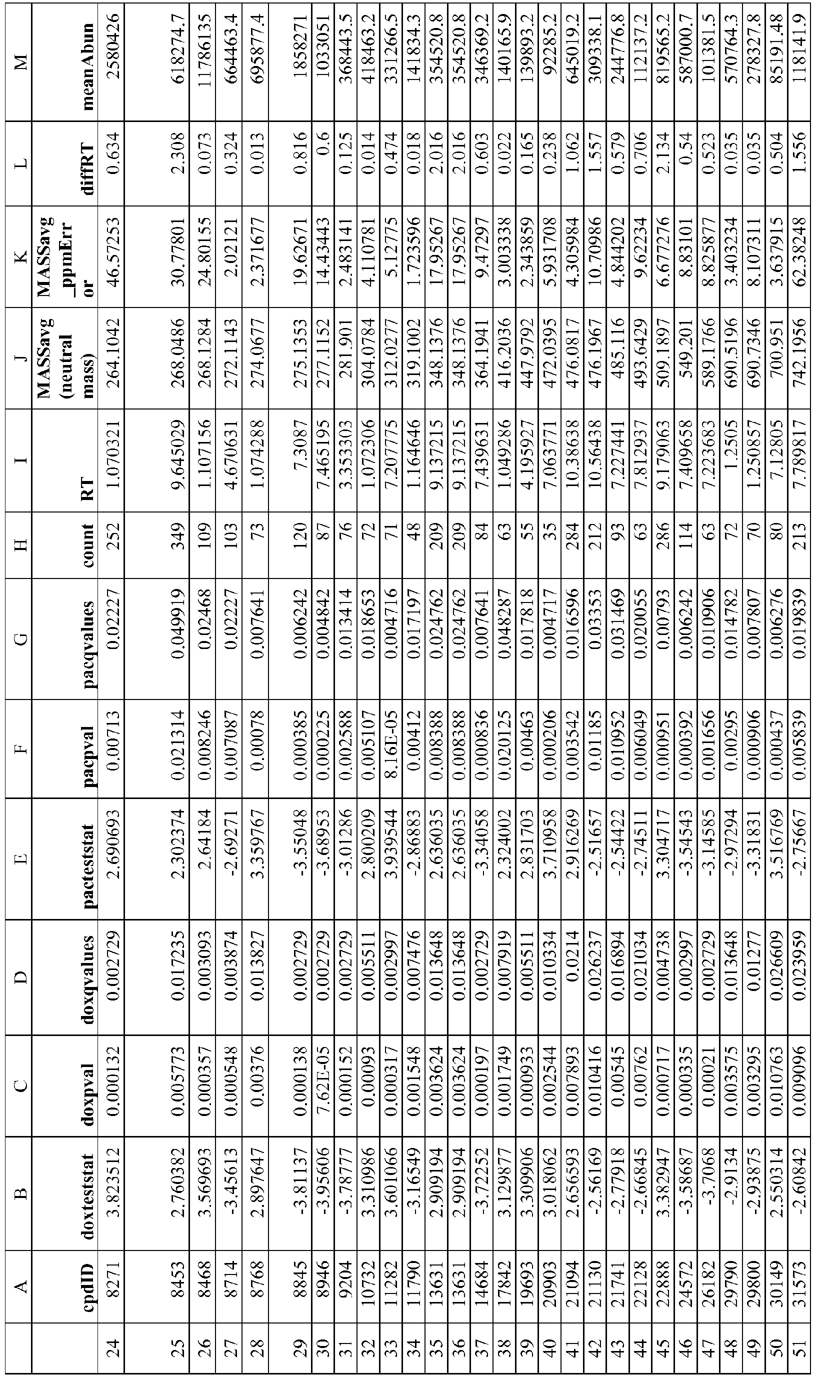

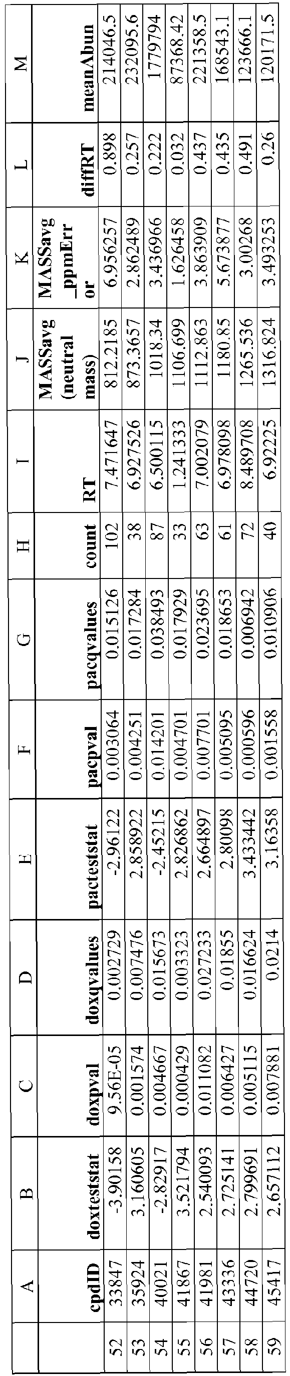

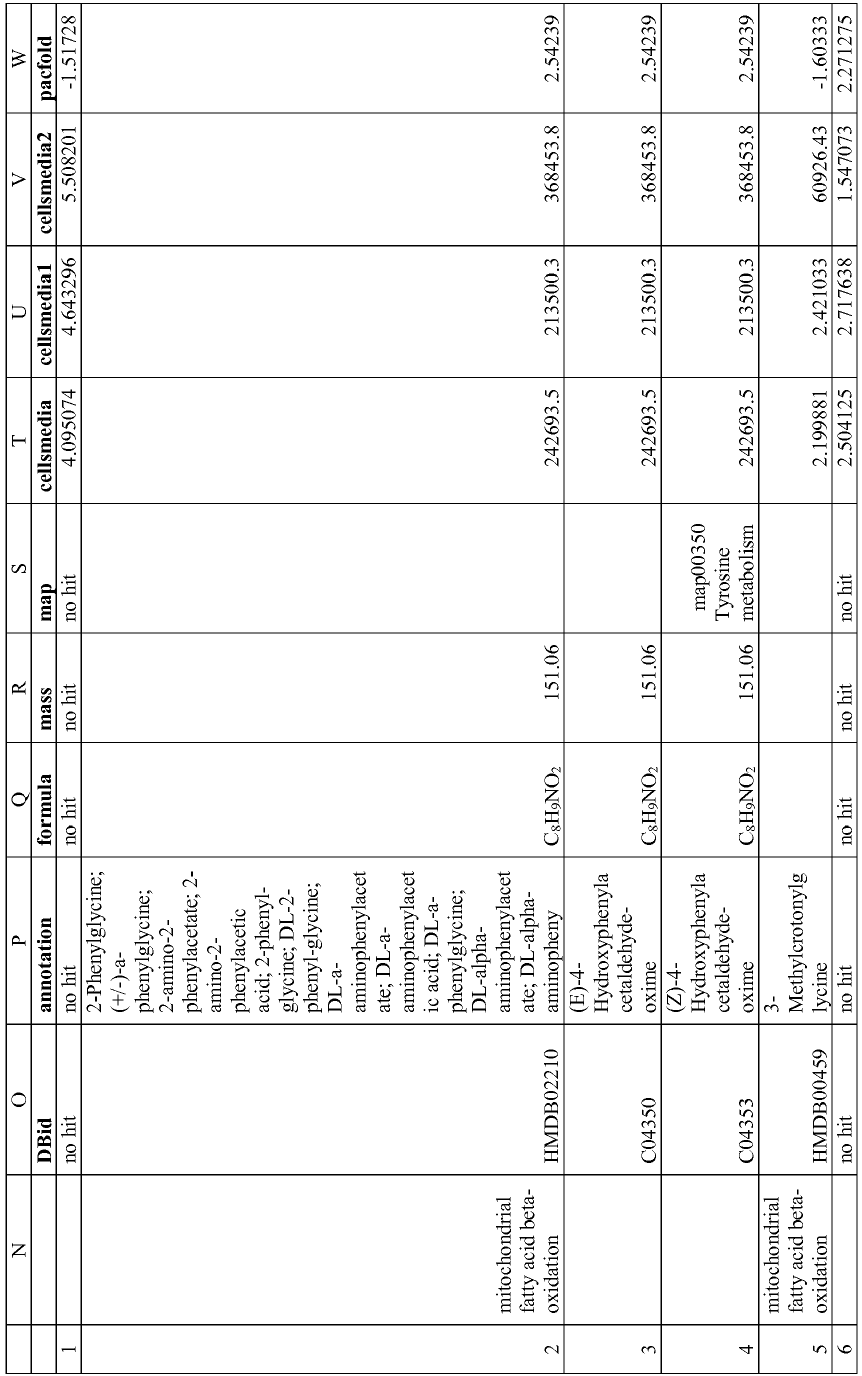

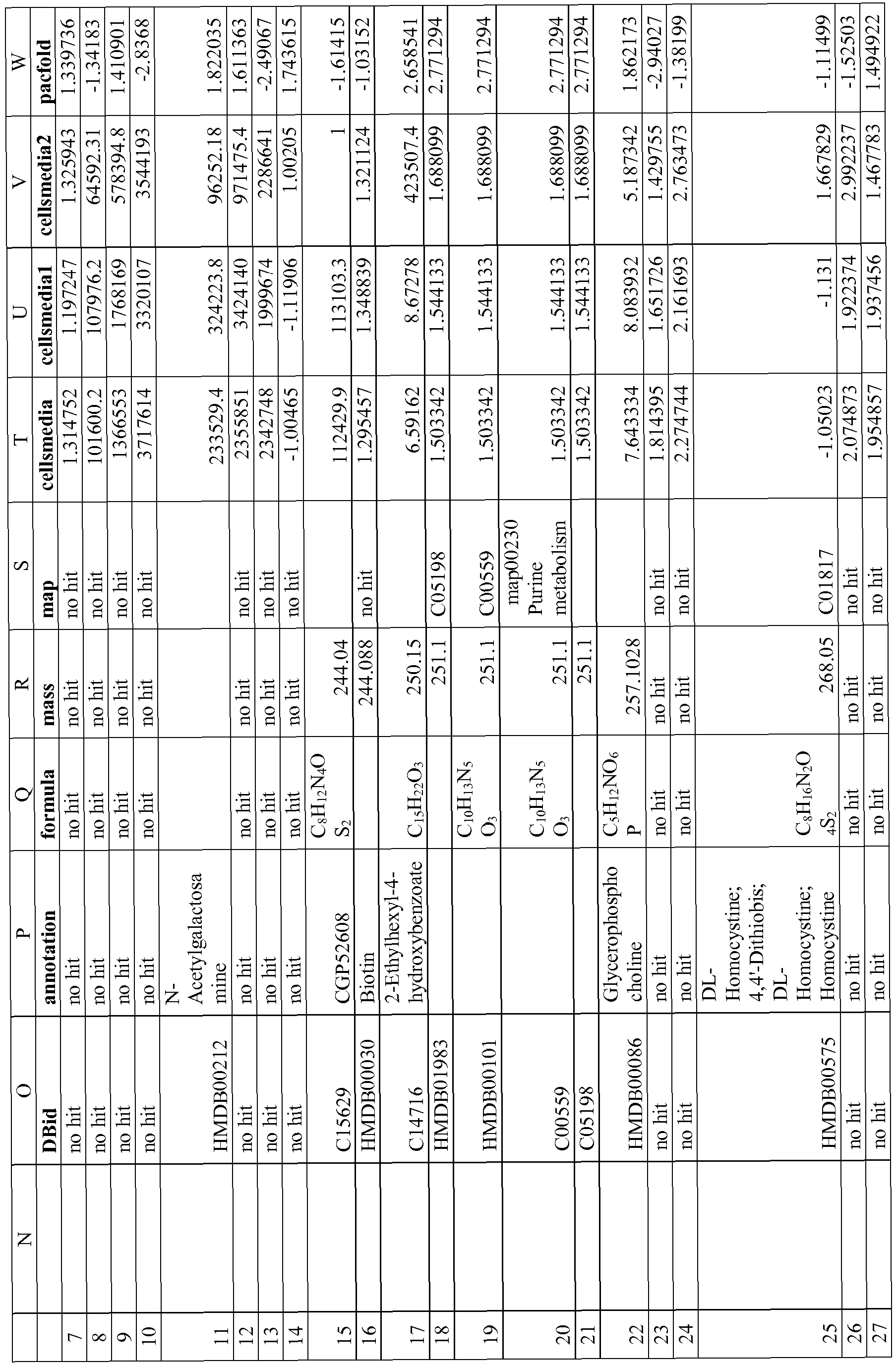

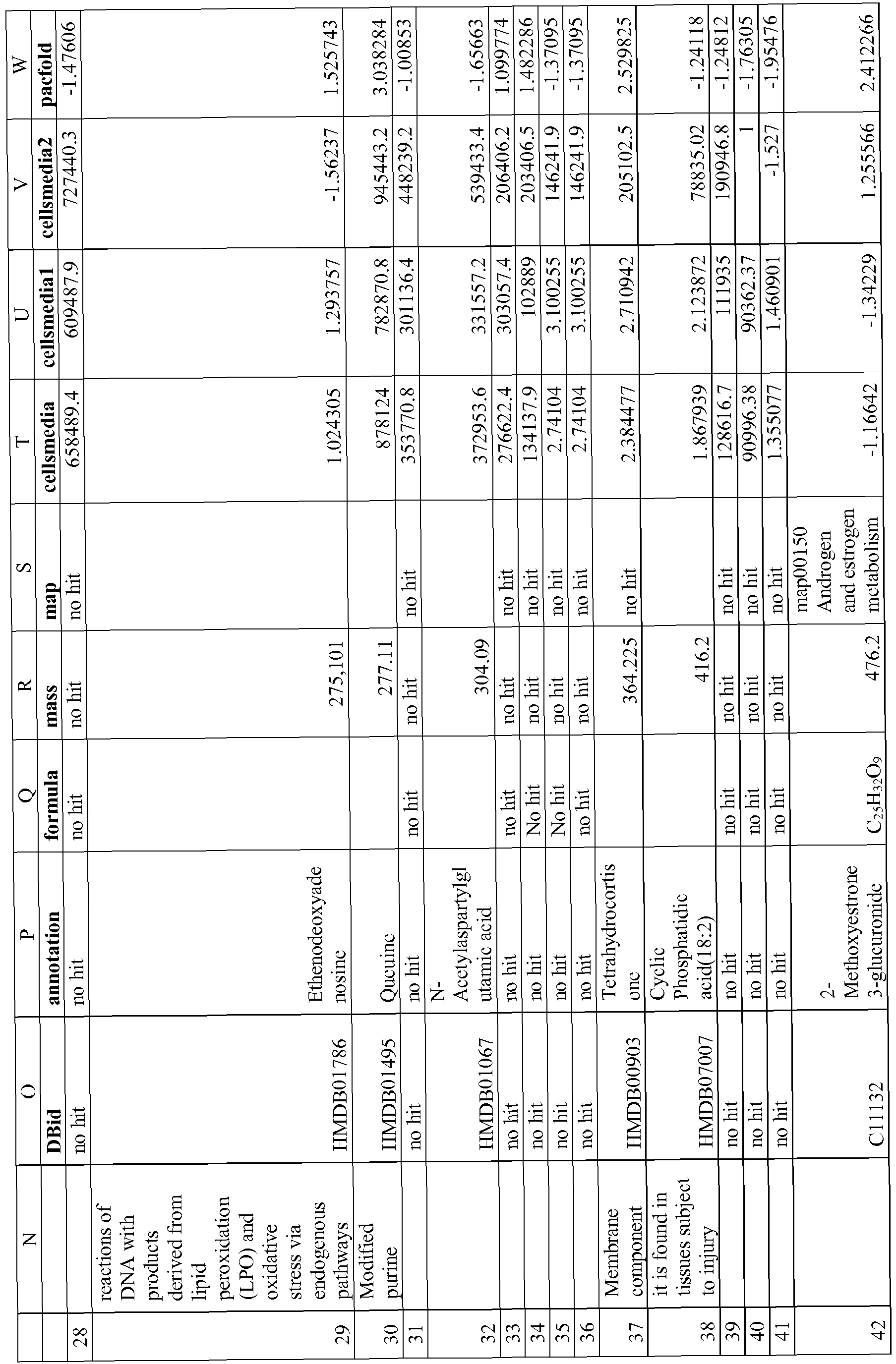

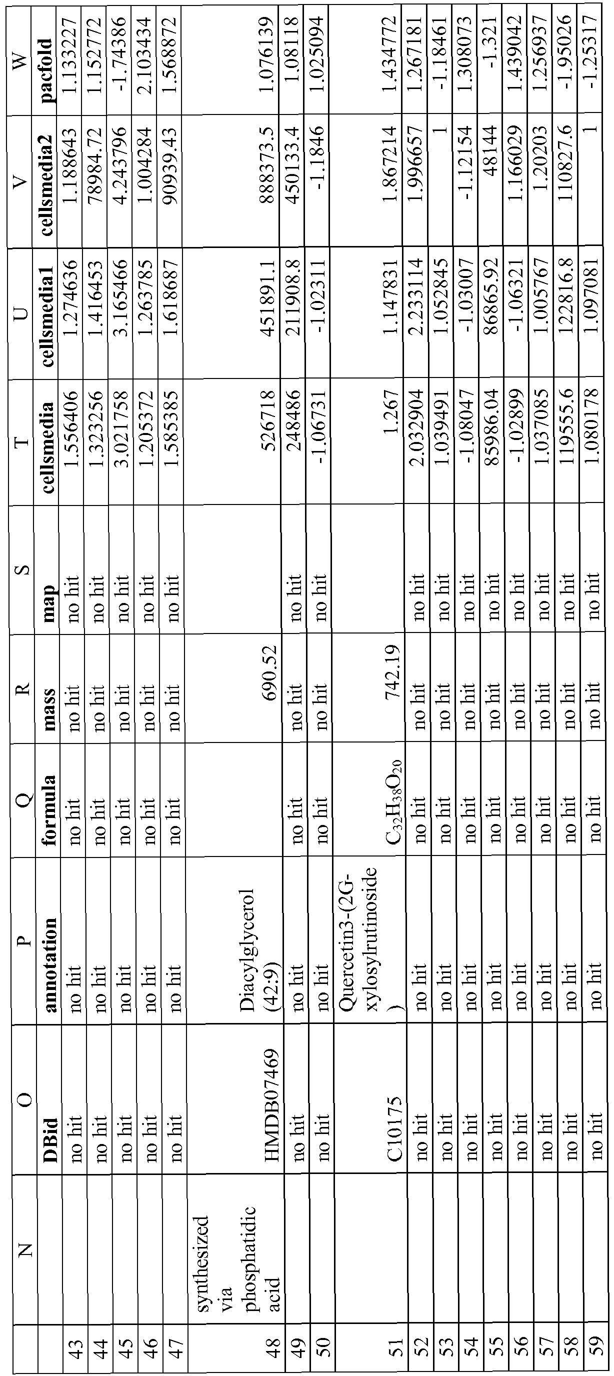

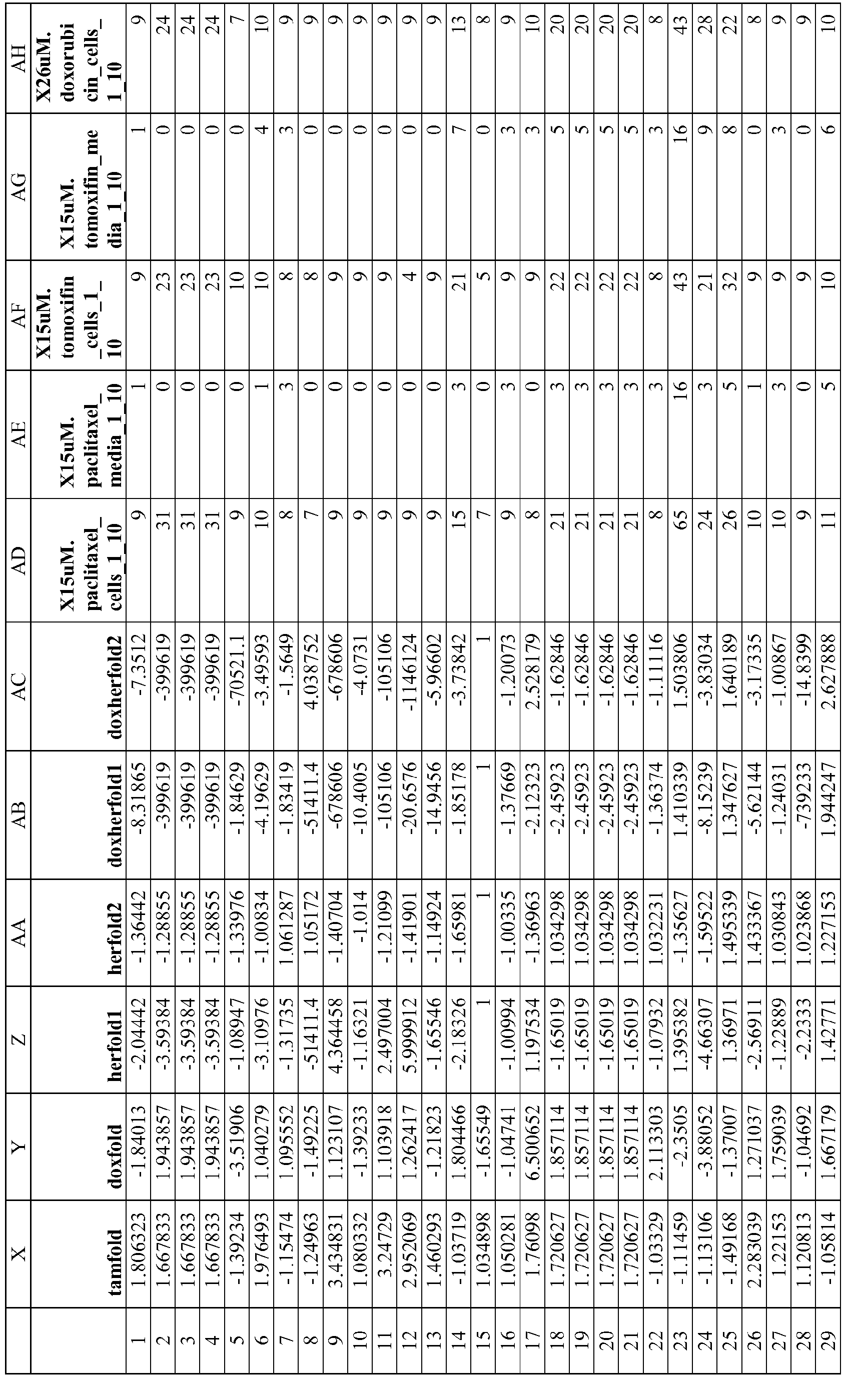

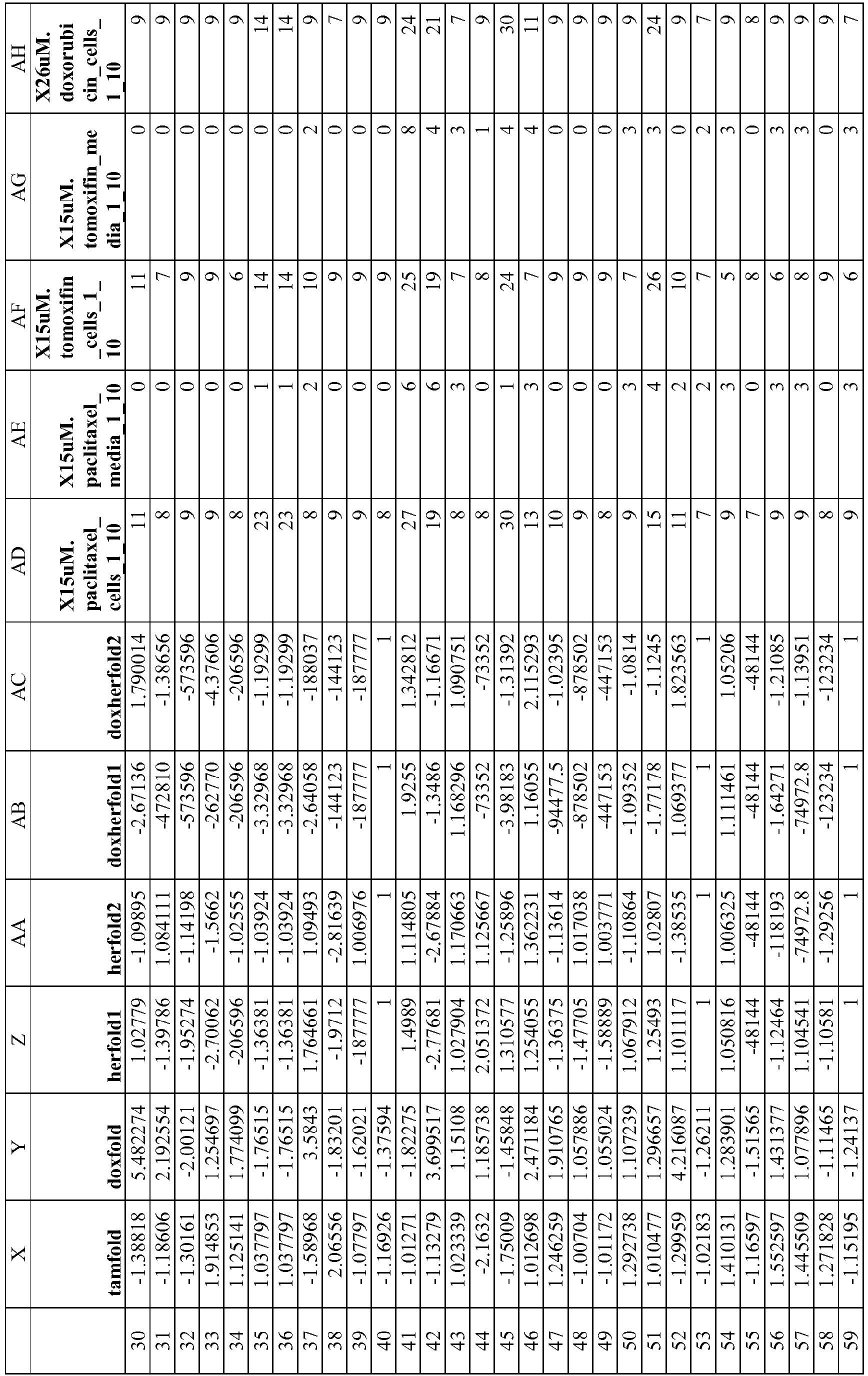

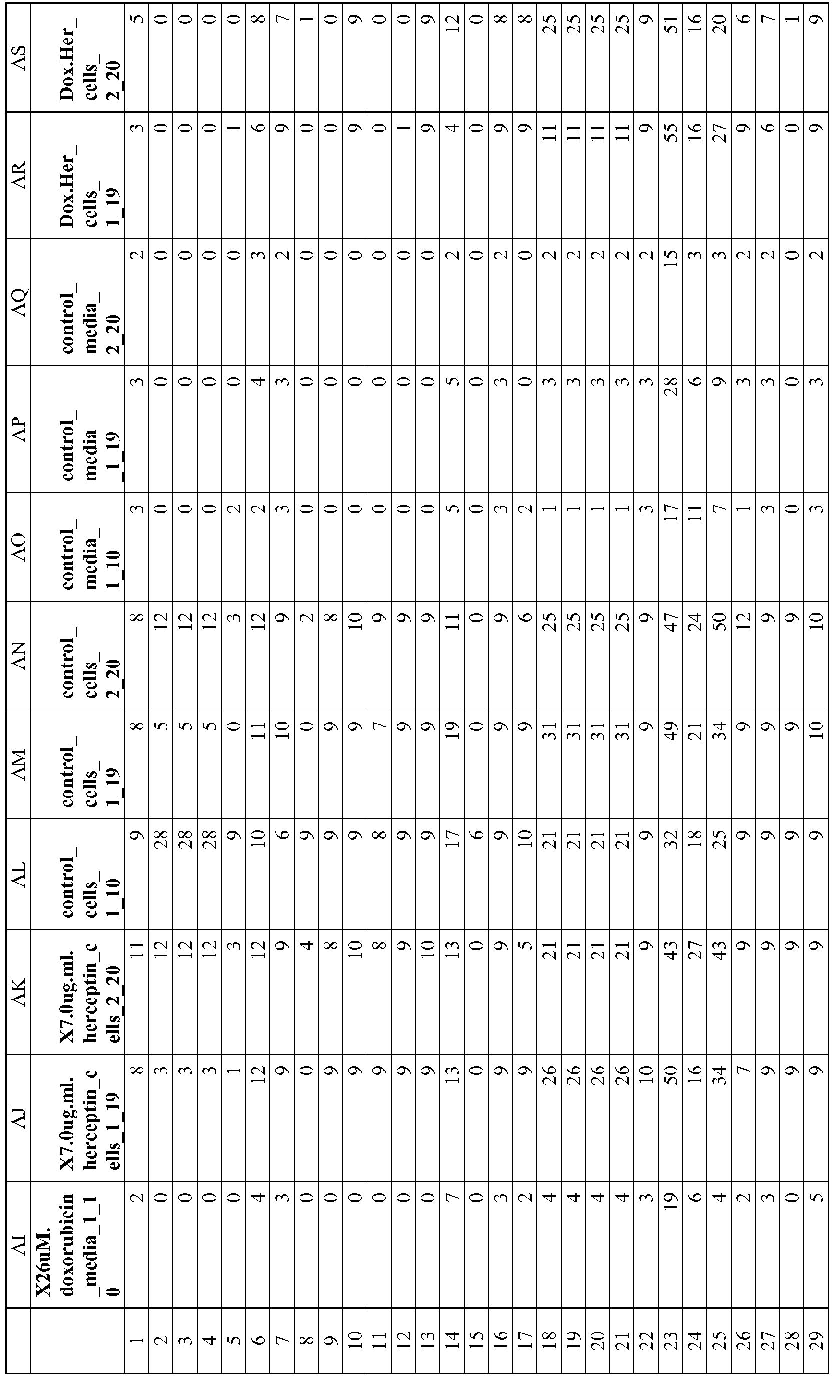

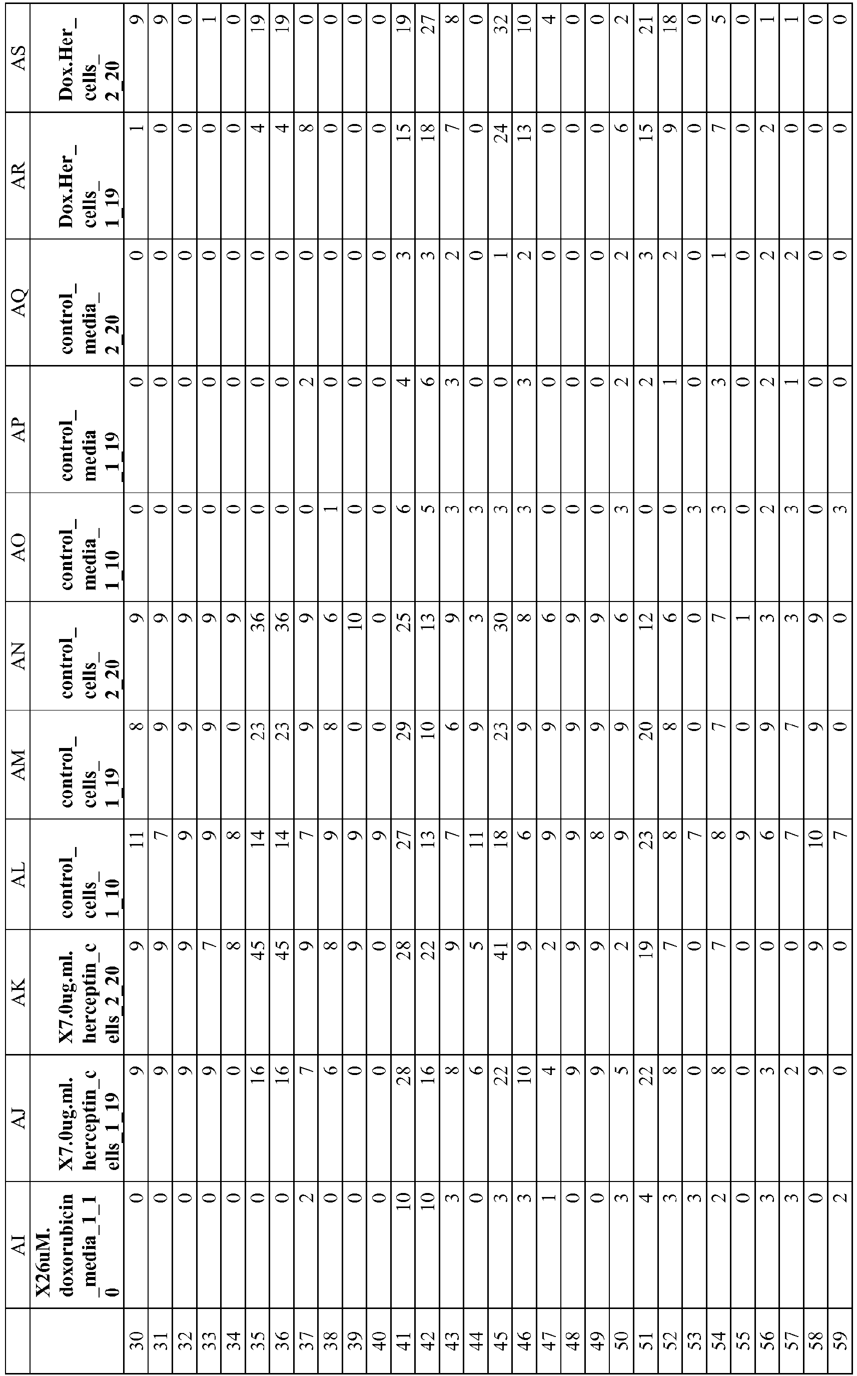

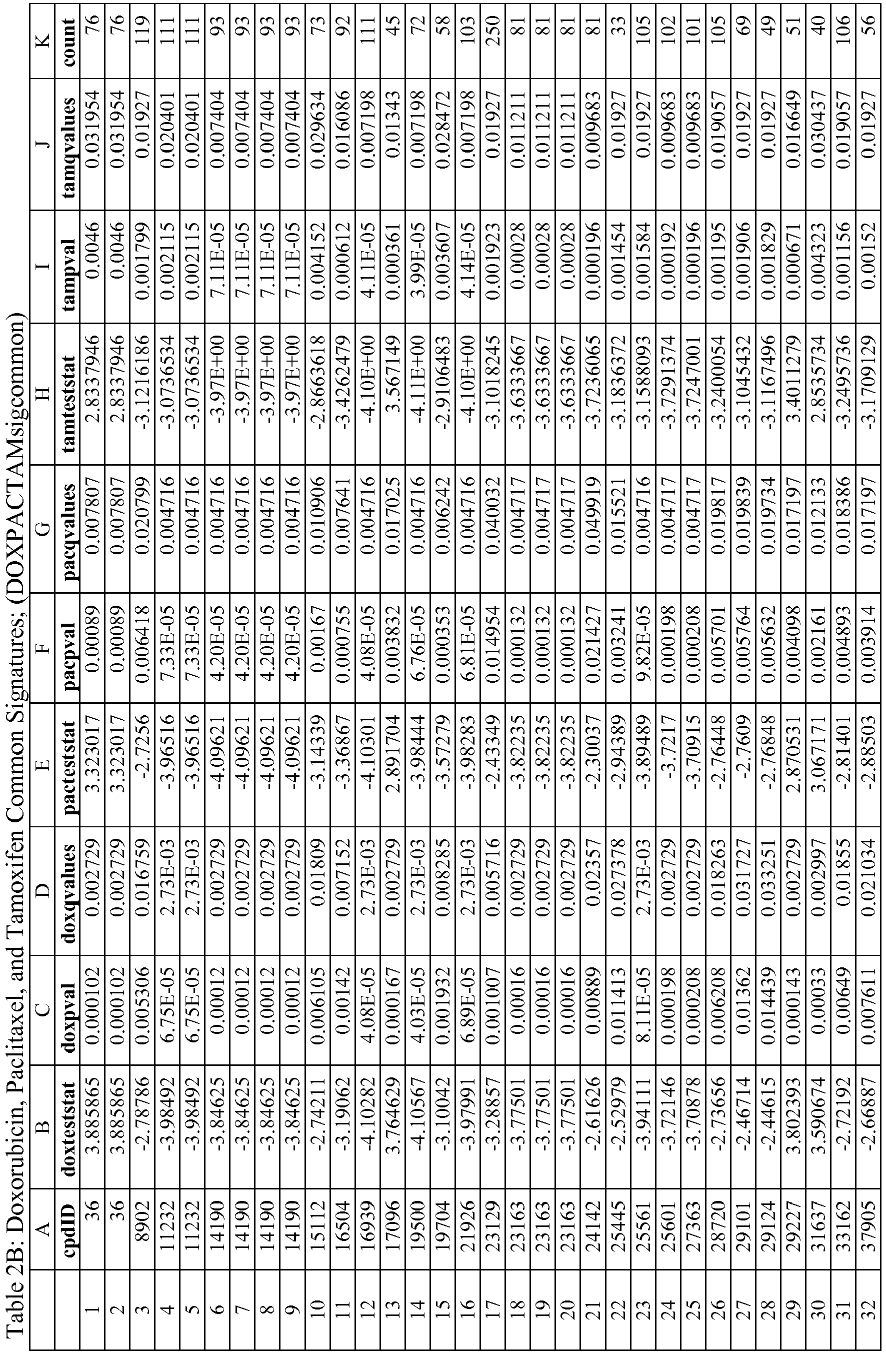

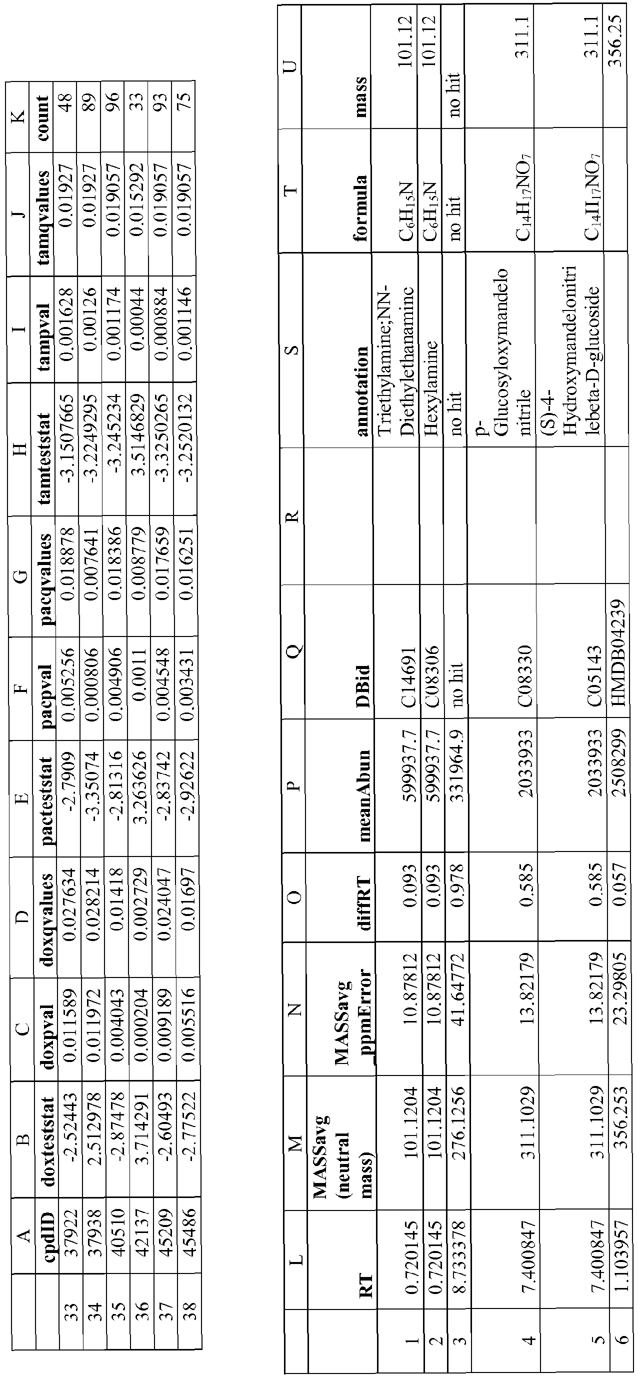

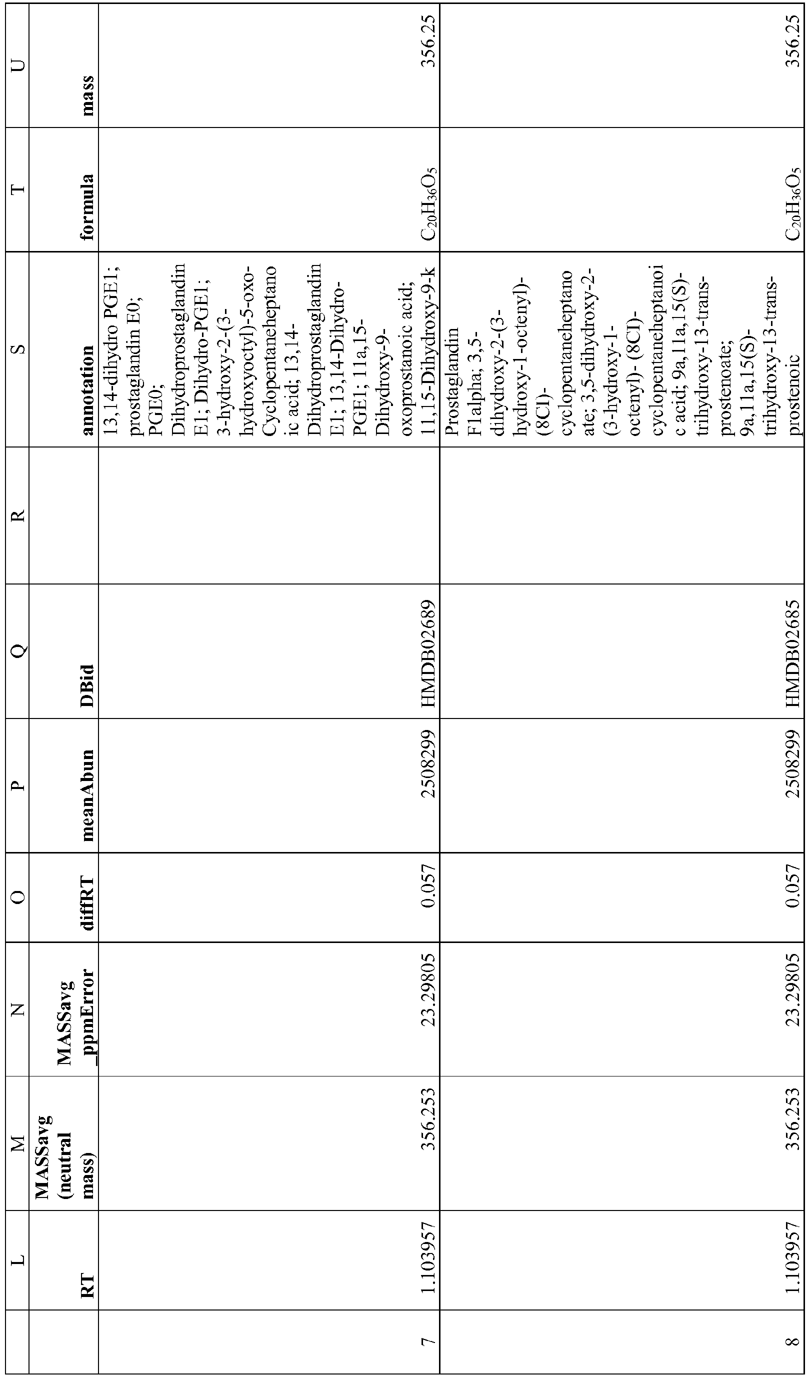

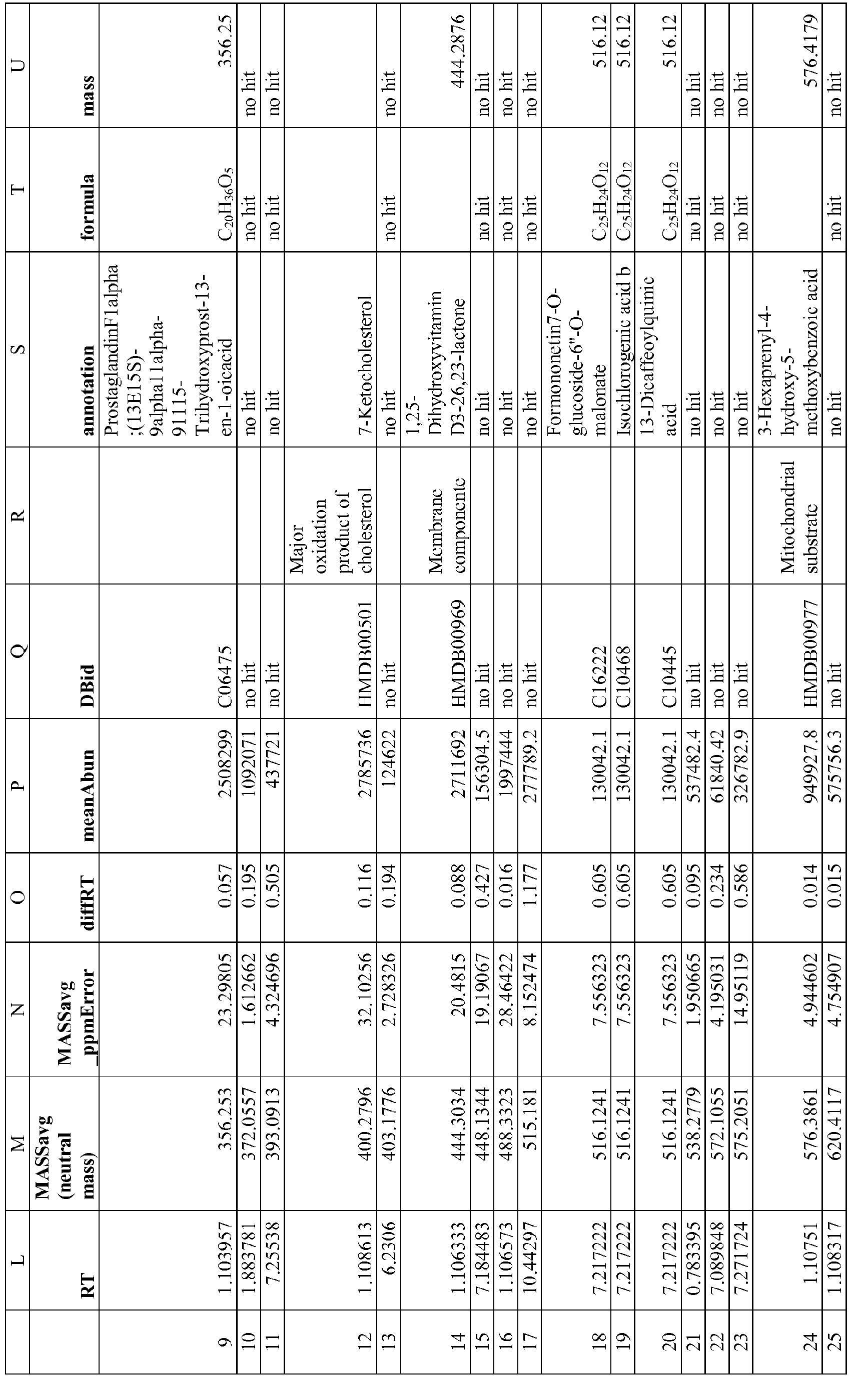

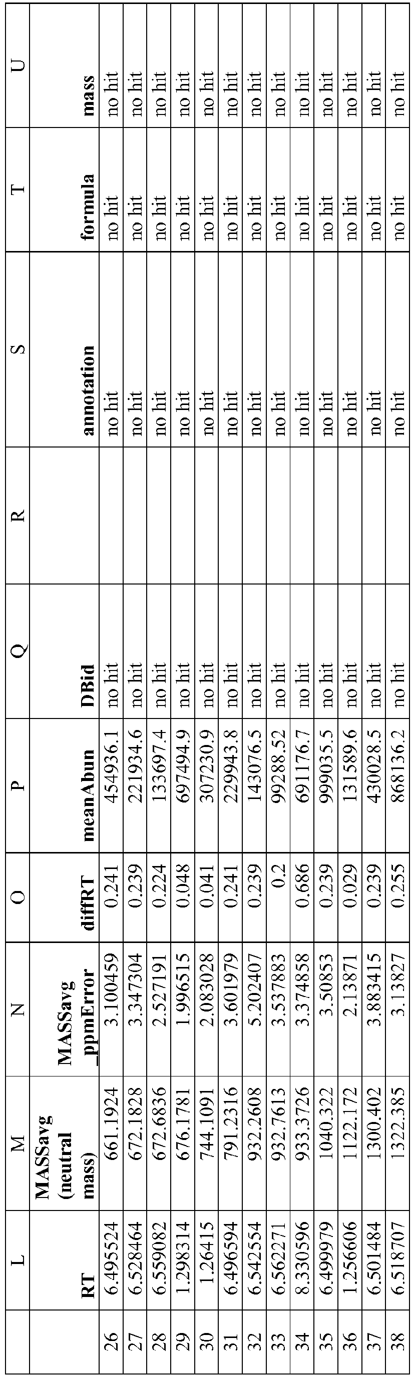

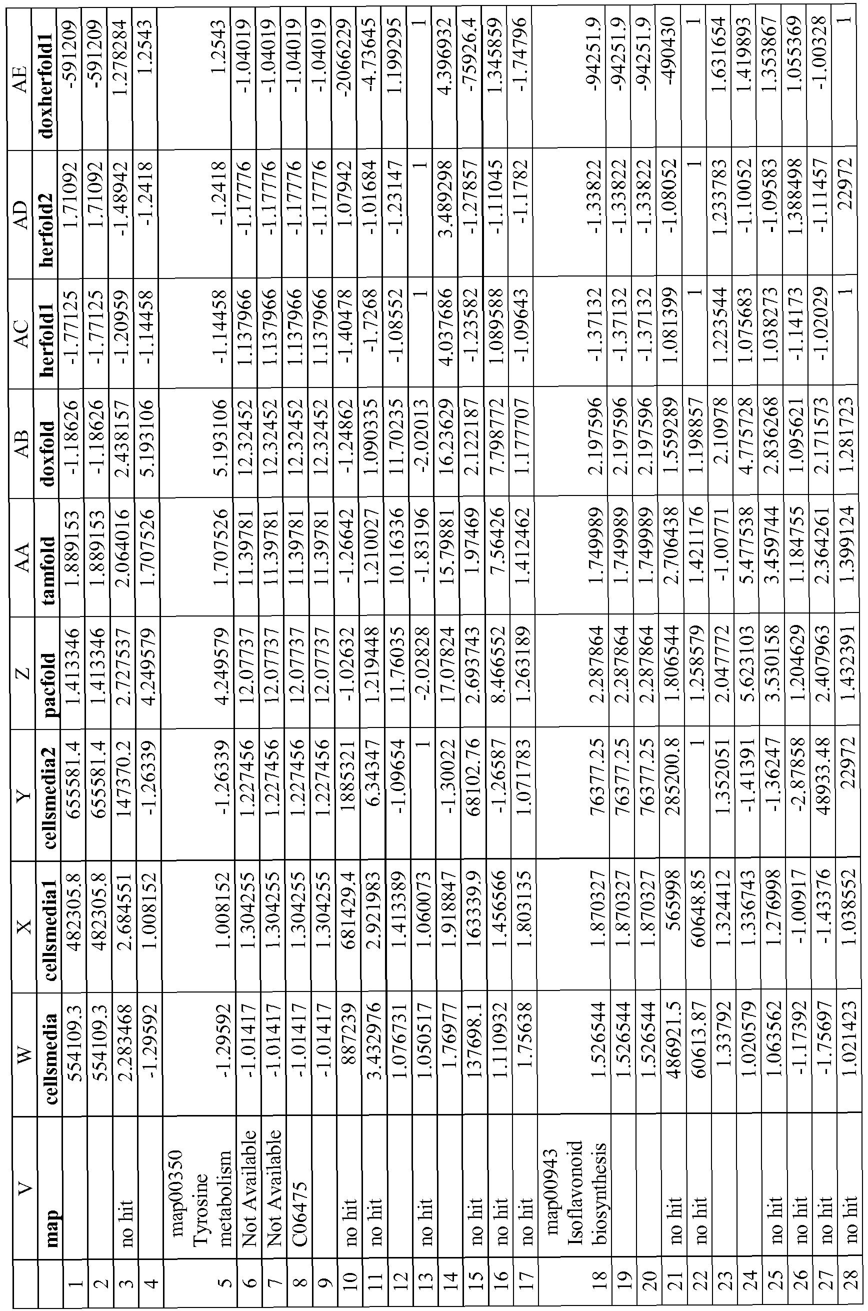

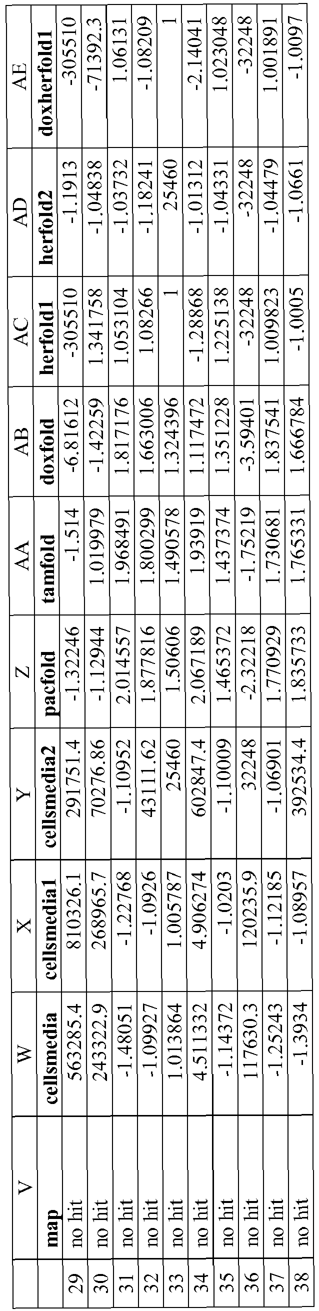

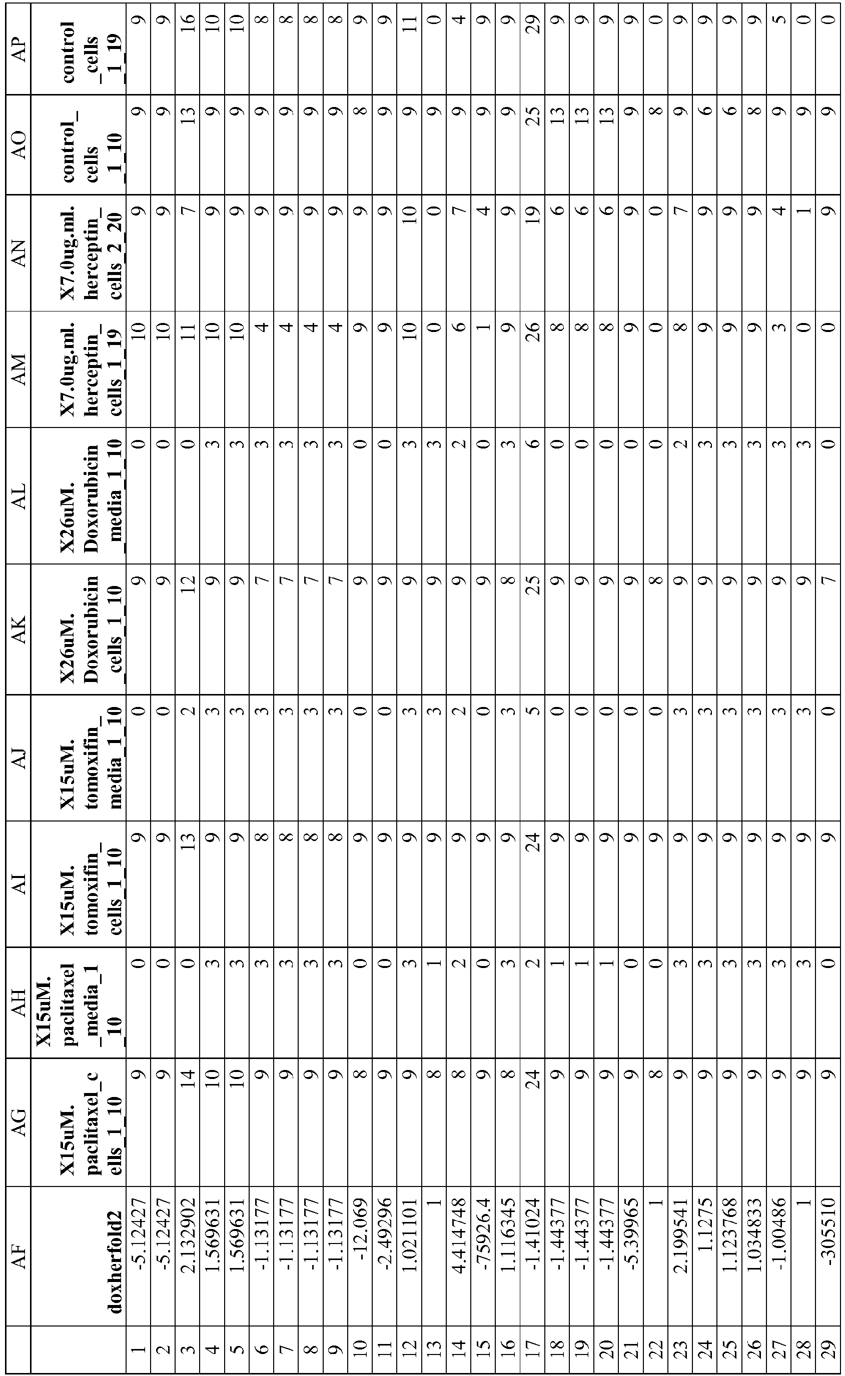

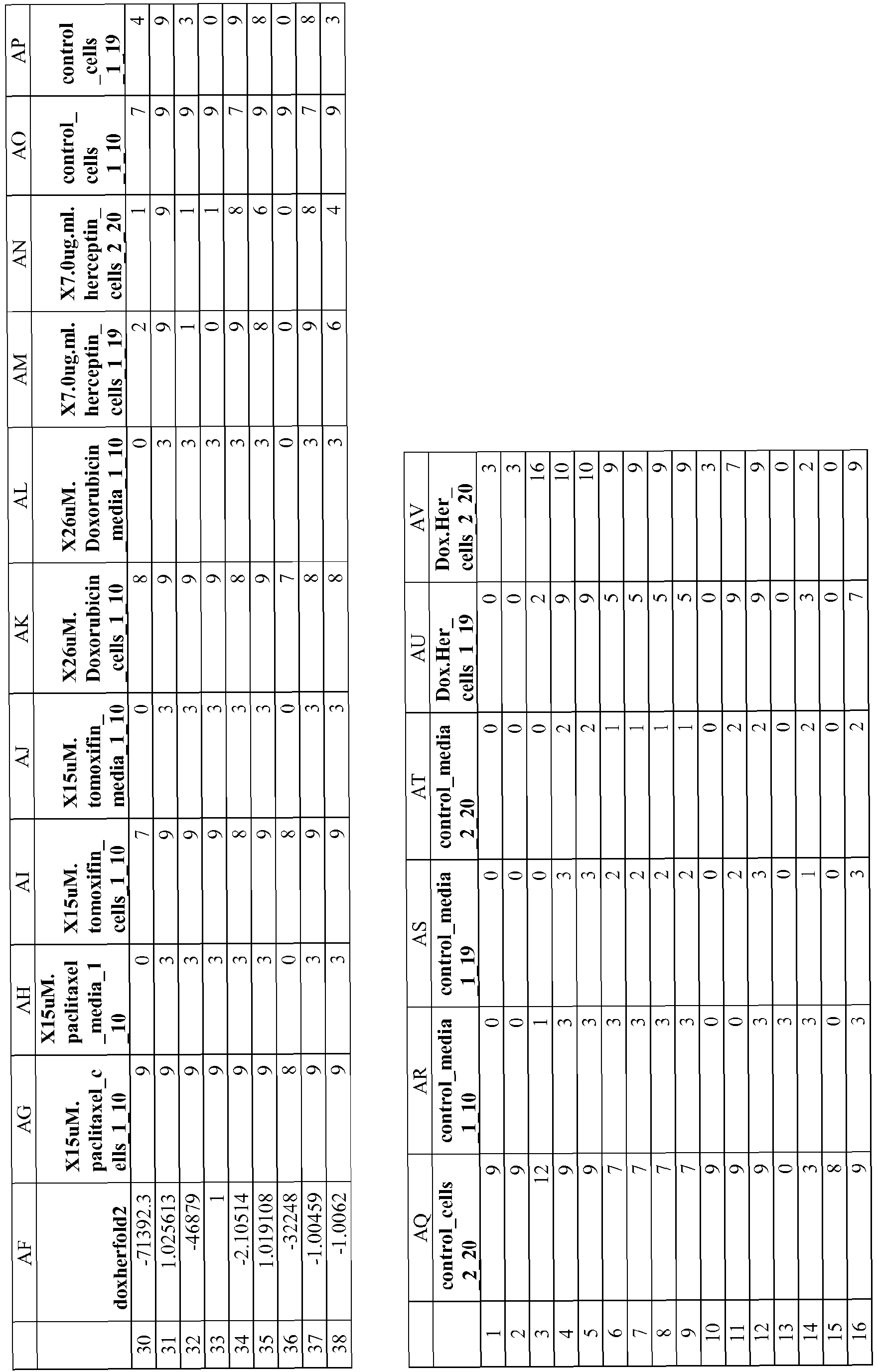

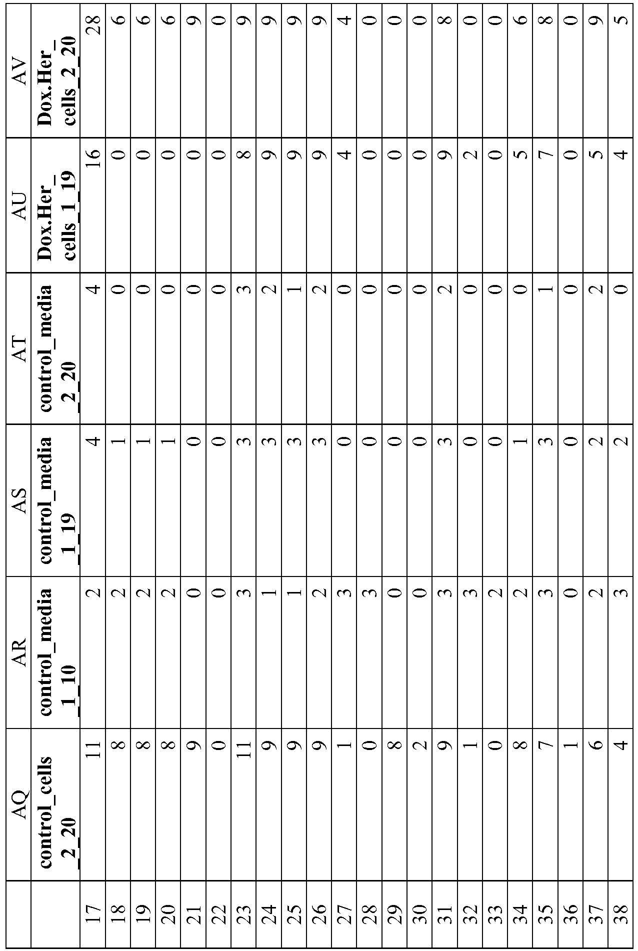

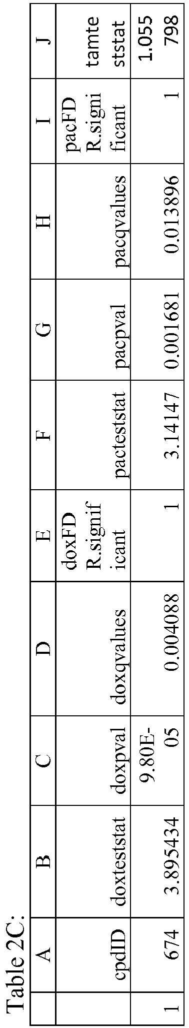

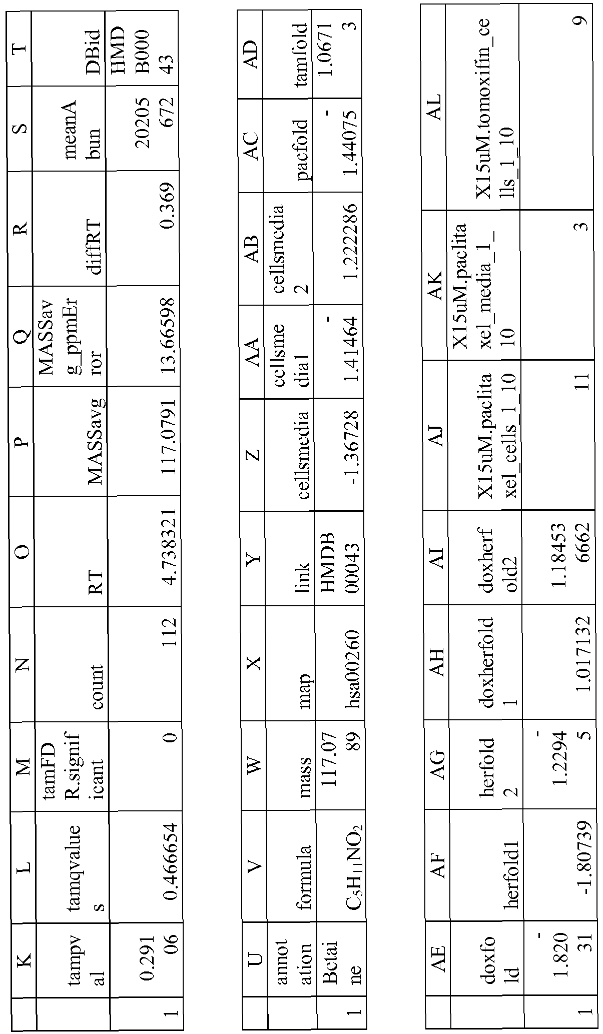

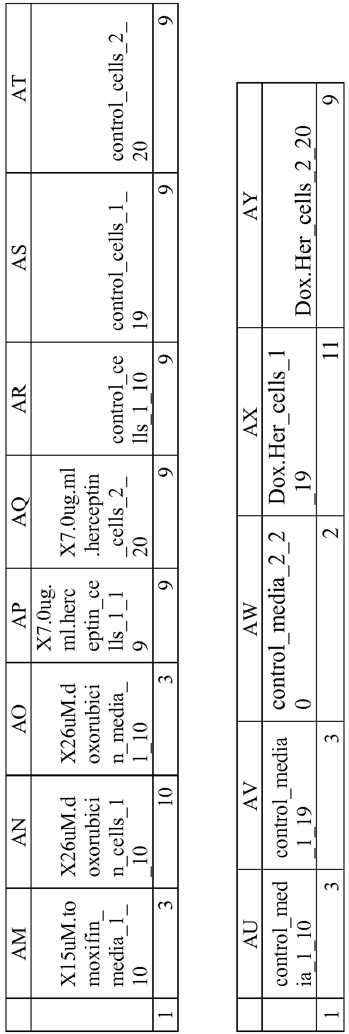

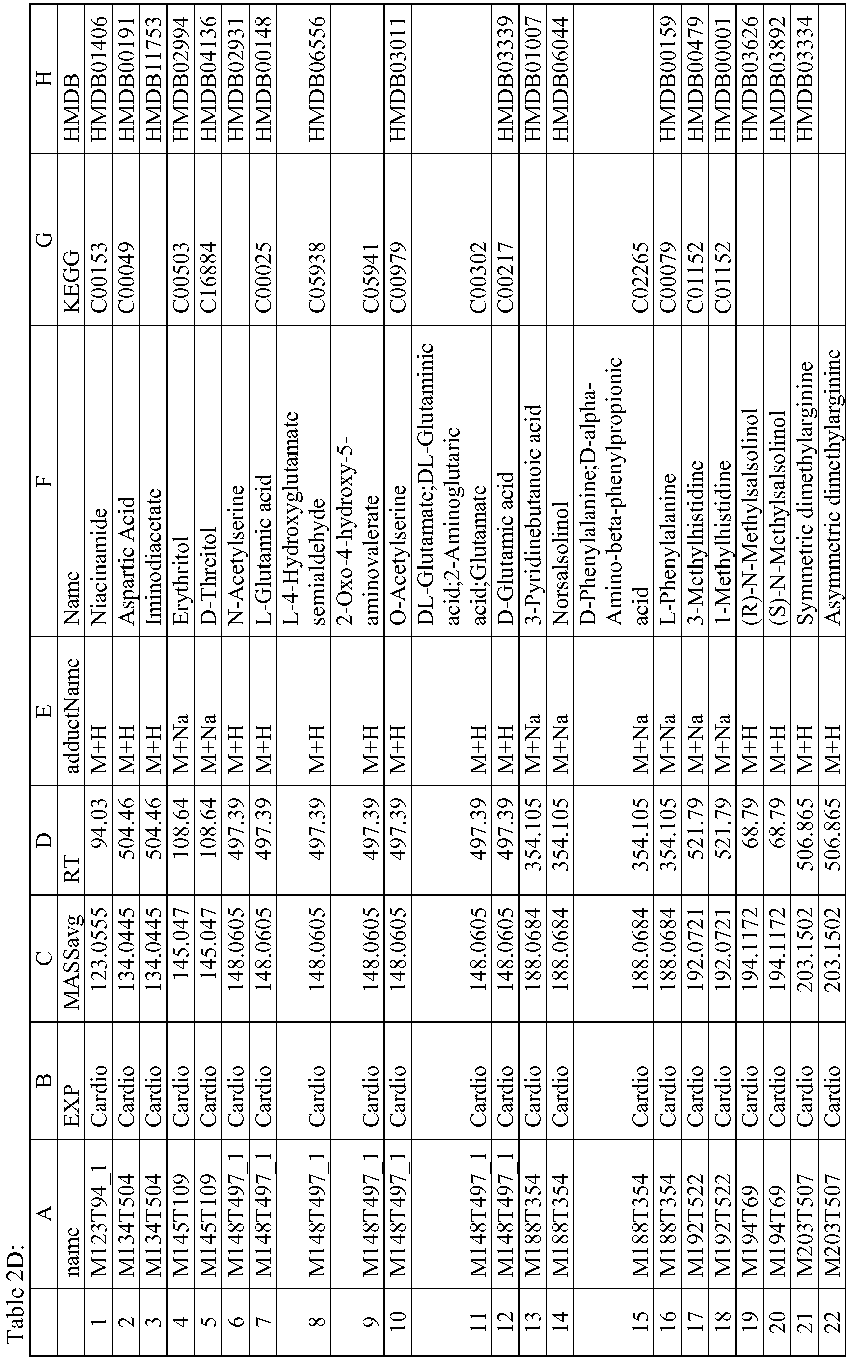

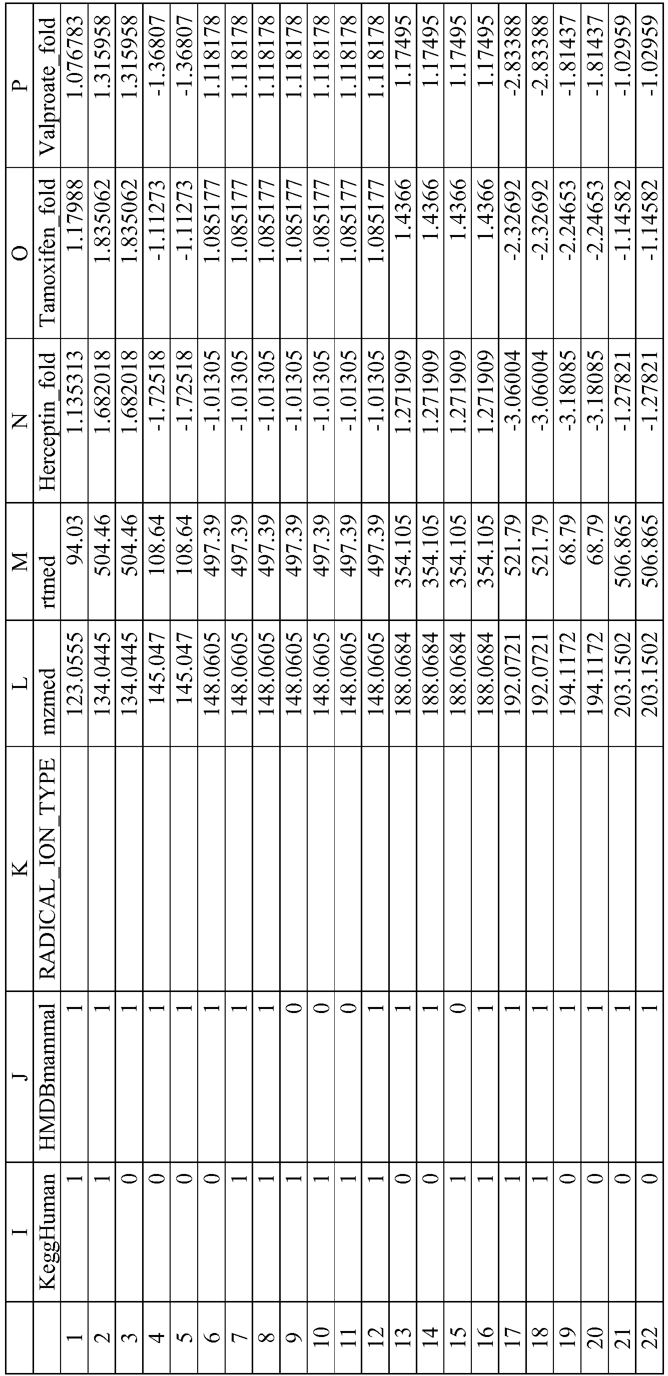

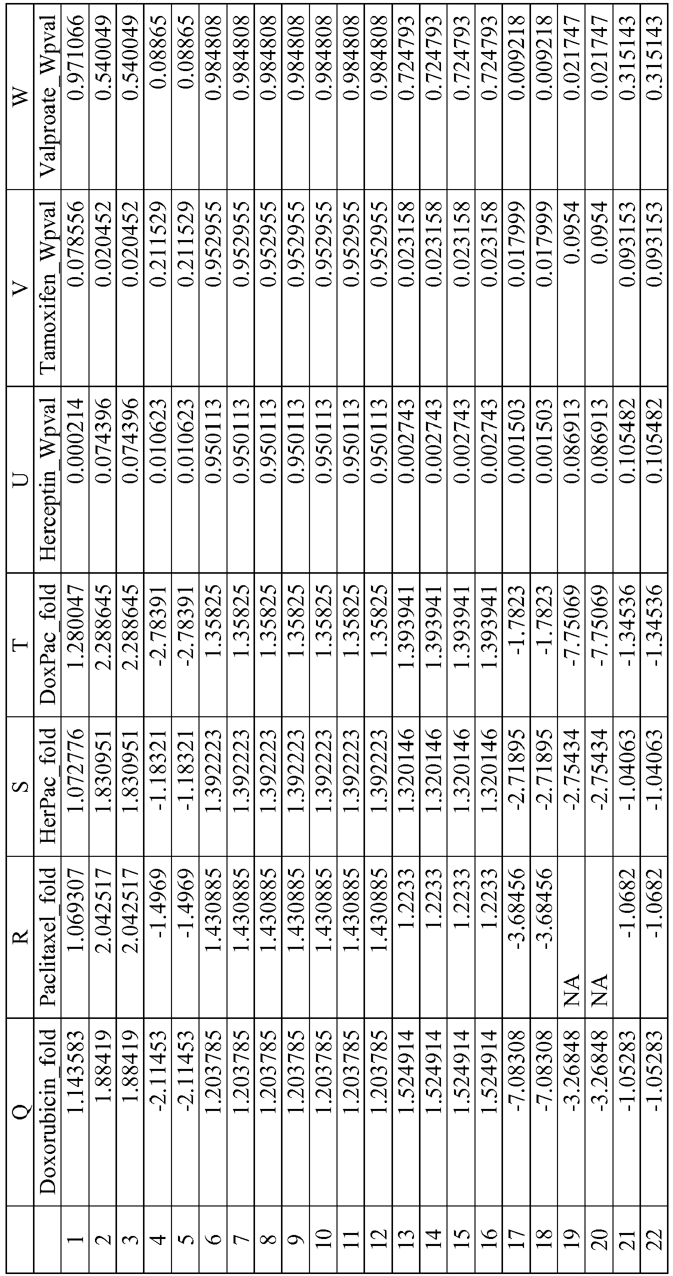

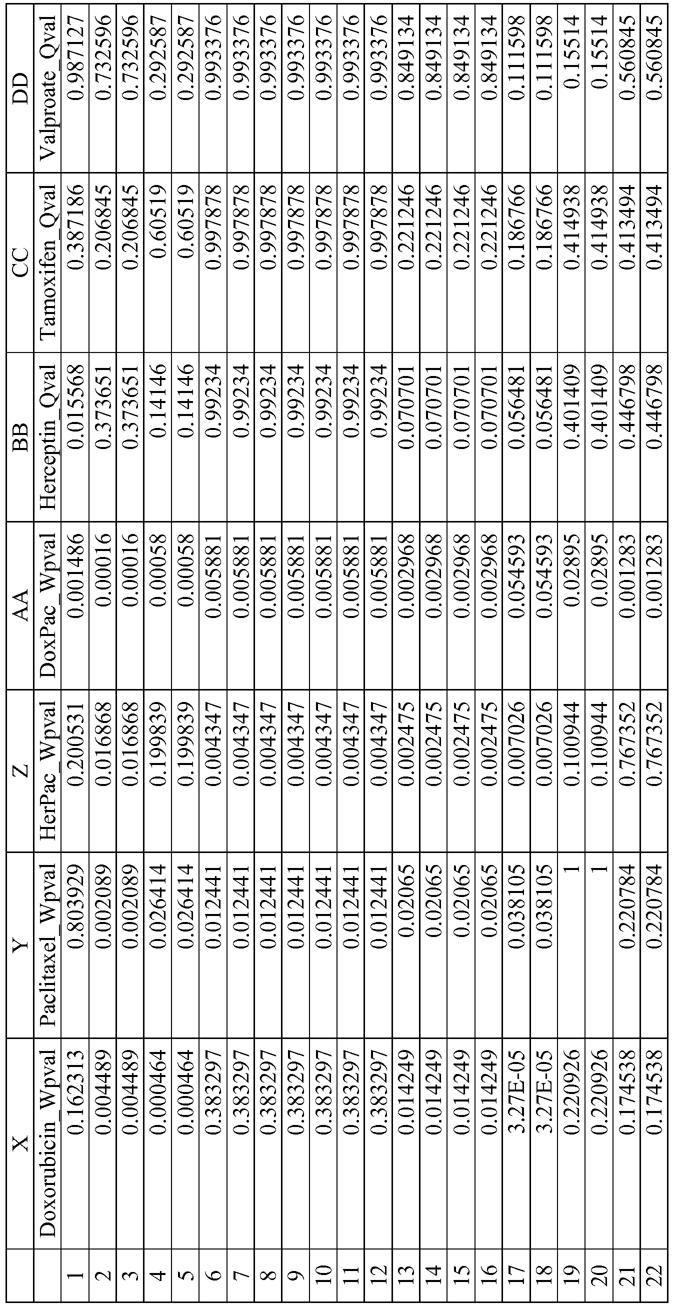

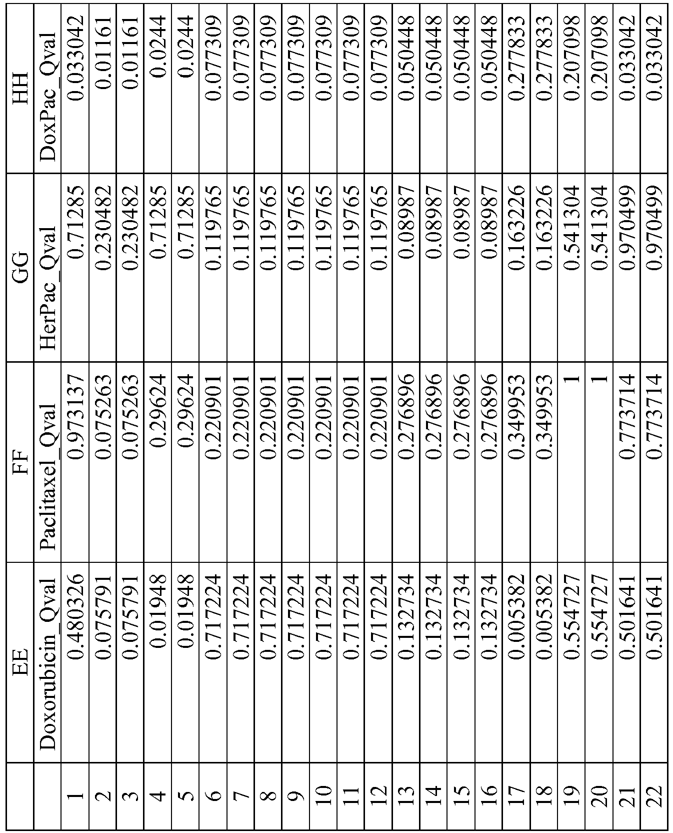

Statistically-significant changes in metabolite secretion can be examined for novel or non-annotated low molecular weight molecules, using the approach reported previously (Cezar et al, 2007, Stem Cells and Development 16: 869-882). Initial experiments have shown that a subset of human metabolites are indeed statistically-significantly altered in response to pharmaceuticals that are strong inducers of cardiomyopathies, namely doxorubicin and paclitaxel, in comparison to weak/moderate inducers such as tamoxifen. (Figure 3).

Data were accrued from n=107 mass spectrometry injections following exposure of human cardiomyocytes (Celprogen 36044-15at, San Pedro, CA) to three experimental treatments with different degrees of cardiotoxicity: (1.) doxorubicin; (2.) pacitaxel; and (3.) tamoxifen (weak toxicant). Following statistical analysis, with False Discovery Rates (FDR 0.05) adjustments, 187 significant features (*e.g*., candidate biomarkers), were identified in response to doxorubicin, 185 significant features in response to paclitaxel and 148 significant features in response to tamoxifen. Seventy-three statistically significant features were found to be in common to the strong cardiotoxicants doxorubicin and paclitaxel as described in the Preferred Embodiments. (Figure 3 and Table 2A).

The putative annotation of the exact neutral masses of such features in chemical databases revealed that several candidate biomarkers map onto energy metabolism pathways, such as NADPH₂: oxygen oxidoreductase activity, UDPglucuronate beta-D-glucuronosyltransferase, glycolysis, gluconeogenesis as well as oxidative stress. These results are consistent with published reports on the mechanisms of cardiotoxicity for these particular compounds.

Strong robustness and high reproducibility of low molecular weight molecules identified following exposure of human cardiomyocytes to the three established cardiotoxins: paclitaxel, doxorubicin, and tamoxifen was observed. The identification of metabolites secreted by cardiomyocytes in response to two or three cardiotoxins permitted enrichment for candidate biomarkers and provided a metabolic signature of cardiotoxicity.

## Claims

1. A method of identifying cellular metabolites differentially produced in human cardiomyocyte cells in the presence or absence of a test compound, the method comprising the steps of:
a) contacting cardiomyocyte cells *in vitro* with a test compound;
b) separating a plurality of cellular metabolites of from about 10 to about 1500 Daltons that are secreted from said cardiomyocyte cells using mass spectrometry; and
c) identifying one or a plurality of cellular metabolites of from about 10 to about 1500 Daltons that are differentially secreted from cardiomyocytes contacted with the test compound compared to cardiomyocytes not contacted with the test compound;
wherein said one or a plurality of cellular metabolites comprise one or a plurality of cellular metabolites set forth in Tables 2A-2D.

2. A method for identifying cellular metabolites differentially produced by human cardiomyocyte cells in the presence or absence of a plurality of cardiotoxic test compounds, the method comprising the steps of:
a) separately contacting each of a plurality of experimental sets of cardiomyocyte cells *in vitro* with a different cardiotoxic test compound;
b) separating a plurality of cellular metabolites of from about 10 to about 1500 Daltons that are secreted from each experimental set of cells using mass spectrometry;
c) identifying one or a plurality of cellular metabolites of from about 10 to about 1500 Daltons that are differentially secreted from cardiomyocytes contacted with each of the cardiotoxic test compounds compared to cardiomyocytes not contacted with the cardiotoxic test compound; and
d) identifying one or a plurality of cellular metabolites differentially produced by substantially all of said experimental sets of cardiomyocyte cells exposed to said test compounds;
wherein said one or a plurality of cellular metabolites comprise one or a plurality of cellular metabolites set forth in Tables 2A-2D.

3. A method of assessing cardiotoxicity of a test compound comprising the steps of:
a) contacting human cardiomyocyte cells *in vitro* with the test compound;
b) separating a plurality of cellular metabolites of from about 10 to about 1500 Daltons that are secreted from said cardiomyocyte cells using mass spectometry; and
c) identifying the test compound as a cardiotoxic compound if at least one or a plurality of cellular metabolites of from about 10 to about 1500 Daltons that are differentially secreted from cardiomyocytes contacted with the test compound comprise a metabolic profile of cardiotoxicity;
wherein said one or a plurality of cellular metabolites comprise one or a plurality of cellular metabolites set forth in Tables 2A-2D.

4. A method according to any one of claims 1-3, wherein at least one of the cellular metabolites is produced in greater amounts in cardiomyocytes contacted with the test compound.

5. A method according to any one of claims 1-3, wherein at least one of the cellular metabolites is produced in greater amounts in cardiomyocytes not contacted with the test compound.

6. A method according to any one of claims 1-3, wherein the mass spectrometry is liquid chromatography/electrospray ionization time of flight mass spectrometry (LC/ESI-TOF-MS).

7. A method according to any one of claims 1-3, wherein the candidate cellular metabolites are identified by neutral mass.

8. A method according to any one of claims 1-3, wherein the test compound is a cardiotoxic compound.

9. A method according to claim 8, wherein the cellular metabolites identified thereby comprise a metabolic profile characteristic of cardiomyocyte cell response to a cardiotoxic compound.

10. A method according to claim 8 or 9, wherein the test compound is doxirubicin, tamoxifen or paclitaxel.

11. A method according to claim 8 or 9, wherein the cellular metabolites comprise one or a plurality of Triethylamine; NN-Diethylamine; Hexylamine; p-Glucosyloxymandelonitrile; (s)-4-Hydroxymandelonitrilebeta-D-glucoside; 13, 14-dihydro PGE1 (Prostaglandin E1); 7-Ketocholesterol; 1,25-Dihydroxyvitamin D3-26,23-lactone; Formononetin 7-O-glucoside-6"-O-malonate; Isochlorogenic acid b; 13-Dicaffeoylquinic acid; 3-Hexaprenyl-4-hydroxy-5-methoxybenzoic acid; 2-Phenylglycine; (E)-4-Hydroxyphenylacetaldehyde-oxime; (Z)-4-Hydroxyphenylacetaldehyde-oxime; Betaine; 2-Ethylhexyl-4-hydroxybenzoate; Glycerophosphocholine; N-Acetylgalactosamine; CGP52608; Biotin; DL-Homocystine; Ethenodeoxyadenosine; Queuine; N-Acetylaspartylglutamic acid; Tetrahydrocortisone; Cyclic Phosphatidic acid; 2-Methoxyestrone3-glucuronide; Diacylglycerol; Quercetin3-(2G-xylosylrutinoside); Niacinamide; Aspartic Acid; Iminodiacetate; Erythritol; D-Threitol; N-Acetylserine; L-Glutamic acid; L-4-Hydroxyglutamate semialdehyde; 2-Oxo-4-hydroxy-5-aminovalerate; O-Acetylserine; DL-Glutamate; DL-Glutaminic acid; 2-Aminoglutaric acid; Glutamate; D-Glutamic acid; 3-Pyridinebutanoic acid; Norsalsolinol; D-Phenylalanine; D-alpha-Amino-beta-phenylpropionic acid; L-Phenylalanine; 3-Methylhistidine; 1-Methylhistidine; (R)-N-Methylsalsolinol; (S)-N-Methylsalsolinol; Symmetric dimethylarginine; or Asymmetric dimethylarginine.

## Patentansprüche

1. Verfahren zum Identifizieren von zellulären Metaboliten, die in humanen Kardiomyozytenzellen in Gegenwart oder Abwesenheit einer Testverbindung unterschiedlich produziert werden, wobei das Verfahren die Schritte umfasst:
a) Inkontaktbringen von Kardiomyozytenzellen *in vitro* mit einer Testverbindung;
b) Trennen einer Mehrzahl von zellulären Metaboliten von ungefähr 10 bis ungefähr 1500 Dalton, die aus den Kardiomyozytenzellen sekretiert werden, unter Verwendung von Massenspektrometrie; und
c) Identifizieren eines oder einer Mehrzahl von zellulären Metaboliten von ungefähr 10 bis ungefähr 1500 Dalton, die anders aus Kardiomyozyten, die mit der Testverbindung in Kontakt gebracht wurden, als aus Kardiomyozyten ohne Kontakt mit der Testverbindung sekretiert werden;
wobei der eine oder eine Mehrzahl von zellulären Metaboliten einen oder eine Mehrzahl von zellulären Metaboliten umfasst, die in den Tabellen 2A-2D angeführt sind.

2. Verfahren zum Identifizieren von zellulären Metaboliten, die von humanen Kardiomyozytenzellen unterschiedlich produziert werden, in Gegenwart oder Abwesenheit einer Mehrzahl von kardiotoxischen Testverbindungverbindungen, wobei das Verfahren die Schritte umfasst:
a) separates Inkontaktbringen jeder einer Mehrzahl von experimentellen Sätzen von Kardiomyozytenzellen *in vitro* mit einer unterschiedlichen kardiotoxischen Testverbindung;
b) Trennen einer Mehrzahl von zellulären Metaboliten von ungefähr 10 bis ungefähr 1500 Dalton, die aus jedem experimentellen Zellsatz sekretiert werden, unter Verwendung von Massenspektrometrie;
c) Identifizieren eines oder einer Mehrzahl von zellulären Metaboliten von ungefähr 10 bis ungefähr 1500 Dalton, die anders aus Kardiomyozyten, die mit jeder der kardiotoxischen Testverbindungen in Kontakt gebracht wurden, als aus Kardiomyozyten ohne Kontakt mit der kardiotoxischen Testverbindung sekretiert werden; und
d) Identifizieren eines oder einer Mehrzahl von zellulären Metaboliten, die von im Wesentlichen allen der experimentellen Sätzen von Kardiomyozytenzellen, die den Testverbindungen ausgesetzt wurden, unterschiedlich produziert werden;
wobei der eine oder eine Mehrzahl von zellulären Metaboliten eine oder eine Mehrzahl von zellulären Metaboliten umfasst, die in den Tabellen 2A-2D angeführt sind.

3. Verfahren zum Bewerten der Kardiotoxizität einer Testverbindung, das die Schritte umfasst:
a) Inkontaktbringen von humanen Kardiomyozytenzellen *in vitro* mit der Testverbindung;
b) Trennen einer Mehrzahl von zellulären Metaboliten von ungefähr 10 bis ungefähr 1500 Dalton, die aus den Kardiomyozytenzellen sekretiert werden, unter Verwendung von Massenspektrometrie; und
c) Identifizieren der Testverbindung als kardiotoxische Verbindung, wenn zumindest einer oder eine Mehrzahl von zellulären Metaboliten von ungefähr 10 bis ungefähr 1500 Dalton, die anders aus Kardiomyozyten sekretiert werden, die mit der Testverbindung in Kontakt gebracht wurden, ein kardiotoxisches Stoffwechselprofil umfasst;
wobei der eine oder eine Mehrzahl von zellulären Metaboliten einen oder eine Mehrzahl von zellulären Metaboliten umfasst, die in den Tabellen 2A-2D angeführt sind.

4. Verfahren nach einem der Ansprüche 1 bis 3, wobei zumindest einer der zellulären Metaboliten in mit der Testverbindung in Kontakt gebrachten Kardiomyozyten in größeren Mengen produziert wird.

5. Verfahren nach einem der Ansprüche 1 bis 3, wobei zumindest einer der zellulären Metaboliten in nicht mit der Testverbindung in Kontakt gebrachten Kardiomyozyten in größeren Mengen produziert wird.

6. Verfahren nach einem der Ansprüche 1 bis 3, wobei die Massenspektrometrie Flüssigkeitschromatographie/Elektrosprayionisation-Flugzeit-Massenspektrometrie (LC/ESI-TOF-MS) ist.

7. Verfahren nach einem der Ansprüche 1 bis 3, wobei die zellulären Kandidatenmetaboliten durch neutrale Masse identifiziert werden.

8. Verfahren nach einem der Ansprüche 1 bis 3, wobei die Testverbindung eine kardiotoxische Verbindung ist.

9. Verfahren nach Anspruch 8, wobei die damit identifizierten zellulären Metaboliten ein Stoffwechselprofil umfassen, das für eine Kardiomyozytenzellantwort auf eine kardiotoxische Verbindung charakteristisch ist.

10. Verfahren nach Anspruch 8 oder 9, wobei die Testverbindung Doxirubicin, Tamoxifen oder Paclitaxel ist.

11. Verfahren nach Anspruch 8 oder 9, wobei die zellulären Metaboliten eines oder mehrere von Triethylamin; NN-Diethylamin; Hexylamin; p-Glucosyloxymandelonitril; (s)-4-Hydroxymandelonitrilbeta-D-glucosid; 13,14-Dihydro-PGE1 (Prostaglandin E1); 7-Ketocholesterol; 1,25-Dihydroxyvitamin-D3-26,23-lacton; Formononetin-7-O-glucosid-6"-O-malonat; Isochlorogensäure b; 13-Dicaffeoylchinasäure; 3-Hexaprenyl-4-hydroxy-5-methoxybenzoesäure; 2-Phenylglycin; (E)-4-Hydroxyphenylacetaldehydoxim; (Z)-4-Hydroxyphenylacetaldehydoxim; Betain; 2-Ethylhexyl-4-hydroxybenzoat; Glycerophosphocholin; N-Acetylgalactosamin; CGP52608; Biotin; DL-Homocystin; Ethenodesoxyadenosin; Queuin; N-Acetylaspartylglutaminsäure; Tetrahydrokortison; cyclischer Phosphatidsäure; 2-Methoxyestron3-glucuronid; Diacylglycerol; Quercetin3-(2G-xylosylrutinosid); Niacinamid; Asparaginsäure; Iminodiacetat; Erythritol; D-Threitol; N-Acetylserin; L-Glutaminsäure; L-4-Hydroxyglutamatsemialdehyd; 2-Oxo-4-hydroxy-5-aminovalerat; O-Acetylserin; DL-Glutamat; DL-Glutaminsäure; 2-Aminoglutarsäure; Glutamat; D-Glutaminsäure; 3-Pyridinbuttersäure; Norsalsolinol; D-Phenylalanin; D-alpha-Amino-beta-phenylpropionsäure; L-Phenylalanin; 3-Methylhistidin; 1-Methylhistidin; (R)-N-Methylsalsolinol; (S)-N-Methylsalsolinol; symmetrischem Dimethylarginin; oder asymmetrischem Dimethylarginin umfassen.

## Revendications

1. Procédé d'identification de métabolites cellulaires produits différentiellement dans des cellules cardiomyocytaires humaines en présence ou en l'absence d'un composé d'essai, le procédé comprenant les étapes consistant à :
a) mettre en contact des cellules cardiomyocytaires *in vitro* avec un composé d'essai ;
b) séparer une pluralité de métabolites cellulaires d'environ 10 à environ 1500 Daltons qui sont sécrétés depuis lesdites cellules cardiomyocytaires à l'aide d'une spectrométrie de masse ; et
c) identifier au moins un métabolite cellulaire d'environ 10 à environ 1500 Daltons qui est sécrété différentiellement depuis les cardiomyocytes mis en contact avec le composé d'essai en comparaison avec les cardiomyocytes non mis en contact avec le composé d'essai ;
ledit au moins un métabolite cellulaire comprenant au moins un métabolite cellulaire indiqué dans les tableaux 2A à 2D.

2. Procédé d'identification de métabolites cellulaires produits différentiellement dans des cellules cardiomyocytaires humaines en présence ou en l'absence d'une pluralité de composés d'essai cardiotoxiques, le procédé comprenant les étapes consistant à :
a) mettre en contact séparément chaque groupe d'une pluralité de groupes expérimentaux de cellules cardiomyocytaires *in vitro* avec un composé d'essai cardiotoxique différent ;
b) séparer une pluralité de métabolites cellulaires d'environ 10 à environ 1500 Daltons qui sont sécrétés depuis chaque groupe expérimental de cellules à l'aide d'une spectrométrie de masse ;
c) identifier au moins un métabolite cellulaire d'environ 10 à environ 1500 Daltons qui est sécrété différentiellement depuis les cardiomyocytes mis en contact avec chacun des composés d'essai cardiotoxiques en comparaison avec les cardiomyocytes non mis en contact avec le composé d'essai cardiotoxique ; et
d) identifier au moins un métabolite cellulaire produit différentiellement par sensiblement la totalité desdits groupes expérimentaux de cellules cardiomyocytaires exposés auxdits composés d'essai ;
ledit au moins un métabolite cellulaire comprenant au moins un métabolite cellulaire indiqué dans les tableaux 2A à 2D.

3. Procédé d'évaluation de cardiotoxicité d'un composé d'essai, comprenant les étapes consistant à :
a) mettre en contact des cellules cardiomyocytaires *in vitro* avec un composé d'essai ;
b) séparer une pluralité de métabolites cellulaires d'environ 10 à environ 1500 Daltons qui sont sécrétés depuis lesdites cellules cardiomyocytaires à l'aide d'une spectrométrie de masse ; et
c) identifier le composé d'essai comme un composé cardiotoxique si au moins un métabolite cellulaire d'environ 10 à environ 1500 Daltons qui est sécrété différentiellement depuis les cardiomyocytes mis en contact avec le composé d'essai comprend un profil métabolique de cardiotoxicité ;
ledit au moins un métabolite cellulaire comprenant au moins un métabolite cellulaire indiqué dans les tableaux 2A à 2D.

4. Procédé selon l'une quelconque des revendications 1 à 3, dans lequel au moins un des métabolites cellulaires est produit en plus grande quantité dans les cardiomyocytes mis en contact avec le composé d'essai.

5. Procédé selon l'une quelconque des revendications 1 à 3, dans lequel au moins un des métabolites cellulaires est produit en plus grande quantité dans les cardiomyocytes non mis en contact avec le composé d'essai.

6. Procédé selon l'une quelconque des revendications 1 à 3, dans lequel la spectrométrie de masse est une chromatographie liquide/spectrométrie de masse à temps de vol à ionisation par électronébulisation (LC/ESI-TOF-MS).

7. Procédé selon l'une quelconque des revendications 1 à 3, dans lequel les métabolites cellulaires candidats sont identifiés par masse neutre.

8. Procédé selon l'une quelconque des revendications 1 à 3, dans lequel le composé d'essai est un composé cardiotoxique.

9. Procédé selon la revendication 8, dans lequel les métabolites cellulaires identifiés comprennent ainsi une caractéristique de profil métabolique de réponse de cellule cardiomyocitaire à un composé cardiotoxique.

10. Procédé selon la revendication 8 ou 9, dans lequel le composé d'essai est la doxirubicine, le tamixofène ou le paclitaxel.

11. Procédé selon la revendication 9, dans lequel les métabolites cellulaires comprenant au moins un élément parmi la triéthylamine ; la NN-diéthylamine ; l'hexylamine ; le p-glucosyloxymandélonitrile ; le (s)-4-hydroxymandélonitrilebêta-D-glucoside ; la 13,14-dihydro PGE1 (prostaglandine E1) ; le 7-cétocholestérol ; la 1,25-dihydroxyvitamine D3-26,23-lactone ; le 7-O-glucoside-6"-O-malonate de formononétine ; l'acide isochlorogénique b ; l'acide 13-dicafféoylquinique ; l'acide 3-hexaprényl-4-hydroxy-5-méthoxybenzoïque ; la 2-phénylglycine ; le (E)-4-hydroxyphénylacétaldéhyde-oxime ; le (Z)-4-hydroxyphénylacétaldéhyde-oxime ; la bétaïne ; le 2-éthylhexyl-4-hydroxybenzoate ; la glycérophsphocholine ; la N-acétylgalactosamine ; CGP52608 ; la biotine ; la DL-homocystéine ; l'éthènodésoxyadénosine ; la queuine ; l'acide N-acétylaspartylglutamique ; la tétrahydrocortisone ; l'acide phosphatidique cyclique ; le 2-méthoxyestérone3-glucuronide ; le diacylglycérol ; le quercétine3-(2G-xylosylrutinoside) ; le niacinamide ; l'acide aspartique ; l'iminodiacétate ; l'érythritol ; le D-thréitol ; la N-acétylsérine ; l'acide L-glutamique ; le L-4-hydroxyglutamate semialdéhyde ; le 2-oxo-4-hydroxy-5-aminovalérate ; l'O-acétylsérine ; le DL-glutamate ; l'acide DL-glutamique ; l'acide 2-aminoglutarique ; le glutamate ; l'acide D-glutamique ; l'acide 3-pyridinebutanoïque ; le norsalsolinol ; la D-phénylalanine ; l'acide D-alpha-amino-bêta-phénylpropionique ; la L-phénylalanine ; la 3-méthylhistidine ; la 1-méthylhistidine ; le (R)-N-méthylsalsolinol ; le (S)-N-méthylsalsolinol ; la diméthylarginine symétrique ; ou la diméthylarginine asymétrique.
